# EUROPEAN PATENT SPECIFICATION

(11) **EP 1 075 656 B1**
(45) Date of publication and mention of the grant of the patent: **06.07.2011**
(21) Application number: 99918252.0
(22) Date of filing: 04.05.1999
(51) Int. Cl.: G01N 27/327, G01N 33/543, G01N 27/00, C12Q 1/68

(54) **DETECTION OF A TARGET IN A SAMPLE**
NACHWEIS EINER ZIELSUBSTANZ IN EINER PROBE
DETECTION D'UNE CIBLE DANS UN ECHANTILLON

(30) Priority: 04.05.1998 IL 12432298
(43) Date of publication of application: 14.02.2001
(73) Proprietor: Technion Research & Development Foundation Limited, Haifa 32000 (IL); Eichen, Yoav, 32922 Haifa (IL); Sivan, Uri, 34602 Haifa (IL); Braun, Erez, 32684 Haifa (IL)
(72) Inventor: EICHEN, Yoav, 32922 Haifa (IL); SIVAN, Uri, 34602 Haifa (IL); BRAUN, Erez, 32684 Haifa (IL)
(74) Representative: Desaix, Anne
(86) International application number: PCT/IL1999/000232
(87) International publication number: WO 1999/057550

(56) References cited:
- EP-A- 0 364 208
- EP-A- 0 444 840
- WO-A-90/05300
- US-A- 5 284 748

## Description

### FIELD OF THE INVENTION

The present invention concerns a device, a system, a method and a kit for assaying a target (for example a biological entity) in a sample.

### BACKGROUND OF THE INVENTION

Detection of biological moieties, such as biological molecules, bacteria, viruses and cells, in a sample is a routine procedure in fields such as medicine, industry and defense. In medicine, detection is routinely carried out for monitoring clinically and bio-chemically important analytes in a sample obtained from a patient which may be a blood sample, urine sample, etc.

Typically, detection of biological molecules, such as proteins, is carried out by employing well known immunoassay techniques, such as ELISA, radio-immuno assays, etc.

Assays for the presence of specific DNA or RNA sequences in a sample have various applications including the detection of microorganismal infections in patients, analysis of food or environmental samples to detect a contamination, detection of genetic diseases caused by mutations, etc. Simultaneous detection of a large number of different nucleic acid sequences became important in genome projects, i.e. sequencing of the full genomes of various organisms, particularly in the human genome project. Such sequencing typically involves the detection of a large number of short, partially overlapping nucleic acid sequences and based thereon constructing a full genetic map. Such a technique which is termed *"sequencing by hybridization "* (SBH), involves the digestion of long DNA molecules into smaller fragments and their subsequent hybridization with an array of short probes. Another application involves the simultaneous detection of a large number of different nucleic acid sequences for the purpose of gene expression and diagnostics.

The presence of a specific DNA or RNA sequence in a sample can be detected by a labeled probe capable of specific hybridization with the DNA or RNA sequence. However, direct detection by a probe is limited in present techniques to relatively high concentrations of the target DNA or RNA. In order to overcome this problem, methods for amplifying nucleic acid sequences have been developed, including PCR (polymerase-chain-reaction), LCR (ligase-chain-reaction) and 3SR (self-sustained sequence replication). All amplification methods consume substantial time and labor, require specific conditions for the activity of the amplifying enzymes and intricate laboratory apparatus.

Attempts have been made to develop electrochemical sensors which can directly measure the concentration of an analyte in a sample. The sensors generally detect a change in physical, electrical or optical properties as a result of interaction with the analyte.

U.S. 4,314,821 discloses a system for detection of antibodies in a sample based on a change in resonance frequency of piezoelectric oscillators, as antibodies bind to the oscillator. U.S. 4,822,566 discloses an apparatus for detecting the presence and/or measuring the concentration of an analyte in a fluid medium, relying on the bio-specific binding of the analyte to a biochemical host system thus modifying the dielectric properties of the sensor.

U.S. 5,312,527 discloses a voltammetric sensor for the detection of a target polynucleotide sequence in a sample which binds to a complementary sequence immobilized on a amperometric electrode. A change in the electrode's electric response then indicates the presence of the target sequence in the sample and includes means for detecting, voltammetrically, immobilized heteroduplexes.

WO 9744651 discloses an apparatus for the detection of a specific nucleic acid sequence in a sample, which involves the use of a biosensor comprising an electrode and a bilayer lipid membrane. The membrane is composed or modified lipid molecules which are assembled into an electrode/ionic reservoir/insulating bilayer combination that is suitable for incorporation of ion channels and ionophores. The conductance of such a membrane is dependent on the presence, or absence of an analyte. The presence of the specific nucleic acid sequence changes the impedance of the membrane which is then measured.

U.S. 5,284,748 which corresponds to WO 90/05300 discloses a method for detecting the occurrence of a binding, or complex-forming reaction between specific substances by utilizing the binding reaction to modify an electric circuit and then measuring the change in electrical state of the circuit. According to this method, biogenic substances such as antigens are coated onto a non-conductive base, present between a pair of electrodes superimposed on the base. Other biogenes such as antibodies which specifically bind with the antigen are pretreated so that they become bound to conductive particles. The particles having antibody bound thereto, are then added to the antigen layer deposited on the non-conductive base. The conductive particles are thereby bound to the base due to the binding between the antigen and the antibody, and thus form aggregates of electrically conductive particles which modify the circuit. The particles can then be selectively coated with a conductive substance which binds essentially only to said particles, and does not bind to the remainder of the path between the electrodes in order to enhance conductiveness of the electric circuit formed by the two electrodes and the aggregates formed therebetween.

### GLOSSARY

In the following, use will be made with some terms, some of which and their meaning are as follows:
***Biological entity** -* an entity which is derived from a biological source and may be a biological molecule (nucleic acid, protein, lipid, antibody, hormone, etc.) a complex of several biological molecules, a bacterium, a virus, a cell (of a multicellular or unicellular organism) a cell organelle (nucleus, nucleozyme, mitochondria).
***Affinity group** -* a group of at least two entities, at least one of which is a biological entity, capable of specifically *binding* (see below) to each other. Examples of affinity groups are: two complementary strands of nucleic acid sequences; antibody-antigen, ligand-receptor; enzyme-substrate, glycoprotein-lectin, bacterium and its antibody; DNA - DNA binding protein; etc. An example of an affinity group containing a non-biological entity is an antibody and its specific non-biological hapten.
***Binding** -* non-covalent specific interactions (ionic, van der Waals, hydrogenic, hydrophobic, etc.) between at least two members of an affinity group, for example, the interactions between a nucleic acid sequence and its specific complementary sequence, the interaction between an antibody and its antigen, etc.
***Target -*** an entity, which is to be assayed in a sample. The target, which may be biological or non-biological entity, is a member of an affinity group with the other member being the *recognition moiety* (see below). The target may be an unmodified analyte as originally present in the sample, or the target may be such an analyte which has been modified to either improve its binding affinity to the recognition moiety and/or modified by binding thereto of *a nucleation-center forming entity* (see below) or a group to which *nucleation-center forming entity* (see below) may subsequently bind.
***Recognition moiety** -* An entity which specifically binds the target. The target and the recognition moiety are thus two members of an affinity group. A recognition moiety is used for the detection of the target in the sample. The recognition moiety may be *attached* (see below) to an *electrode* (see below) or to a substrate disposed between two electrodes.
***Binding moiety -*** a term referring collectively to either a recognition moiety or a moiety which can bind to the target in a non-specific manner or in a semi-specific manner (semi-specific binding meaning binding to a group of entities which display a common characteristic, e.g. binding to molecules with a specific charge, binding to mRNA in general, binding to a class of antibodies, e.g. to all human IgG antibodies, etc.).
***Attachment (attach)** -* the interaction between the recognition or binding moiety and a substrate (for example the electrode and/or the substrate between electrodes) whereby said moiety becomes immobilized onto said substrate. The interaction may be by covalent or by non-covalent binding.
***Conductive bridge** -* A physical link between at least two electrodes which conducts electric current. A conductive bridge can form between electrodes only if the recognition moiety binds present on at least one of the electrodes or on the substrate between the electrodes binding to the target to form a complex between recognition moieties and target. The complex of recognition-moiety and thetarget, together with *nucleation-center forming entities* (see below) bound to the complex, serves as a base for growth of a conductive substance between the electrodes to yield the conductive bridge. Alternatively, the complex may serve as a base for the binding of polymerizable monomers which, upon polymerization, form a conductive polymer. A conductive bridge may at times also be referred to herein as a *"wire*".
***Nucleation-center forming entities** -* Entities which allow specific deposition of a conductive substance or serve as catalysts for growth of a conductive substance. The nucleation-center forming entities bind specifically to the target-recognition moiety complex on an assay set, for example by having a moiety allowing specific or non-specific binding to a group previously attached to the target. The nucleation-center forming entities can also be attached to the target before it is complexed with the recognition moiety on the assay set. Examples for nucleation-center forming entities are: gold colloids, other metal colloids or gold or other metal atom containing species, or conjugated polymer forming precursors. Nucleation-center forming entitles can, for example, be attached to streptavidin that can bind to a target entity having a biotin group, or alternatively to an anti-double stranded DNA antibody.
***Electrode** -* A conducting substrate, which may be made of metal or of any other conducting material or coated by metal or other conducting material, which serves for connection of the recognition or binding moiety to external electronic or electric components or circuitry, thus serving as an input/output (I/O) interface with an external component or circuitry.
***External circuitry, external component*** at times referred to as *"**electric or electronic module**" -* An electronic or electric circuitry or an electronic or electric component, situated electrically external to the electrodes and typically comprises prior art electric or electronic components, including standard solid-state microelectronic components.
***Linker** -* An agent (molecule, complex of molecules, supramolecular structure, macromolecule, aggregate, colloid particle, molecular clusters, etc.) that acts in providing a physical link between the recognition moiety and the electrode or a substrate, thus serving to attach the recognition moiety to the electrode or the substrate. The linkers may have chemical groups for covalent or non covalent anchoring, (e.g. complexation or sorption, etc.) to the electrode or substrate, on the one hand, and to the recognition moiety on the other hand. Examples of linkers are: nucleic acid binding proteins; synthetic molecules with a binding ability to a specific nucleic acid sequence; a short, single or multiple stranded nucleic acid sequence (e.g. an oligonucleotide), e.g. having a *"sticky end"* and being modified at its other end, to allow it binding to the electrode; and non-biological molecules like derivatized alkyl silanes, etc.
***Sample** -* A medium which is to be tested for the presence of the target therein. Typically is a fluid obtained from a biological source, such as blood, urine, milk, food suspension, etc.
***Assaying (assay)** -* a term referring collectively to both qualitative and/or quantitative determination of the target in a sample.
***Assay device** -* a device for use in the assaying of the target.

### SUMMARY OF THE INVENTION

The present invention is based on a novel concept for detecting a target in a sample. The invention makes use of a system comprising an assay device which comprises one or more sets of electrodes physically separated from one another. Each set of electrodes forms, together with a recognition moiety immobilized either on at least one of the electrodes of the set, or on the substrate between the electrodes or on both electrodes, an assay set (also referred to occasionally hereinafter as *"detection site ").* The system further comprises external circuitry (electric or electronic module) which can determine the formation of a conductive bridge between at least two electrodes of a set. Formation of the conductive bridge occurs in the presence of reagents for growing a conductive substance between the electrodes, only if a complex between the target at the recognition moiety is formed as will be explained hereinbelow. Measurement of electric current, conductance, or resistance between the electrodes is indicative of the formation of said conductive bridge, which formation is in turn depends on the presence of the target in the sample. In other words, in the presence of the target in the sample, an electrically conducting bridge is created between the electrodes and is then detected by performing electrical measurements utilizing the electric or electronic circuitry. At minimum, the detection assay utilizes the system of the invention gives a qualitative result of the presence of the target in the sample, namely a *"yes"* or *"no"* answer; in other cases, the potential/current relationships can serve as a gauge for the concentration of the target in the sample as will be explained in detail hereinbelow.

According to one aspect of the invention, the conductive bridge is formed by growing conductive substance on nucleation-center forming entities which are either bound to or deposited on the complex formed between the recognition moiety and the targets.

In accordance with said first aspect the present invention provides a system for assaying one or more targets in a sample, comprising:
(a) an assay device having one or more assay sets at least one for each target to be assayed; each of the assay sets comprising at least two electrodes and a recognition moiety immobilized either to one or more of the at least two electrodes and/or on a substrate between the at least two electrodes; the recognition moiety being capable of specific binding to one of the targets;
(b) an electric or electronic module for determining electric conductance between the at least two electrodes of each assay set; and
(c) reagents for growing a conducting substance from nucleation-centers forming entities deposited onto or bound to a complex formed between said recognition moiety and said target, which substance forms a conductive bridge between at least two of the electrodes of a set.

Examples of nucleation-center forming entities are colloids of metal particles such as: gold, platinum, palladium rhodium, ruthenium, etc., as well as clusters and complexes of such metals.

The inclusion of the nucleation-center forming entities in the complexes formed between the target and the recognition moiety on the device may be achieved either by allowing the nucleation-center forming entities to bind to the complexes after their formation or, alternatively, by reacting the sample with the nucleation-center forming entities prior to contact with the device to allow their attachment to the target. In accordance with one embodiment, the nucleation-center forming entities bind directly to the complexes or the targets. Alternatively the nucleation-center forming entity is bound to an agent which recognizes a specific moiety on the target or on the complex. For example, the nucleation-center forming entity may be bound to avidin and the target then comprises a biotin entity to allow their binding to one another. Such a biotin entity may be bound to the analyte by a prior step of reacting a sample with appropriate reagents. In the above biotin-avidin example, the nucleation-center may be added either to the target when it is free in the sample or to the complex. Another example, applicable where both the target and the recognition moiety are single- stranded nucleic acid sequences, are nucleation-center forming entities, attached to antibodies against double-stranded nucleic acid sequences. In such a case, the nucleation-center forming entity is attached to the complex formed between the target and recognition moiety.

By another aspect of the present invention the conductive bridge of the present invention is a conductive polymer. In that case the invention provides a system for assaying one or more targets in a sample, comprising:
(a) an assay device having one or more assay sets at least one for each target to be assayed; each of the assay sets comprising at least two electrodes and a recognition moiety immobilized either to one or more of the at least two electrodes or on a substrate between the at least two electrodes; the recognition moiety being capable of specific binding to one of the targets;
(b) an electric or electronic module for determining electric conductance between the at least two electrodes of each assay set; and
(c) reagents comprising monomers of a conducting polymer which can bind to a complex formed between said recognition moiety and said target, such that upon polymerization of the monomers a conducting bridge between the at least two electrodes of a set is formed.

In accordance with one embodiment the polymer is *a priori* conductive. In accordance with another embodiment, the polymers become conducting by a step of doping. (The manner of doping polymers to render them conductive is well known to the artisan).

An example of this embodiment is the use of polyanyline formation as bridge.

For example, biotin can be conjugated to target if present in sample. Then a peroxidase-streptavidin complex is added after hybridization so that this complex binds only to a detection assay which comprise a complex between a target and a recognition moiety. Later, anyline and peroxide source are added to solution to enable polymerization only where peroxidase exists and finally the treatment is completed by doping of the polymers to afford conductivity.

By another alternative the conductive polymer can be grown utilizing nucleation-center forming entities which can be either bound to the targets or to the complexes between the recognition moiety and the targets as explained above.

The targets may be one or more nucleic acid sequences and in that case the recognition moiety may also be oligonucleotides having a sequence complementary to at least a portion of the target sequences.

Where the targets are nucleic acid sequences, the assay device may be in the form of a nucleic acid sequence chip, for example, a DNA chip.

The targets may also be non-nucleic acid sequences, such as sugars, hormones, proteins, or proteinaceous substances. Where the target is a protein, the recognition moiety may be protein binding molecules such as antibodies, or antibody fragments which still maintain the proteinaceous binding properties of the full antibody. The assay device in that case may be in the form of an antibody chip.

The properties of the system of the present invention allow formation of a fully conductive bridge between the two electrodes, (utilizing either nucleation-center forming entities, and a layer of conductive substance grown thereon, or formation of a conductive polymer between the two electrodes), even if the complexes between the targets and the recognition moiety do not form a physical connection between the two electrodes. For example, if the recognition moiety is immobilized on the substrate present between two electrodes of a set, it is possible that a complex formed between a target (bearing a nucleation-center forming entity), and the recognition moiety, does not physically connect between the two electrodes. The nucleation-center forming entities may serve as a nucleus for growth of a conductive substance, which eventually connects between the two electrodes thereby forming a conductive bridge between the electrodes. Thus, the system of the present invention, is highly sensitive, allowing the formation of a conductive bridge even where few, or even a single complex between a recognition moiety and a target is formed between, or on the electrodes of an assay set.

The electrodes of the system or other electrodes which are not part of the system may be used to create an electric field for directing a charged target to the recognition moiety. Thus, in accordance with an embodiment of the invention the electric or electronic circuitry is adapted for creating of such an electric field either through the same or different electrodes than the measuring electrodes. The electric field may also be of assistance in the step of forming the bridge between electrode of an assay set.

The present invention further concerns methods for assaying a target in a sample. The methods of the invention may be based on the concept of the first aspect of the system of the invention, wherein the conductive layer is formed on nucleation-center forming entities, which are deposited or bound to the complexes between the recognition moiety and the target. Thus the invention provides a method for assaying one or more targets in a sample comprising:
(a) providing an assay device having one or more assay sets at least one for each target to be assayed; each of the assay sets comprising at least two electrodes and a recognition moiety immobilized either to one or more of the at least two electrodes or on a substrate between the at least two electrodes; the recognition moiety being capable of specific binding to one of the targets;
(b) contacting said assay device with said sample under conditions permitting binding of targets to specific recognition moieties;
(c) contacting said device with a first reagent solutions to form nucleation-center forming entities on complexes formed between a target and a recognition moiety;
(d) connecting said device with a second reagent solution to grow a conducting metal substance from said nucleation-center forming entities for a time sufficient to yield a conductive bridge between said at least two electrodes;
(e) contacting said at least two electrodes to an electric or electronic module to measure conductance between said at least two electrodes; and
(f) determining conductance between said at least two electrodes, conductance above a threshold conductance indicating the presence of a respective target in the sample.

Alternatively, the nucleation centers may be attached to or deposited on the free target while still present in the sample and in that case the present invention provides a method for assaying one or more targets in a sample, comprising:
(a) reacting the targets with a first reagent solution to bind nucleation-center forming entities to said targets;
(b) providing an assay device having one or more assay sets at least one for each target to be assayed; each of the assay sets comprising at least two electrodes and a recognition moiety immobilized either to one or more of the at least two electrodes or on a substrate between the at least two electrodes; the recognition moiety being capable of specific binding to one of the targets;
(c) contacting said assay device with said sample under conditions permitting binding of targets to specific recognition moieties;
(d) contacting said device with a second reagent solution to grow a conducting metal substance from said nucleation center for a time sufficient to yield a conductive bridge between said at least two electrodes;
(e) connecting said at least two electrodes to an electric or electronic module to measure conductance between said at least two electrodes; and
(f) determining conductance between said at least two electrodes, conductance above a threshold conductance indicating the presence of a respective target in the sample.

The method may also be based on the second aspect of the method of the invention and in that case the conductive bridge is composed of a conductive polymer. Thus the invention provides a method for assaying one or more targets in a sample, comprising:
(a) providing an assay device having one or more assay sets at least one for each target to be assayed; each of the assay sets comprising at least two electrodes and a recognition moiety immobilized either to one or more of theat least two electrodes and/or on a substrate between the at least two electrodes; the recognition moiety being capable of specific binding to one of the targets;
(b) contacting said assay device with said sample under conditions permitting binding of targets to specific recognition moieties;
(c) contacting said device with a first reagent solution comprising monomers of a conductive polymer such that said monomers can bind to complexes formed between the targets and recognition moieties;
(d) treating said device such that said monomers will polymerize to form a conducting polymer, and thereby forming a conducting bridge between at least two electrodes of at least one assay set; and
(e) determining a conductance between said at least two electrodes, conductance above a threshold conductance indicating the presence of a respective target in the sample.

The method may have step (a₀) before step (a):
(a₀) reacting the sample with a second reagent solution containing entities which can form nucleation centers for growing therefrom a conducting polymer from said monomers, such that said entities bind to said targets if present in the sample.

Alternatively, the method may have step (aₗ) after step (a):
(aₗ) contacting said assay device with a second reagent solution containing entities which can form nucleation centers for growing therefrom a conductive polymer from said monomers, such that said entities bind to said targets if bound to said recognition moieties.

As explained in connection with the system, in the context of the method, the nucleation-center forming entities may be attached directly to the target or complex or may be attached to the target or complex indirectly, for example, by use of moieties such as biotin-avidin antibodies, etc.

The measure of conductance in accordance with any of the methods of the invention can be used to determine concentration as will be explained hereinbelow. In addition, a plurality of detection sites may be used, where the proportion of conductive sites is indicative of concentrations as will be explained hereinbelow.

The term *"determination"* or *"determine* " should be understood as referring collectively both to a qualitative measurement in order to deform in formation of a bridge (to achieve a *"yes"* or *"no"* answer) as well as to a quantitative measurement intended also to determine the extent of bridging.

The assay device comprises one or more assay sets each with at least two electrodes and a recognition moiety. In accordance with one embodiment, at least one electrode, and preferably two, of each assay set, have a recognition moiety immobilized thereon. Other electrodes of each assay set may have a binding moiety (namely a moiety which can bind a target in a semi specific or non specific manner) immobilized thereon or having surface properties such which allow non specific binding of the target thereto. Where recognition moiety is immobilized on at least two electrodes of an assay set, these recognition units may be the same or may be different (although binding to the same target). For example, one recognition moiety may be capable of specific binding to one epitope of the target and the other recognition moiety to another epitope. A specific example is the case of a nucleic acid sequence, where one recognition moiety comprises a sequence complementary to that of one portion of the target nucleic acid sequence and the other recognition moiety comprises a sequence complementary to another portion of the target nucleic acid sequence.

In accordance with another embodiment, the recognition moiety is immobilized on a substrate which is present between the two electrodes.

The present invention also concerns an electric device for determining one or more targets in a sample, comprising:
an integrated circuit comprising the first group of N₁ conductors and a second group of N₂ conductors, defining between them N₁xN₂ junctions, each such junction being formed with an electronic module comprising two electrodes, each one linked to or defined as an integral portion of one of the conductors, and comprises a diode or non-linear component permitting current flow through the electronic module only in the direction from the first group of conductors to the second group of conductors, whereby a current flowing between one conductor of the first group to one conductor of the second group of conductors defines a single junction point between them; each pair of electrodes forming part of an assay set, each assay set having a recognition moiety bound either to at least one of the electrodes or to a non- conducting substance disposed between the electrodes.

The electronic device of the present invention may be used for determining a plurality of different targets in a sample, determining concentration of a single target in a sample or determining the concentrations of a plurality of different targets in a sample as will be explained hereinbelow.

The electronic device of the invention may also be used to optimize *"signal-to-noise ratio",* and thus to expand a dynamic range when assaying simultaneously a plurality of targets, each being at a different concentration in the sample as will be explained hereinbelow.

Typically, the electronic device is in the form of an array when the first group of conductors is at the X axis and the second group at the Y axis and allowing multiplexing testing. The advantage of the electronic device of the invention is its small dimensions where the distance between the center of each assay set to the center of an adjacent assay set is 100 µM or less, thus allowing formation of a plurality of assay sets in a relatively small area.

The assay sets in the device of the invention may be formed as a two-dimensional array on a non-conductive substrate. Preferably, however, the electrodes of each assay set are open ends of conductive layers of a substrate which are separated from one another by non-conductive (i.e. insulating) layers. Such a device is formed from a multi-layer substrate consisting, as aforesaid from conductive layers separate by non-conductive layers, where the electrodes are formed by either boring holes or cutting openings in the substrates or cutting out portions of the substrates, in a direction essentially normal to the layers. The recognition moieties may be immobilized on the open ends of the conductive layers or in the open ends of the non-conductive layers between two open ends (electrodes) of conductive layers.

The formation of a conductive bridge when performing the assay, for detection of a target indicates the presence of the target in the sample. It will obviously be clear to the artisan, that a small degree of electric conductance, and hence electric current when electric potential is applied between the electrode, may exist between electrodes of an assay set also under control conditions (e.g. under conditions identical to the assay conditions without a target in the sample). Thus whenever the existence of currents is discussed herein, it should be understood that this means current which is larger than under control conditions. While the assay may be limited to detection of the target in an all-or-none fashion (to give a *"yes*" or *"no"* answer), according to some embodiments of the invention, the assay may also be performed for the purpose of quantitative determination of the content of the target in the sample, e.g. determining its quantity (amount or concentration).

Quantitative measurements may be carried out by using a plurality of assay sets, the recognition moieties of all are capable of binding to the same targets. All assay sets which are capable of binding to the same target are usually grouped together in the electronic device of the invention in one spatially distinct region, and the region in which they are grouped will be termed *"hybridization site".* Each individual assay set in the hybridization site will be termed hereinafter *"detection site".* The proportion of detection sites which conduct electricity (due to presence of target and consequence formation of conductive bridges) in a single hybridization site, will be proportional to the amount or concentration of the target in the sample.

If the electronic device comprises a plurality of hybridization sites (i.e. plurality of groups of assay sets, where the sets in each group all bear recognition moieties for binding to the same target) the system can simultaneously assay for a plurality of different targets and, by determining the proportion of detection sites which are conductive in one hybridization site, can also simultaneously give the amount of each target in the sample.

Furthermore, since as indicated above, some assay sets (i.e. detection sites) in a hybridization site may be conductive even in the absence of the targets, the fact that a plurality of detection sites is available for each hybridization site (suitable for the detection of one target) allows to improve the *"noise-to-signal ratio".* Only when a predetermined proportion of detection sites, which proportion is above a certain threshold value, will conduct electric current, this will then be regarded as indicating a positive result (namely presence of the target in the sample). This threshold is not necessarily identical for all hybridization sites. For example for one hybridization site for assaying a target which is predicted to be abundant in a sample the threshold may be set to 50% of detection sites (assay sets) being conductive, while for another target which is predicted to be rare in the sample the threshold may be set to 15% of detection sites being conductive. Thus the *"dynamic range"* of the electronic device of the invention may be quite broad.

It is also possible to form the device with a plurality of hybridization sites all of which are designed for assaying the same target, although with different sensitivities: for example, one site may be formed with a high amount of recognition moieties, or with moieties with a higher affinity to the target which will give rise to a signal even in the presence of a low concentration of the target in the sample; while other sites may be formed with a lower amount of the recognition moieties or with recognition moieties having a lower affinity to the targets, thus allowing a quantitative determination of the targets over a concentration range as compared to the former sites. Such a device then enables the use of a single device for assaying many different samples where the concentration of the target may vary from one sample to the other by several folds.

A device in accordance with the of the invention may comprise one or a plurality of assay sets. In case of a plurality of assay sets all may have the same target specificity i.e. may have a single hybridization site with a plurality of detection sites (namely the recognition moieties of all detection sites will bind the same target) in which case, as pointed out above, the extent of bridging between electrodes in different assay sets (detection sites) may then serve as a quantitative measure for the concentration of the target in a sample. Alternatively, the device may comprise two or more groups of assay sets (hybridization sites) each characterized by having recognition moieties with a different target specificity, which render the device useful in a multiplexing assay for assaying a panel of corresponding two or more different target entities (e.g. different nucleic acid sequences).

For example, each assay set or a group of assay sets may be specific for a different nucleotide sequence. Such an electronic device may be useful in a variety of multiplexing diagnostic applications, i.e. simultaneous detection of a number of targets, detection of an unknown target which is then characterized by its binding (and hence bridge formation) to one of the recognition moieties in the different assay sets (e.g. randomly prepared nucleic acid sequences, each forming the recognition moiety in a different assay set). A specific example is the case of high throughput assays, for example, such aimed at finding a molecule, e.g. peptide, which specifically binds to another molecule, e.g. a receptor; assays for sequencing of unknown nucleotide strands (e.g. genome sequences); etc. In the case of a sequencing assay, a plurality of different sequences may be attached by linkers to electrodes of different assay sets and then a bridge formed in a specific assay set (where the specific nucleic acid sequence serves as the recognition moiety) identifies the specific sequence.

The target may be any one of a wide variety of entities including proteins, nucleic acid sequences, peptides, organic molecules, large macromolecular complexes, cell membranes, and many others. In case of a large entity such as a cell, cell membrane, viral particle, macromolecular complex, etc., the entire large entity, or only the portion thereof binding to the recognition moiety may be regarded as the target. For example, in the case of microorganism, the entire microorganism may be regarded as a target, or at times, where the recognition moiety binds to a specific antigen on the surface of the cell, such an antigen may then be regarded as the target.

Depending on the nature of the target, the recognition moiety may be a single-stranded nucleic acid sequence or a double-stranded sequence with a sticky end, an antibody, a receptor, a lectin, a sugar, an antigen, etc. The recognition moiety and the target are thus members of an affinity group: one member of the affinity group is the target, the other member of the group serves as the recognition moiety.

The recognition moiety may be immobilized on the electrode and/or on a non-electrode substrate present between the electrodes, by the use of linkers which may be selected from a wide variety of molecules capable of attachment to a solid substrate on the one hand, and covalently binding or complexing to the recognition moiety on the other hand.. Examples are a variety of sulfur-containing molecules which can, through their sulfur-containing moiety, beam associated with a metal substrate such as gold, silver, platinum, etc. Such a linker may be covalently bound to the recognition moiety, or may be complexed thereto.

The measurement of electric conductance between an electrode of a set may be performed directly, by measuring current-potential relationship, or by performing other measurements indicative of the passage of current through the bridge. For example, the bridge may be treated in a manner that in addition to conducting current, it also emits light, in which case the electric connectivity of the bridge may be determined according to light emission.

The invention will now be illustrated in the following detailed description with occasional references to the annexed drawings. As will be appreciated, the description below is exemplary and should not be construed as limiting the scope of the invention as defined in the appended claims.

### BRIEF DESCRIPTION OF THE DRAWINGS

**Fig. 1** is a schematic illustration of an assay device in a manner of performing the assay in accordance with an embodiment of the invention;
**Fig. 2** is a schematic illustration of an assay device in a manner for performing the method in accordance with an embodiment of the invention;
**Figs. 3A-3E** show a different combination of recognition moieties, immobilized on at least one electrode of an assay device for the detection of target entities, in accordance with several different embodiments of the invention;
**Figs. 4A, 4B** and **4C** are schematic illustrations of three embodiments of the invention where the recognition moiety is immobilized on a support member which is other than an electrode;
**Fig. 5** is a schematic illustration of an assay device and the performance of a method in accordance with an embodiment of the invention, involving functionalization of the bridge.
**Fig. 6** is a schematic illustration of an embodiment of the invention where the concentration of the target can be determined;
**Fig. 7** is a schematic illustration of another embodiment of the invention for determining concentration of the target in the sample.
**Fig. 8** is a schematic illustration of a multiplexing embodiment of the invention for detection of a variety of different target entities;
**Fig. 9** is a schematic illustrating of an embodiment of the invention where each two adjacent assay sets share an electrode;
**Fig. 10** illustrates an assay device and method for the detection of a DNA sequence in a sample;
**Fig. 11** shows two exemplary current-voltage relationship of a functionalized bridge formed after metal deposition on a bridge-forming target as illustrated in Fig. 10;
**Fig. 12** illustrates an assay device and method for the detection of a DNA sequence in a sample where the bridge is functionalized by deposition of poly-p-phenylene vinylene (PPV);
**Fig. 13** illustrates another embodiment of functionalizing a nucleic acid bridge;

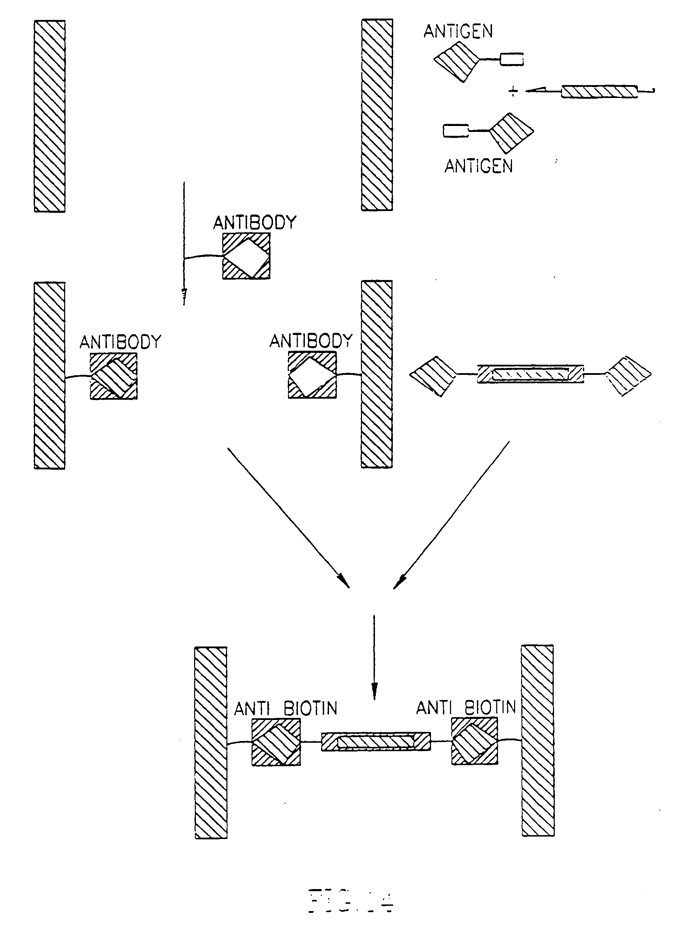

**Fig. 14** shows an embodiment of the invention for assaying of an antigen;
**Fig. 15** illustrates an embodiment of immobilization of oligonucleotide recognition moieties onto the electrodes;
**Fig. 16** shows a scheme for synthesizing an oligonucleotide, as described in Example 1(a);
**Fig. 17** shows a fluorescently labeled λ-DNA bridge stretched between two gold electrodes (dark strips) 12 µm apart;
**Fig. 18** shows atomic force microscope (AFM) images of a DNA bridge coated by silver connecting two gold electrodes 12 µm apart 1.5 µm and field size;
**Fig. 19** is two terminal I-V curves of a DNA bridge coated by silver prepared according to Example 8. The arrows indicate the voltage scan direction. The solid-line curves are repeated scans and demonstrate the stability of the samples. Note the different asymmetry in the I-V curves corresponding to the two scanning directions;
**Fig. 20** shows the I-V curves of a different silver wire in which the silver growth was more extensive than in Fig. 19. The more extensive silver growth resulted in a smaller current plateau, on the order of 0.5V, and a lower resistance (13MΩ vs. 30 MΩ in Fig. 17). By driving large currents through the wire, the plateau has been eliminated to give an ohmic behavior (dashed line), over the whole measurement range;
**Fig. 21** shows a schematic representation of the steps of performing a detection assay for the presence of a nucleic acid sequence in a sample;
**Fig. 22** shows a schematic representation of the steps of a method for preparation of a chip for nucleic acid attachment;
**Fig. 23** shows a schematic representation of the steps of a method for covalent attachment of nucleic acid probes to the chip produced by the method described in Fig. 22;
**Fig. 24** shows a schematic representation of an assay set comprising two electrodes being open ends of conductive layers which are separated from each other by the open ends a non-conductive (insulating) layer;
**Fig. 25** shows a schematic representation of a process for attaching a biotin group to target nucleic acids in a sample;
**Fig. 26** shows schematically hybridization between biotin-containing nucleic acid targets in a sample and recognition moieties on a chip;
**Fig. 27** shows essentially the same as Fig. 26, wherein the recognition moieties are present on electrodes of Fig. 24;
**Fig. 28** shows schematically attachment of avidin-containing nucleation-center forming entities to biotin-containing targets which are present in a target-recognition moiety complex;
**Fig. 29** shows essentially the same as Fig. 28, wherein the complexes are present on the electrodes of Fig. 24;
**Fig. 30** shows schematically the process of deposition of gold in one assay set comprising two electrodes;
**Fig. 31** shows three AFM pictures of a chip which underwent a process of contact with sample, attachment of nucleation centers and exposure to reagents allowing formation of gold crystallization wherein:
**Fig. 31(A)** shows a chip lacking DNA binding moieties; Fig. 31(B) shows a chip having binding moieties which are partially complementary to sequence of target in a sample; and Fig. 31(C) shows a chip having recognition moieties which are fully complementary to target sequences;
**Fig. 32** shows AFM pictures of a single assay set comprising electrodes bridged by gold particles (right top) or non bridged by gold particles (left top) and corresponding current voltage curves (right bottom and left bottom, respectively);
**Fig. 33** shows an electronic detection device having a multiplexing array;
**Fig. 34** shows schematically a multiplex array of electronic detection device wherein each hybridization site comprises a plurality of detection sites;
**Fig. 35** shows a microelectronic embodiment of multiplex DNA array of Fig. 33;
**Fig. 36** shows a detailed view of cross section in plane A of Fig. 35; and
**Fig. 37** shows a detailed view of a cross-section similar to the one shown in Fig. 36 with minor alterations used in a microfluidic system.

### DETAILED DESCRIPTION OF THE INVENTION

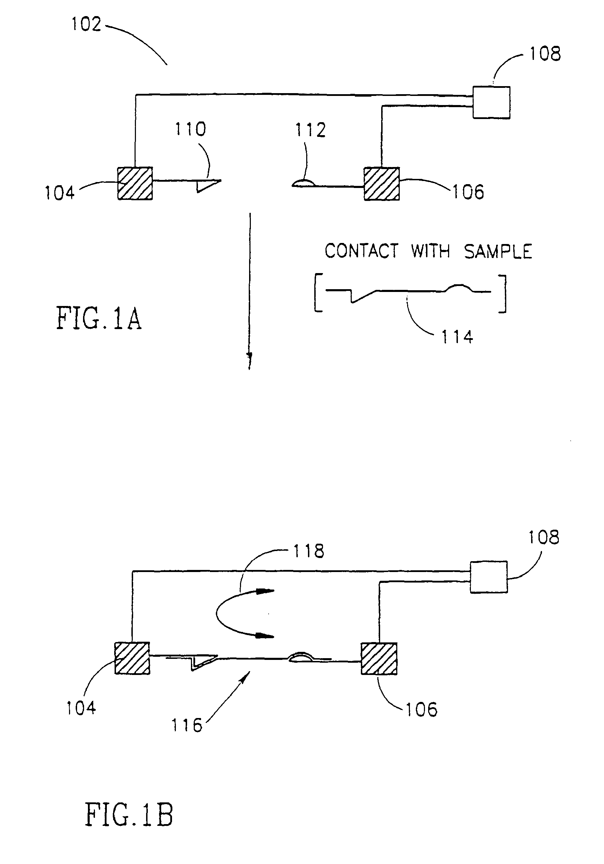

Reference is first being made to Fig. 1, illustrating an assay device **102** which forms part of the system of the invention consisting of a single assay set with two electrodes **104** and **106** connected to one electric or an electronic circuitry **108.** Immobilized on electrodes **104** and **106** are recognition moieties **110** and **112.** In (a) there is no electric contact between electrodes **104** and **106.**

When the assay device **102** is contacted with a sample comprising a target **114,** a path **116** forms between the two electrodes **104** and 106. By subsequent steps (see below) a conductive bridge is formed and current can flow through the bridge between the two terminals of module **108,** as represented by the B-directional arrow **118 (b).** In the embodiment shown in Fig. 1, the assay set comprises two electrodes.

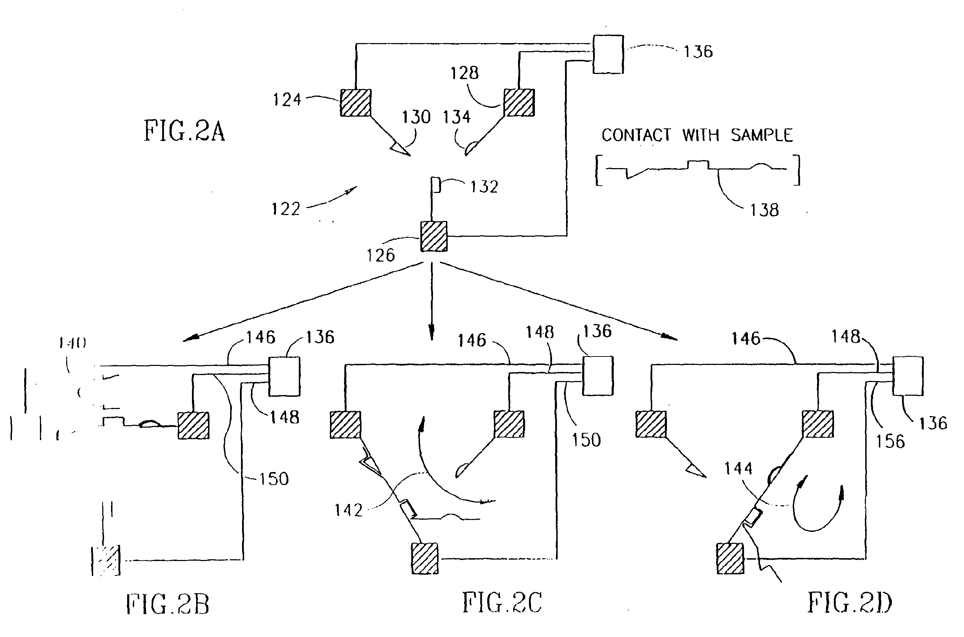

In Fig. 2, a different embodiment is illustrated, where an assay set **122** consists of three electrodes **124, 126** and **128** having three different recognition moieties **130, 132** and **134,** respectively, immobilized thereon. These three electrodes are connected to an electric or electronic control module **136.** Each of the immobilized recognition moieties **130, 132** and **134,** can bind to a different region in the target **138.**

When the device (a) is contacted with the sample containing the target **138,** target entities can bind to the different electrodes in one of the manners illustrated schematically under (b1), (b2) and (b3). Following subsequent steps for yielding a conductive bridge, current can flow through the formed conductive bridges as illustrated by arrows **140, 142** and **144.** Measurement of current flow in either one of the formed circuits, namely, between terminals **146-148, 146-150** or **148-156** of module **136,** yield information on the target.

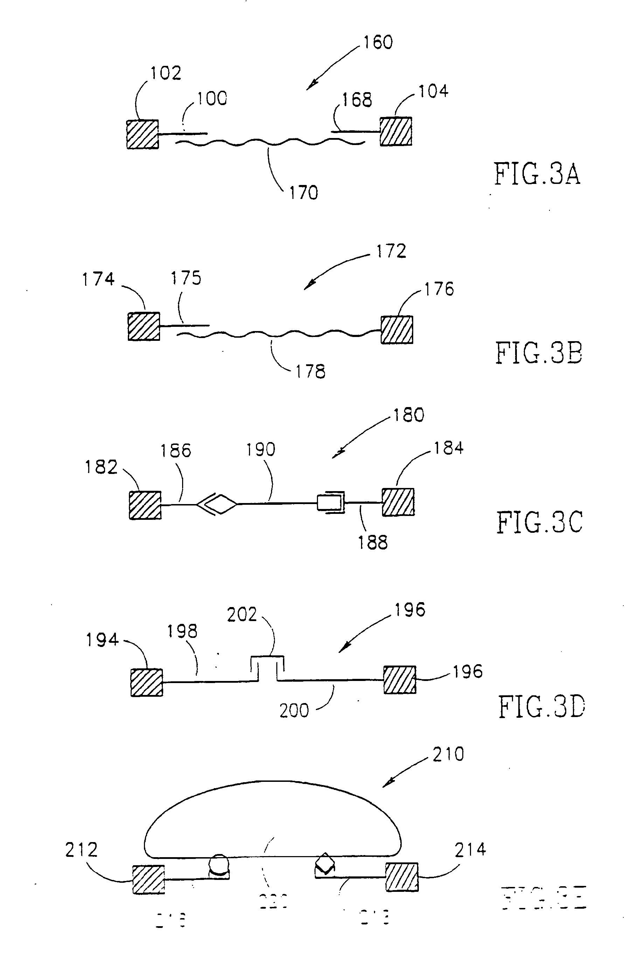

Figs. 3A-3E show different configurations of assay sets in accordance with different embodiments of the invention. In assay set 160, shown in Fig. 3A, each of electrodes **162** and **164** has immobilized thereon a recognition moiety **166** and **168,** respectively. For example, recognition moieties **166, 168** may be oligonucleotides complementary to terminal sequences in a target nucleic acid sequence **170.**

In Fig. 3B, of the two electrodes **174** and **176** of assay set **172,** only the former has immobilized thereon a recognition moiety 175, e.g. an oligonucleotide which is complementary to terminal sequence of target nucleic acid molecule **178.** Target nucleic acid molecule **178** binds specifically to recognition moiety **175** and then non-specifically or semi-specifically to electrode **176.**

Fig. 3C, illustrates an assay set **180** which has two electrodes **182** and **184** with immobilized recognition moieties **186** and **188,** which in this specific embodiment are different antibodies, recognizing different antigenic epitopes of a target **190.**

Assay set **192** shown in Fig. 3D, has two electrodes **194** and **196** each of which has immobilized thereon a relatively long oligonucleotide **198** and 200, respectively, the terminal sequence of which constitutes the recognition moiety for the target, in this specific case a short oligonucleotide **202.** Target oligonucleotide **202** thus brings together two oligonucleotides **198** and **200.**

Assay set **210** shown in Fig. 3E consists of two electrodes **212** and **214** having each a recognition moiety **216** and **218,** respectively, immobilized thereon. Each of these recognition moieties binds to an epitope on the external surface of a cell **220.**

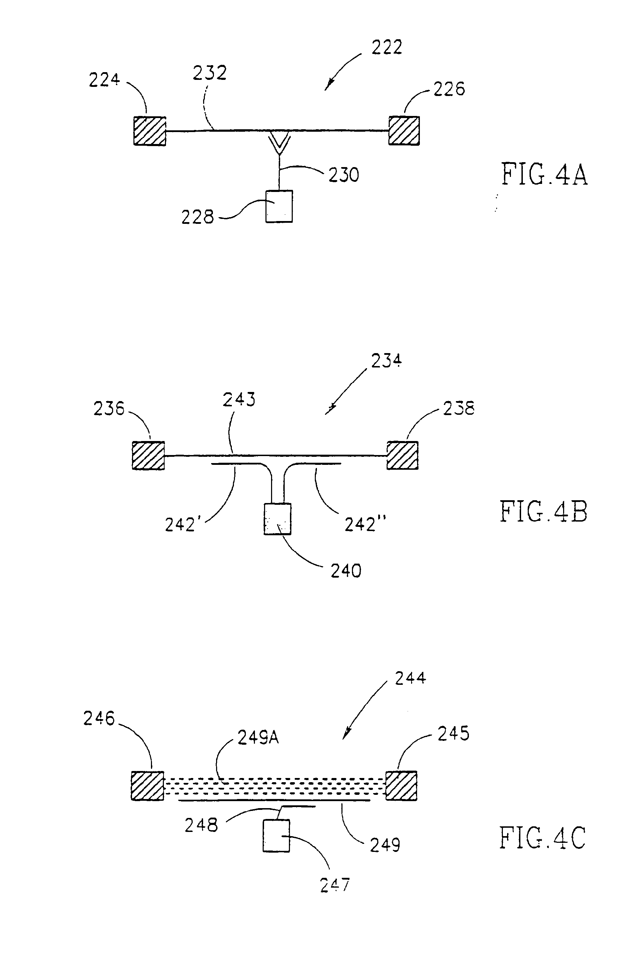

In all the embodiments shown in Figs. 3A-3E, a recognition moiety is immobilized on at least one electrode of an assay set. Against the case of the embodiments of the assay sets **222** and **234** and **244** shown in Figs. 4A-4C, no recognition moiety is immobilized on the respective electrodes **224** and **226, 236** and **238** and **245** and **246.** Rather, in this case, each of the assay sets **222, 234** and **244** have a substrate member **228, 240** and **247,** respectively, which are other than the electrodes, on which the respective recognition moieties **230, 242'** and **242"** and **248** are bound. In the case of assay set **222,** immobilized on member **228** is a single recognition moiety **230,** which in this specific embodiment is an antibody directed to an epitope of target **232** (the target may be a nucleic acid sequence, a polymer, a polypeptide, etc.). In the case of assay set **234,** member **240** has immobilized thereon two oligonucleotide substrates **242'** and **242"** which are complementary to portions of target nucleic acid sequence **244.** In both cases, after binding to the recognition moieties, a conductive bridge between the two electrodes of the assay sets is formed by a non-specific or semi-specific binding or association and typically by growth of a conductive layer from nucleation centers present on the targets or on complexes between targets and recognition moieties. In the case of assay set **244,** after the oligonucleotide target **249** binds to the recognition moiety **248,** it serves a template for synthesis of other nucleic acid sequences, and this synthesis eventually forms a path **249A** between the two electrodes **245** and **246.**

Each of the assay sets shown in Fig. 4A-4C, have two electrodes. It will readily be understood that the illustrated embodiments apply also to the case of more than two electrodes in each assay set.

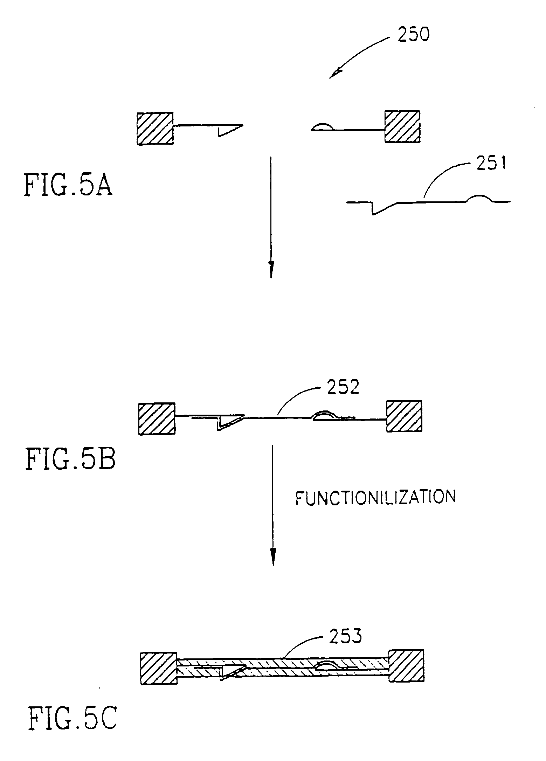

Fig. 5 is a schematic illustration of a manner of performance of the method in accordance with an embodiment of the invention involving steps for formation of a conductive bridge. Assay set **250** (a) is contacted with a target **251** to form a path **252** (b). After the various steps (as will be explained hereinbelow), a conductive bridge **253** is formed (c).

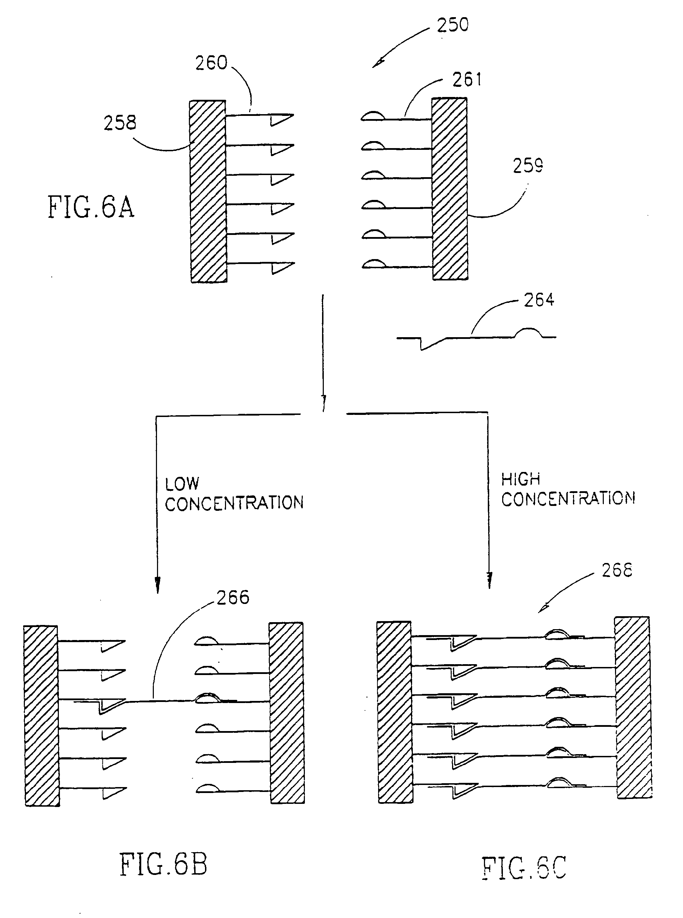

Fig. 6 shows the manner of determining concentration of a target in a sample in accordance with an embodiment of the invention. Each of electrodes **258** and **259** of assay set **256,** has immobilized thereon a plurality of recognition moieties **260** and **261,** respectively. After contact with a target **264,** one or more paths between the electrodes form. In a case of low concentration of the target **264** (b1), a small number of paths forms in a given time period (illustrated here by a single path **266)** and in the case of a high concentration (b2), a large number of paths, illustrated here by six paths **268** formed within the same time period. After steps for processing the paths to yield conductive bridges are carried out, it is clear that the measured resistance during the same time period is lower in the case of a high concentration as compared to a low concentration. This difference in the potential/current relationship can thus serve as a measure (after proper calibration) of concentration of the target **264** in a sample.

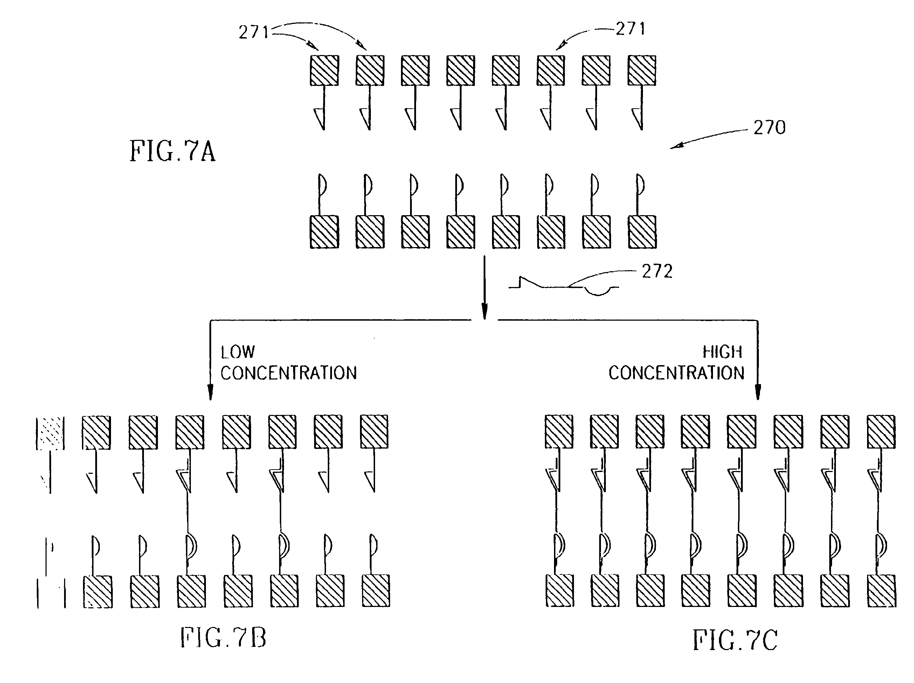

Fig. 7 is a schematic illustration of a device **270** having a plurality of assay sets **271,** each comprising two electrodes and having the same recognition moieties immobilized thereon. When the naive assay device **270** is contacted, for a given time period, with a target **272,** in the case of a low concentration, paths between the different electrodes of each assay set forms only in a few assay sets, whereas in the case of a high concentration (b2) bridges form in many (at times all) After steps for yielding a conductive bridge are carried out, counting the number of units where current is detected, indicates the concentration of the target **272** in the sample.

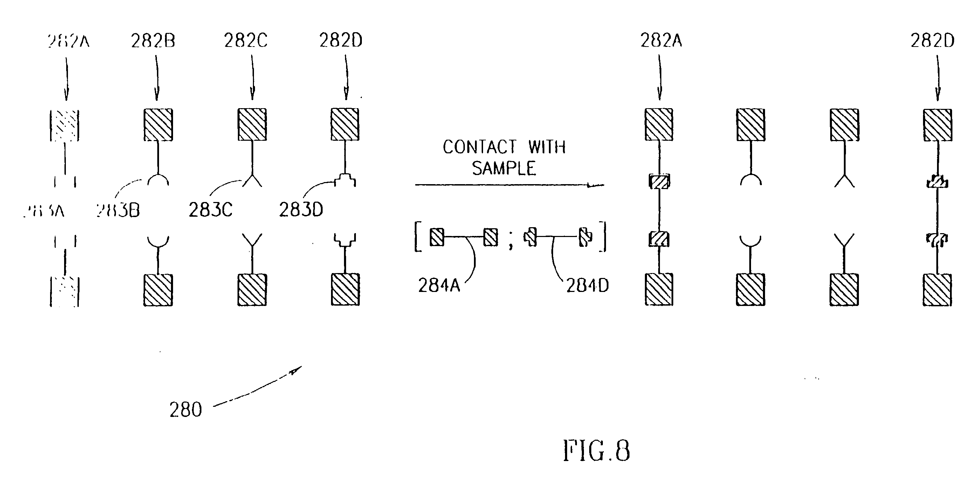

Fig. 8 illustrates a multiplexing embodiment, where each assay set or a group of assay sets is designed for binding a different target. This allows a diagnostic assay for the simultaneous detection of a number of target entities in a sample. In the illustration, each of the assay sets or group of assay sets **282A-282D,** have a different target specificity, as illustrated by the different shapes of the immobilized recognition moieties **283A-283D** respectively. When the assay device **280** is contacted with a sample, comprising, for example, target entities **284A** and **284D,** paths form only in assay sets **282A** and **282D** which, after forming then to conductive bridges, then serves as an indication of the presence of the respective targets in the sample.

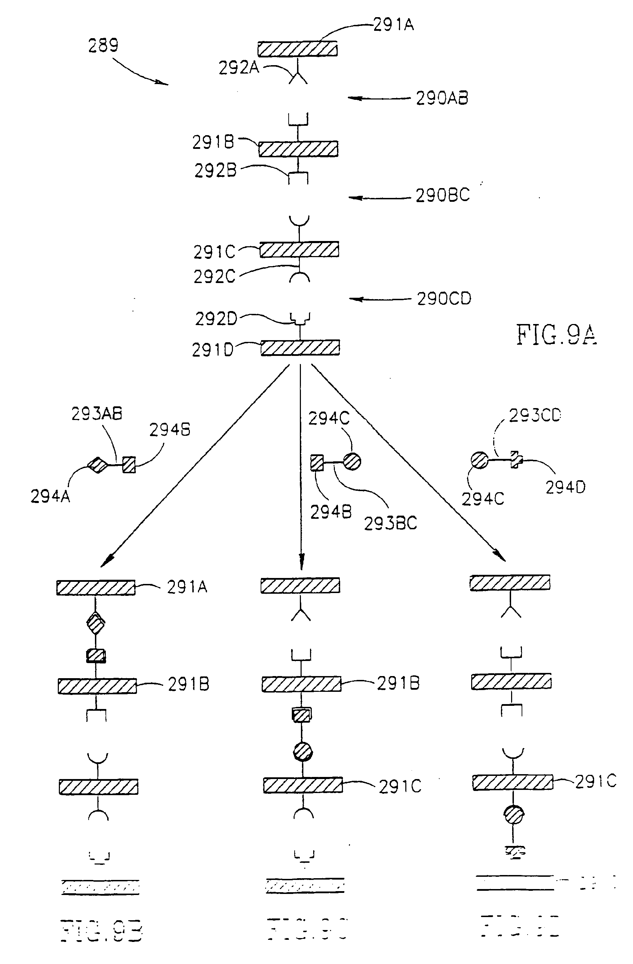

In all assay devices illustrated in Figs. 1-8, each assay set has its own electrodes. At times, however, two or more assay sets may have common electrodes. In a sense, the assay set **122** of Fig. 2 can be viewed as three assay sets with each two electrodes defining a different assay set. An illustration of an embodiment where each two adjacent assay sets share an electrode can be seen in Fig. 9. As illustrated in (a) of this figure, the assay device **289** has a plurality of assay sets of which three, **290AB, 290BC** and **290CD,** can be seen. Seen in this illustration are four consecutive electrodes, **291A, 291B, 291C** and **291D** having immobilized thereon respective recognition moieties **292A, 292B, 292C** and **292D.** Each two adjacent electrodes define one of the assay sets, e.g. electrodes **291A** and **291B** defining assay set **290AB,** with each two adjacent assay sets sharing a common electrode: assay sets **290AB** and **290BC** sharing electrode **291B** and assay sets **290BC** and **290CD** sharing electrode **291C.**

The recognition moieties **292A, 292B, 292C** and **292D** bind to respective epitopes **294A, 294B, 294C** and **294D.** Thus assay set **290AB** will be specific for target **293AB** having epitopes **294A** and **294B,** assay set **290BC** will be specific for target **293BC** having epitopes **294B** and **294C** and assay set **290CD** will be specific for target **293CD** having epitopes **294C and 294D.** Consequently, when contacted with a sample, a bridge will form in an assay set, depending on the type of target in the sample as illustrated under (b1), (b2) and (b3).

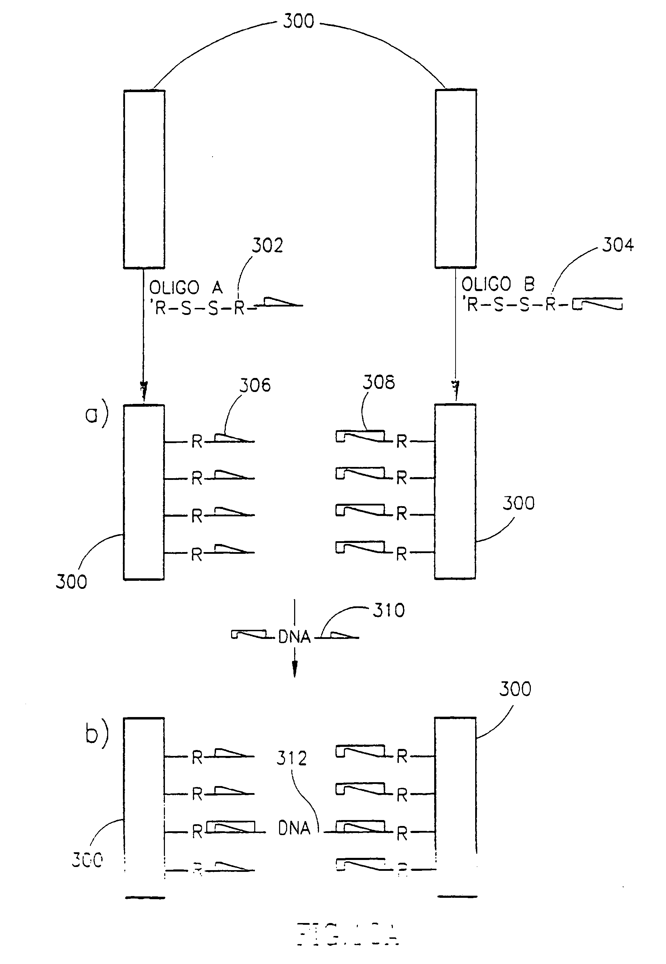

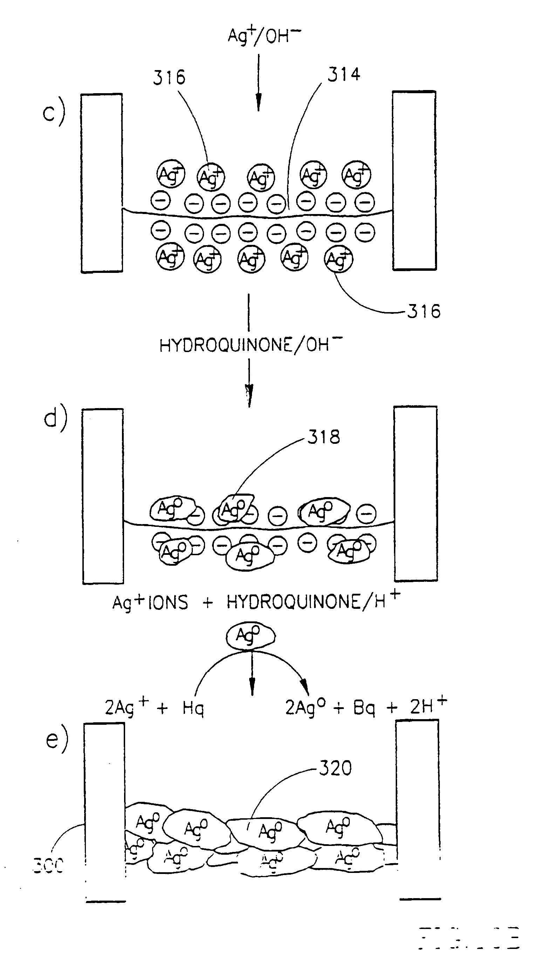

Reference is now being made to Fig. 10, illustrating an assay device and method for detection of a specific target DNA sequence **310** in a sample. The detection is carried by formation of a path **312** between two electrodes 300, which is then typically coated by metals such as gold, platinum, silver, etc. to eventually yield a conductive bridge **320.** For the formation of the assay device, electrodes **300** may be first treated in a manner to facilitate subsequent binding of molecules **302** and **304.** For this purpose the electrodes may be first wetted separately with a solution containing either molecules **302** or **304,** each being a single-stranded oligonucleotide, which serves as a recognition moiety *("Oligo A"* and *"Oligo B",* respectively), derivatized by a disulfide group for attachment of molecules **302** and **304** to the electrodes. Oligonucleotides **302** and **304** are each complementary to one of the two terminal sequences of the target **DNA sequence 310.** When these recognition moieties are deposited on electrodes **300,** under appropriate conditions, the disulfide group bind to the electrodes **300,** to form recognition moieties.

For detection of the target in a sample, the assay device is contacted with a sample suspected of comprising a target nucleotide sequence, in this specific example a single or a double-stranded DNA sequence **310.** The sample may be a blood sample, a food sample, a water sample, etc. In the case where the target is a double-stranded sequence, the target in the sample may be treated to form single-stranded sticky ends at the terminals of a double-stranded target DNA **310.** These sticky ends are complementary to the sequences of the oligonucleotides in recognition moieties **306** and **308.** Electrodes **300** are spaced from one another at a distance which should not exceed the combined length of the target DNA **310** and the recognition moieties **306** and **308.** When the electrodes are contacted with a sample comprising target DNA sequence **310,** the target terminal ends connect to their complementary oligonucleotide sequence in the recognition moieties **306** and **308** to form a path **312** between the two electrodes **300** (step (b)). In the case of targets which are double-stranded, following hybridization, the binding of the recognition moieties **306** and **308** to the target nucleic acid sequence **310** may be strengthened by ligating the nicks to form covalent binding.

At times, particularly where sequence **310** is long, it may not be practical to rely on diffusion for hybridization of the target **310** to the recognition moieties **306** and **308.** In such a case, the target **310** may be made to connect to one electrode and then, by a directional stream of fluid from the first electrode to the second, or by applying an electric field the nucleic acid bridge is made to extend so that its end reaches the second electrode.

It is also clear that in order to avoid folding of the nucleic acid molecules and to ensure proper binding, appropriate solutions may at times be needed. In addition, after hybridization, rinsing may at times be required in order to remove unbound nucleic acid strands.

The formation of a conductive bridge between the electrodes begins, according to the specifically illustrated embodiment, by an ion exchange step involving exposure of the nucleic acid fiber to an alkaline solution comprising silver ions (Ag⁺). The silver ions replace the sodium ions or other ions normally associated with the nucleic acid molecule and complex with the negatively charged nucleic acid sequences (step (c)). (It should be noted that Ag⁺ ions may also be made to bind to nucleic acid molecules in various other ways in particular by intercalation). These steps give rise to a nucleic acid sequence **314** loaded with silver ions **316.** It should be noted, that rather than silver ions, a wide variety of other ions can also be used, including for example, cobalt, copper, nickel, iron, gold, etc. Furthermore, metal aggregates, semiconductor particles, complexes or clusters, e.g. colloidal gold, colloidal silver, gold clusters, etc., may also be deposited on the nucleic acid sequence via a variety of different interactions. Conductive oligomers and polymers may also serve to render the nucleic acid bridge conductive.

At a next step (step (d)), the sequence is exposed to a reducing agent, e.g. hydroquinone, or to an electromagnetic radiation, to reduce the metal ions *in situ* into metallic silver which forms nucleation sites **318.** In a different embodiment metal nucleation centers may be formed by attaching a host of colloids or clusters to the DNA path in a sequence specific or non-specific manner. After rinsing a reagent solution comprising metal ions and a reducing agent, e.g. hydroquinone under acidic conditions, is added. Under these conditions, the ions are converted to metal only at nucleation sites and consequently the nucleation centers grow and merge with each other to form a conductive functionalized bridge **320** (step (e)).

The so formed functionalized bridge **320** may be subjected to a variety of post fabrication treatments, which may include, for example, thermal treatment intended to increase the bridge's thickness and homogeneity; passivation treatment for the purpose of forming an electrically insulating layer around the bridge, e.g. by exposure to alkane thiol; electrochemical or photochemical coating of the wire using polymers.

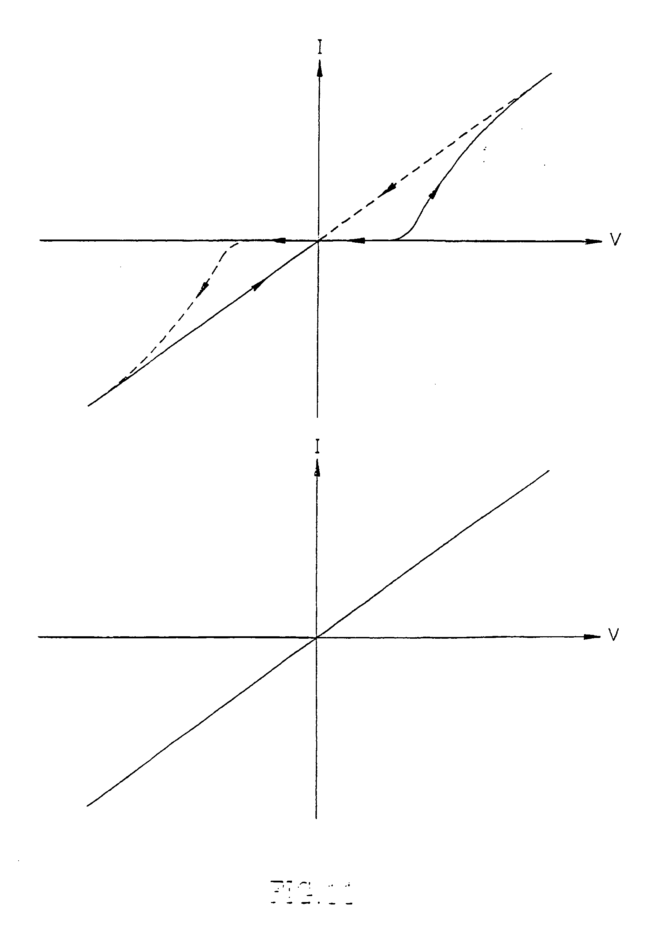

Fig. 11 illustrates two exemplary current-voltage relationships of a conductive bridge formed by the procedure illustrated in Fig. 9. Different current-voltage relationships may be obtained depending on the type of conductor and the functionalization process, etc.

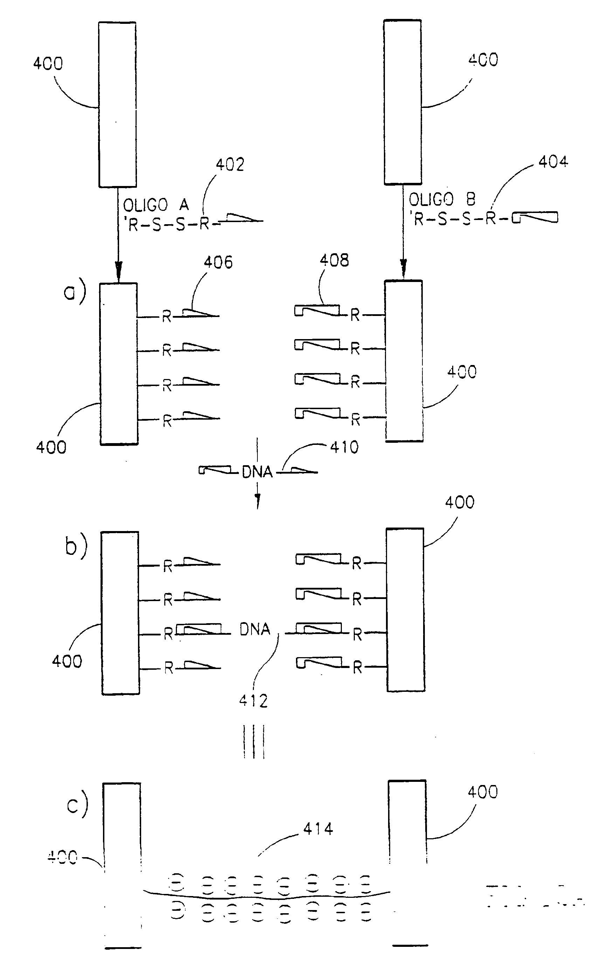

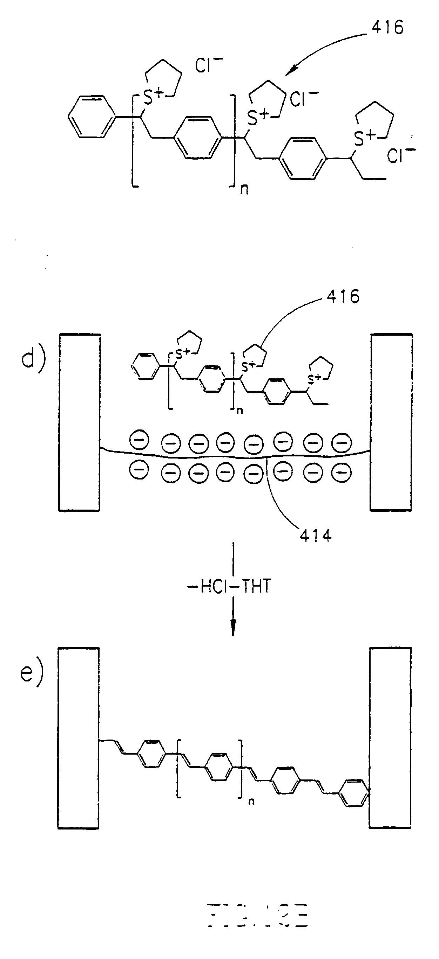

Reference is now being made to Fig. 12, illustrating a device and method of the invention wherein the conductive bridge is formed by depositing a conductive polymer PPV, (poly-*p*-phenylene vinylene). Electrodes **400,** may be the same as electrodes **300** shown in Fig. 10. The first two steps of the detection method (steps (a) and (b)) may be identical to the corresponding steps in Fig. 10 (identical components have been given a reference numeral with the same last two digits as the corresponding components in Fig. 10: e.g. **402** is the same as **302, 404** as **304,** etc.). The formed path **412** may be strengthened, similarly as above, by covalent binding of path **410** to the recognition moieties **406** and **408** to yield a path **414** connecting the two electrodes (step (c)).

A solution comprising pre-PPV molecules **416** is then contacted with path **414.** By the virtue of being positively charged, pre-PPV **416** becomes complexed with the negatively charged DNA path **414** (step (d)). At a next step, conjugation is induced by removal of tetrahydrothiophene groups and hydrochloric acid from each repeat unit, yielding a luminescent PPV bridge (step (e)). This component is suitable for optical detection. Alternatively, the PPV may be doped with agents which either cause electron deficiency (holes) or give rise to extra electrons and thus converted into a conducting polyer. Doping may be performed by many known methods e.g. exposure to H₂SO₄ vapor. The extent of doping determines the conductivity of the PPVwire.

Many other conductive polymers may be used instead or in addition to PPV in accordance with the invention. This includes a variety of polymers with positively charged side groups as well as polymers with positively charged groups in the backbone, polymers with recognition groups capable of binding to nucleic acid fibers or polymers that complex with DNA. In addition in a similar manner, *mutatis mutandis,* other types of conducting substances (n-type or p-type) may be bound to the fiber.

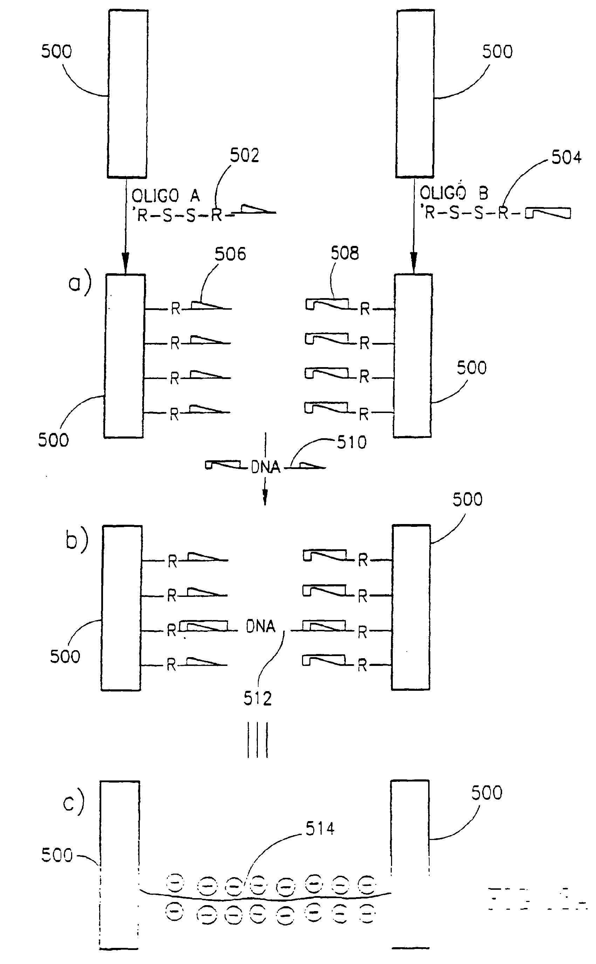

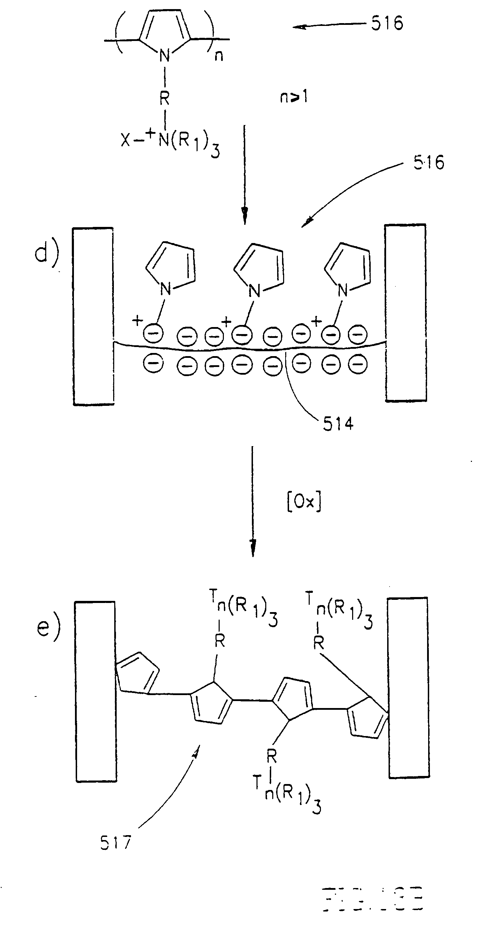

Yet another embodiment of the invention is illustrated in Fig. 13. In this figure, identical components to those of Fig. 12 are shown by numerals having the same two last digits. Nucleotide bridge **514** formed by the recognition process described with reference to Fig. 12, is exposed to a solution containing a monomer oligomer, or polymer **516.** As a result, ion exchange or other complexing occurs, leaving the nucleotide bridge **514** loaded with substance **516.** Polymerization step is then applied to form the conductive bridge **517** connecting the electrodes concerning detection of an antigen by use of an antibody. A doping process renders the functionalized bridge conductive.

### EXAMPLES

### Example 1: Preparation of Linkers between the Electrodes and the Recognition moiety

### (a) Disulfide based linkers:

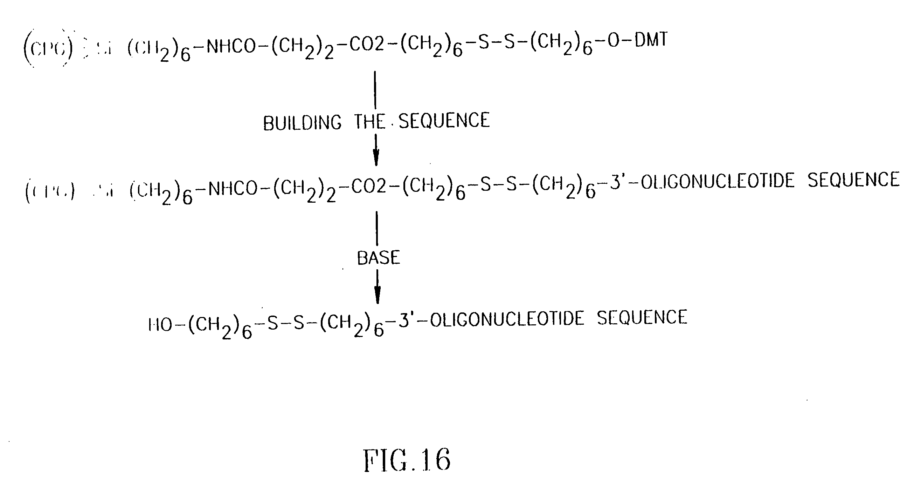

Controlled pore glass (CPG) derivatized with a disulfide group is used for the synthesis (starting from its 3' side) of an oligonucleotide having a free 5' site which serves as the recognition moiety. The oligonucleotide is prepared using a conventional DNA synthesizer (see scheme in Fig. 16).

### (b) Thiol-based linkers:

Linkers are being prepared according to (a) above and the disulfide bond is cleaved to obtain a free thiol.

### (c) Biotin-streptavidin complex based linkers:

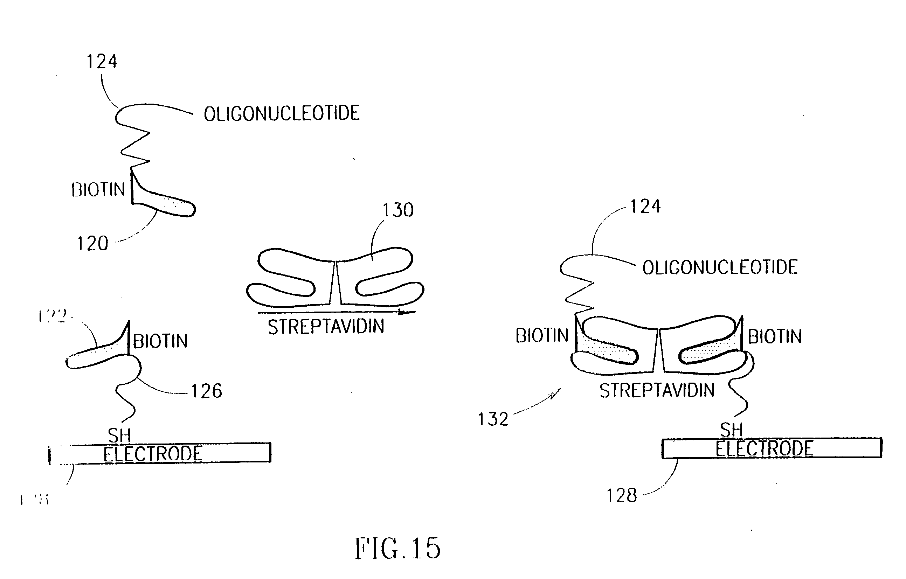

Biotin moiety is attached to an oligonucleotide having a specific sequence, as known *per se* which will serve as the recognition moiety. The biotin-oligonucleotide is coupled via a streptavidin molecule to another molecule containing a biotin moiety at one side (see also Fig. 15) and a thiol or disulfide group on the other side.

### (d) Repressor based linkers:

A nucleic acid binding protein, such as the *lac* repressor, is covalently attached to a thiol group. A DNA sequence, serving as the recognition moiety is synthesized containing also the specific sequence to which the repressor binds. The DNA is coupled to the repressor through said specific sequence.

### (e) Thiophosphate based linkers:

The construction (starting from its 3' side) of an oligonucleotide, serving as a recognition moiety, is carried out using a conventional DNA synthesizer wherein thiophosphates containing-nucleotides are used instead of standard nucleotides.

### (f) Artificial site specific based linkers:

A synthetic site-specific moiety such as, for example Rh(Phen)₂Phi, known to bind 5'-pyr-pyr-pur-3 sequence (pyr = pyrimidine, pur = purine), is covalently coupled to a thiol group.

### Example 2: Attachment of the linker to an electrode

### (a) Micropipette Wetting:

Electrodes are exposed to solutions of the appropriate linkers, for example, by employing pipettes or micropipettes or by any liquid dispensers. Such liquid dispensers may be fixed onto a manipulator that may be computer controlled. Different types of linkers can be deposited on each electrode. Additionally, different types of linkers can be deposited simultaneously or sequentially on different electrodes.

### (b) Jet printing:

Ink-jet like printing techniques are used for the selective exposure of different electrodes to different linkers. By utilizing such a technique, it is possible to attain high precision, resolution, and to increase rates of production, facilitating large scale production.

### Electrode-linker synthesis:

### (c1) Using selective masking techniques:

The well developed technology used for synthesizing DNA sequences may be harnessed for the *ab-initio* preparation of a complex electrode-linker array. For example: a substrate composed of an assay set of electrodes on an inert substrate is partially coated with an inert coating yielding two types of electrodes: coated electrodes (A) and uncoated electrodes (B). The substrate is exposed to a solution of a thiol linker linked to a nucleic acid sequence serving as a seed for DNA synthesis of a sequence which will eventually serve as the recognition moiety. Due to the inert coating, only the uncoated B electrodes react with the thiol. Using standard DNA synthesizing techniques, a pre-defined sequence, being the recognition moiety, is produced on the B electrodes. The substrate is then rinsed and the masked electrodes are uncovered followed by the selective coating of B electrode. This procedure allows the production of two types of electrodes differing one from the other by the type of recognition moieties bound thereto. The same technique with some additional steps (several steps of masking and unmasking) allows the fabrication of various substrates having many different electrodes with different recognition moieties bound thereto.

### (c2) Using photodeprotection techniques:

This approach involves the utilization of photolabile groups for the protection of the start point of DNA synthesis. Inactivated start point groups are unable to react with nucleotides. Using selective irradiation by means of a mask and/or a light conductor and/or any other addressable light source, the activation of different selected electrodes is achieved by the photoremoval of protecting groups from the DNA synthesis seeds on selected electrodes.

### (c3) Using blockers:

Using the masking technique ((cl) above) an assay set of electrodes is prepared for oligonucleotide synthesis. Once a DNA sequence, which serves as the recognition moiety, is completed on one assay set of electrodes, a terminating group (blocker) is attached to the oligonucleotide ensuring their inertness. Other sequences can be further synthesized on different electrodes that are prepared according to the previous step but become active according to this step. It should be noted that the assay set of linkers constructed in the previous step is not affected due to the blockers attached to their end points.

### (c4) Electrode printing:

Recognition moieties are attached to conducting beads such as gold colloids. The colloids are then dispersed in a controllable manner to form conducting metal pads with linkers and recognition moieties attached thereto. Dispensing may be achieved by the different techniques outlined above or by any conventional technique. The electrode may be made conductive, *a priori,* or at the end of its preparation.

The above techniques may be used alone or in any combination with other techniques.

### Example 3: Connection of a nucleic acid fiber to an electrode or a carrying substrate

Attachment of a nucleic acid fiber to a substrate, which may be the electrode or the substrate on which the -device is formed is carried out using DNA binding proteins. For example, repressors from a bacterial origin (*lac -* repressor or λ repressor) which can bind to both the substrate (for example a plastic substrate) and the DNA thus joining-the two. Such connection may be later possessed, together with the nucleic acid fiber, to render the bridge conductive, for example, when it connects two electrodes. Alternatively such connection may merely serve to stabilize the conductive bridge to the carrier substrate without participating in the electric functionality.

### Example 4: Preparation of an integrated circuit for detection purposes

The integrated circuit (IC) is composed of a substrate such as silicon, derivatized silicon, silica, derivatized silica, organic polymer or any other substance capable of acting as a support for the fabrication or mechanical fixation or stabilization of the functionalized bridge. The substrate may serve an electrical function.

A typical example for IC preparation is described in the following:

### Example 5: Passivation of a glass substrate

A glass substrate is immersed in fuming nitric acid (100% HNO₃) for 10 min, rinsed with deionized (DI) water, then immersed in 1 N NaOH solution for an additional 10 min and rinsed with DI water. The cleaned glass is dried thoroughly, then immersed for c.a. 12 hrs in a solution of an alkyl tricholorosilane (octyl trichlorosilane, trimethyl trichlorosilane etc.) in tetrachloroethane (1:5 v/v). The glass plate is then rinsed carefully several times with tetrachloroethane and isopropanol, then dried thoroughly.

### Example 6: Electrode fabrication

Electrodes are fabricated according to one of the following routes: (i) Standard photo, electron, or x-ray lithography on the substrate and subsequent deposition of a conductive substance (e.g. metal). Alternatively, the conductive substance may be deposited first and patterned next. (ii) Electrode assembly onto the surface: Patterning of the glass surface using polyelectrolytes such as polyetheyleneimine, polyalcoholes, polyacids, polypyridines etc. or other ligating agents such as a thiol monolayer (fabricated from organic compounds containing thiol and silane moieties at opposite sites on the molecular skeleton) followed by the fixation of electrically conducting components such as Gold colloids enabling the assembly of conducting electrodes onto the substrate.

### Example 7: Forming of a conductive bridge composed of a nucleic acid affinity group - metal based conductive bridges

(i) The path made of two complementary nucleic acid sequences is exposed to a solution containing the appropriate metal ion, thus, ion exchange occurs at the phosphate groups of the nucleic acid skeleton exposed to the solution. Intercalation of ions inside the nucleic acid may also take place under certain conditions;
(ii) The ion exchanged nucleic acid complex is then reduced by a reducing agent such as hydroquinone or by exposure to electomagnetic radiation.
   Cycles (i) and (ii) can be repeated in a sequential order until a conducting bridge is achieved. Alternatively, the formation of conducting metal bridge includes the following steps as stand-alone processes or in conjunction with steps (i) and (ii) or combined with one or more of the following techniques.
(iii) The relevant part of the ion-exchanged bridge is exposed to a metastable mixture of the reducing agent and metal ions. Reduction takes place only at the surface of the metal clusters formed by steps (i) and (ii) thus, the gap between the metal clusters is bridged by the metal deposition process.
(iv) The ion exchanged nucleic acid sequence or the partially treated nucleic acid bridge is exposed to electrochemical processors, transforming the ions loaded on the nucleic acid polyelectrolyte into a metallic conductor. In addition, electrochemical processes along the nucleic acid molecule promote the vectorial growth of the metal wire along it.
(v) Photochemical deposition of the metal from its corresponding ions for the formation of the metallic wire.
(vi) Clusters or colloids are adsorbed onto the nucleic acid bridge using sequence selective components, for example, specific sequences which are capable of binding to specific sites on the nucleic acid sequence non-sequence-specific binding agents, e.g. polyelectrolytes undergoing electrostatic interactions with the DNA. These Clusters and/or colloids serve as catalysts for processes (iii)-(v) above.
(vii) Defects in granular wires fabricated by one or more of the above techniques may be annealed using diverse methods such as thermal annealing processes, electrodeposition, etc.

An example of the fabrication of a silver-functionalized bridge is as follows:
(i) A DNA fiber fixed on a substrate is exposed to a basic solution of silver ions (pH=10.5, NH₄OH, 0.1 M AgNO₃). After the DNA polyelectrolyte is exchanged by the silver ions, the substrate is rinsed carefully with deionized water (DI) and dried.
(ii) The silver loaded DNA bridge fixed on a substrate is exposed to a basic solution of hydroquinone (0.05 M, pH = 5) as a reducing agent. Steps (i) and (ii) are repeated sequentially until an electrically conducting wire is formed.

### Complementary processes:

### (a) step (iii) is performed after one or more (i)+(ii) cycles:

(iii) The DNA fiber loaded with silver metal clusters (after cycles (i) and (ii) have been performed) and after final rinsing with DI water is exposed to an acidic solution of hydroquinone (citrate buffer, pH=3.5, 0.05 M hydroquinone) and AgNO₃ (0.1 M). Cycle (iii) is terminated when the wire width attains the desired dimension. The process can be made light sensitive and thus can also be controlled by the illumination conditions.

### (b) Electrochemical deposition for improved process:

(iv) In order to expedite and improve the metallic conductor, an electrochemical process is performed. For that purpose, pre-treatment with an alkane thiol is performed prior to the (i)+(ii) processes. This ensures the inertness of the metal electrodes against electrochemical metal deposition. After one or more of the (i)+(ii) cycles, the electrodes connected through the DNA-covered metal wire are connected to a current or bias controlled electrical source and the relevant part of the DNA fiber is exposed to a solution of the metal ion (different concentrations according to a specific protocol). The gaps between the conducting domains are filled via electrochemical metallic deposition.

### (c) Photochemical deposition for an improved process:

(v) In order to improve the metallic conductor, a photochemical process is performed in a similar manner to the electrochemical process outlined above but using photochemical reaction as driving processes. For example, metalization of a DNA fiber may be obtained using an electron donor (triethanolamine, oxalic acid, DTT etc.), a photosensitizer (Ru-polypyridine complexes, xanthene dyes semiconductor particles such as TiO₂, CdS etc.), an electron relay such as different bipyridinium salts and the relevant metal ion or metal complex. The photosensitizer transduces the absorbed light energy into a thermodynamic potential through electron transfer processes involving the electron donor and electron acceptor in any of the possible sequences. The reduced electron acceptor acts as an electron relay and charges the metal clusters/colloids with electrons. The charged clusters/colloids act as catalysts for the reduction of the metal ions thus inducing the growth of the metal conductor.

### (d) Gold clusters and/or colloids as nucleation centers:

(vi) Instead of performing the first (i)+(ii) cycles, the relevant part of the DNA bridge is exposed to a solution of gold colloids pre-coated (partially) with cationic thiols (such as pyridinium alkane thiol). The Gold colloids are being adsorbed to the DNA skeleton by ion pairing and the growth of the wire is attained using one or more of the above techniques. Alternatively, the gold colloids may be attached by various means such as biotin-streptavidin binding to modified nucleotides, e.g. modified with biotin.

### (e) Curing processes:

(vii) Defects in a granular wire obtained by one or a combination of the above techniques are annealed using diverse processes such as thermal annealing processes (hydrogen atmosphere (10% H₂ in N₂), 300C over several hours).

### Example 8: Detection of the presence of λ-DNA in a sample

### (a) Device preparation:

The detection of λ-DNA relies on the fact that such a molecule possesses two 12-base sticky ends. Fig 10 outlines the fabrication of a device capable of detecting λ-DNA. A glass coverslip is first passivated against spurious DNA binding. Subsequently, two parallel gold electrodes are deposited on the coverslip using standard microelectronic techniques. One gold electrode is then wetted with a micron size droplet of an aqueous solution containing a 12-base, specific sequence oligonucleotides, derivatitized with a disulfide group attached to their 3' side. Similarly, the second electrode is marked with a different oligonucleotide sequence. The two sequences (oligo A and B in the figure) are complementary to the λ-DNA sticky ends. After rinsing the device is ready for detection.

### (b) Detection:

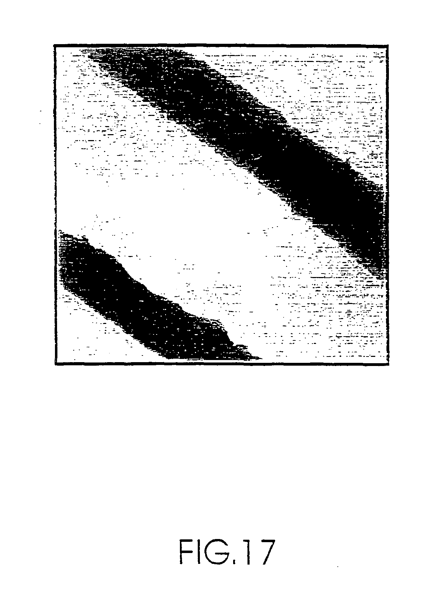

A solution suspected of containing a 16 µm long λ-DNA, having two 12-base sticky ends that are complementary to the oligonucleotides attached to the gold electrodes is made to flow normal to the electrodes. The flow is induced to stretch the DNA, allowing its hybridization with the two distance surface-bound oligonucleotides. In case the sample contains λ-DNA molecules they bind and form a bridge connecting the electrodes. Fig. 17 depicts the results of such an experiment; a fluorescently-labeled λ-DNA bridging the two electrodes.

Two-terminal measurements performed on these samples prove that the stretched DNA molecule is practically an insulator with a resistance higher than 10¹³Ω. To detect the presence of DNA paths they are first instilled to be electrically conductive, by vectorially depositing silver metal along the DNA molecule. The three-step chemical deposition process (Fig. 10 (c)-(e)) is based on selective localization of silver ions along the DNA through Ag⁺/Na⁺ ion exchange and formation of complexes between the silver and the DNA molecules. After rinsing, the silver ion-exchanged -DNA complex is reduced using basic hydroquinone solution. This step results in the formation of nanometer size metallic silver aggregates bound to the DNA skeleton. These silver aggregates serve as spatially localized nucleation sites for subsequent growth of the wire. The ion-exchange process is highly selective and restricted to the DNA only. The silver aggregates, bound to the DNA, are further *"developed",* much as in the standard photographic procedure, using an acidic mixture of hydroquinone and silver ions under low light conditions. Acidic solutions of hydroquinone and silver ions are metastable but spontaneous metal deposition is normally very slow. The presence of metal catalysts (such as the silver nucleation sites on the DNA), significantly accelerates the process. Under these experimental cnditions, metal deposition therefore occurs only along the DNA skeleton, leaving the passivated glass practically clean of silver.

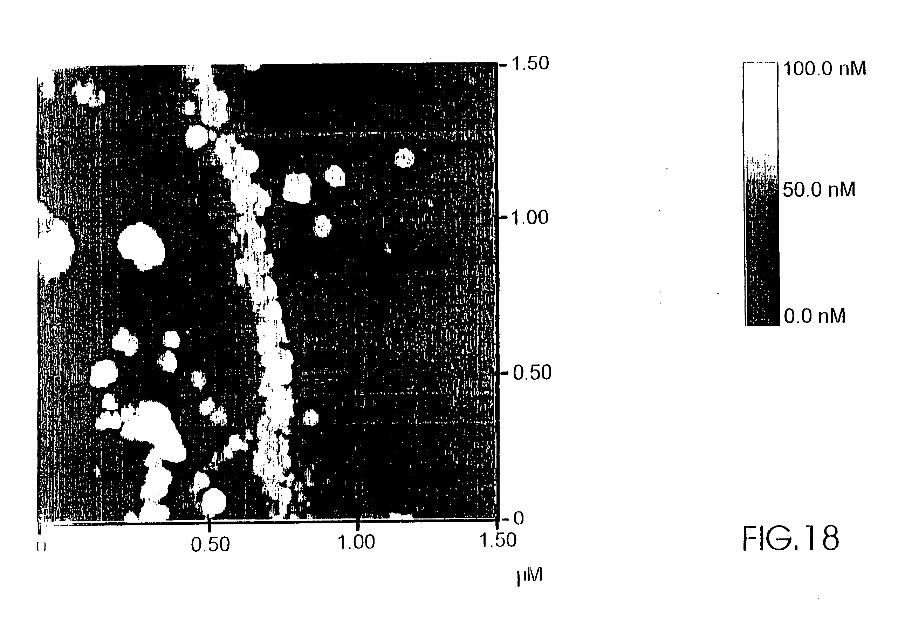

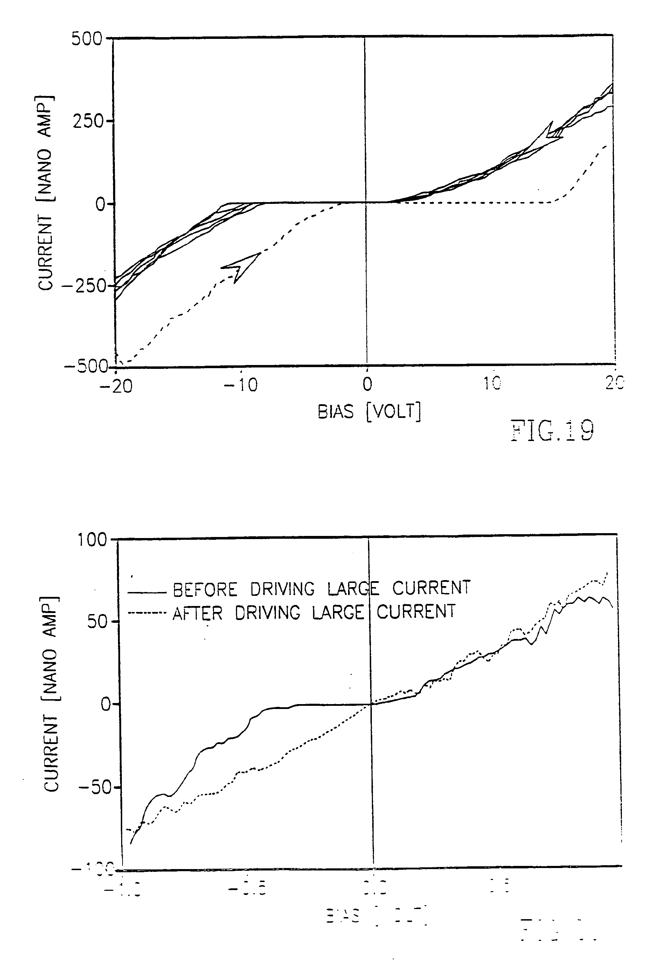

Atomic force microscope (AFM) images of a section of a 100 nm wide, 12 µm long wire are presented in Fig. 18. As clearly seen, the wire comprises of 30-50 nm-diameter grains deposited along the DNA skeleton. Fig. 19 shows the I-V curves of the silver presented in Fig. 18. The length of the zero bias plateau in different wires can be tuned from zero volt to roughly 10 volts. The solid line in Fig. 20 depicts, for example, the I-V curve of a different wire in which the silver growth on the DNA was more extensive. As a result, the plateau can be eliminated to give an ohmic behavior (dashed line in Fig. 20).

This example proves that λ-DNA molecules can indeed be detected using the present invention.

### Example 9: Organic conjugated-polymer based conducting wires

A schematic representation of the manner of production of organic conjugated polymers is shown in Fig. 12 step (a)-(b) are similar to those disclosed in Fig. 10.
(i) The relevant part of the path is exposed to a solution containing a cationic segment capable of forming a conjugated-polymer by a chemical transformation or a cationic non conjugated-polymer capable of undergoing conjugation by a chemical transformation or a cationic conjugated-polymer. Thus, ion exchange process occurs at the phosphate groups of the DNA skeleton exposed to the solution.
(ii) The ion exchanged DNA complex is treated according to the nature of the organic species that is bound to the polyanionic skeleton. Electrical conductance is achieved either by the former process or by a sequential doping process. Doping may be achieved via conventional redox processes, by protonation - deprotonation processes, by electrochemical means or by photochemical means. Additionally, sequence selective processes between the DNA skeleton and the building blocks of the above organic conjugated-polymer based conducting wires can be utilized for the production of wires.

I. The fabrication of a PPV (poly-p-phenylene vinylene) conducting wire is as follows:
   (i) A DNA fiber fixed on a substrate (b) is exposed to a solution of a pre-PPV water soluble polymer. After the DNA polyelectrolyte is exchanged by the pre-PPV polymer, the substrate is rinsed carefully and dried.
   (ii) The pre-PPV polymer loaded DNA fiber fixed on the substrate is reacted in a vacuum oven (e.g 1e-6 bar, 300 C, 6hr.).
   (iii) The resulting luminescent PPV polymer is doped using conventional methods until displaying conductivity.
II. An alternative route for the fabrication of a PPV conductive wire is as follows:
   (i) A DNA path fixed on a substrate (Fig. 12(a) and 12(b) is exposed to a solution of a bis-(tetrahydrothiophenium)-p-xylilene dichloride (Fig. 12(c). After the DNA polyelectrolyte is exchanged by the bis-(tetrahydro-thiophenium)-p-xylilene dichloride, the substrate is rinsed carefully and dried.
   (ii) The bis-(tetrahydrothiophenium)-p-xylilene dichloride loaded DNA sequences fixed on a substrate is polymerized in a basic solution to form a pre-PPV polymer attached to the DNA backbone (Fig. 12(d).
   (iii) The pre-PPV polymer loaded DNA sequences fixed on a substrate is reacted in a vacuum oven (1e-6 bar, 300 C, 6hr.).
   (iv) The resulting luminescent PPV polymer is doped using conventional methods until displaying desired conductivity.
III. The fabrication of a PANI (polyaniline) conducting wire is carried out as follows:
   (i) A DNA bridge fixed on a substrate is exposed to a solution of an acid soluble PANI polymer. After the DNA polyelectrolyte is exchanged by PANI polymer, the substrate is rinsed carefully and dried.
   (ii) The resulting PANI polymer is doped using conventional methods until displaying desired conductivity.
IV. An alternative route to the fabrication of a PANI conducting wire is as follows:
   (i) A DNA bridge fixed on a substrate is exposed to a solution of anilinium ions. After the DNA polyelectrolyte is exchanged by the anilinium ion, the substrate is rinsed carefully and dried.
   (ii) The anilinium ions loaded on the DNA sequences are oxidized using a solution of an oxidizing agent such as peroxidisulphate ions, yielding a polyaniline polymer. The resulting PANI polymer is doped using conventional methods until displaying desired conductivity.
V. An alternative route to the fabrication of a PANI conducting wire is as follows:
   (i) A DNA bridge fixed on a substrate is exposed to a solution of a short oligomers of PANI (>1 repeat unit). After the DNA polyelectrolyte is exchanged by the PANI oligomer, the substrate is rinsed carefully and dried.
   (ii) The PANI oligomer ions loaded on the DNA sequence are oxidized using a solution of an oxidizing agent such as peroxidisulphate ions, yielding a polyaniline polymer. The resulting PANI polymer is doped using conventional methods until displaying desired conductivity.

### Example 10: PPV functionalized fiber as a light source

The process described in Example 9 may be followed up to and including step I(ii). The resulting PPV component is highly luminescent. Fabricating the PPV component between electrodes of appropriate work functions then forms an electroluminescent device.

### Example 11: Polymer supported recognition moiety for selective attachment of recognition moieties to electrodes

### Specific Examples:

N-(2-ethyl maleimido) pyrrole is attached to a 3'-thio modified specific sequence of monostranded oligonucleotide. Electro-oxidation of a solution containing the 3'-mercapto(N-(2-ethyl succine imido) pyrrole) oligonucleotide induces the formation of a polypyrrole coated electrode bearing specific sequence oligonucleotides. The polymer is deposited exclusively at the anode side allowing the selective coating of a plurality of electrodes simply by dipping the electrode assay set into a series of solutions containing the desired sequences each time using a different electrode as the anode. Since polypyrrole is a conductor in its doped state, electric connectivity of the polymer layer is enabled upon electrodoping the layer.

### Example 12: Detection of short strands of DNA using direct electrical measurements

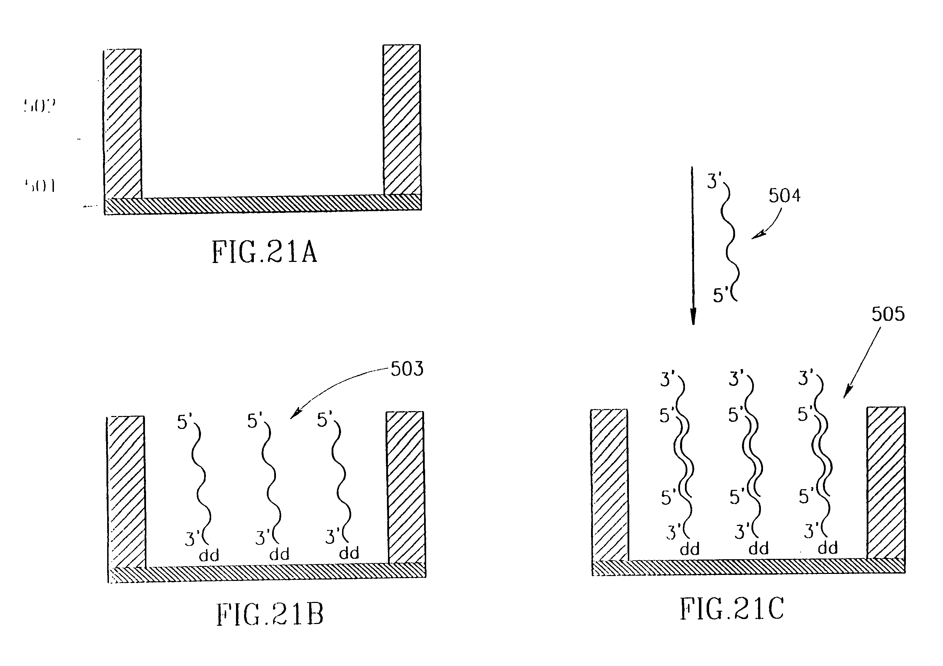

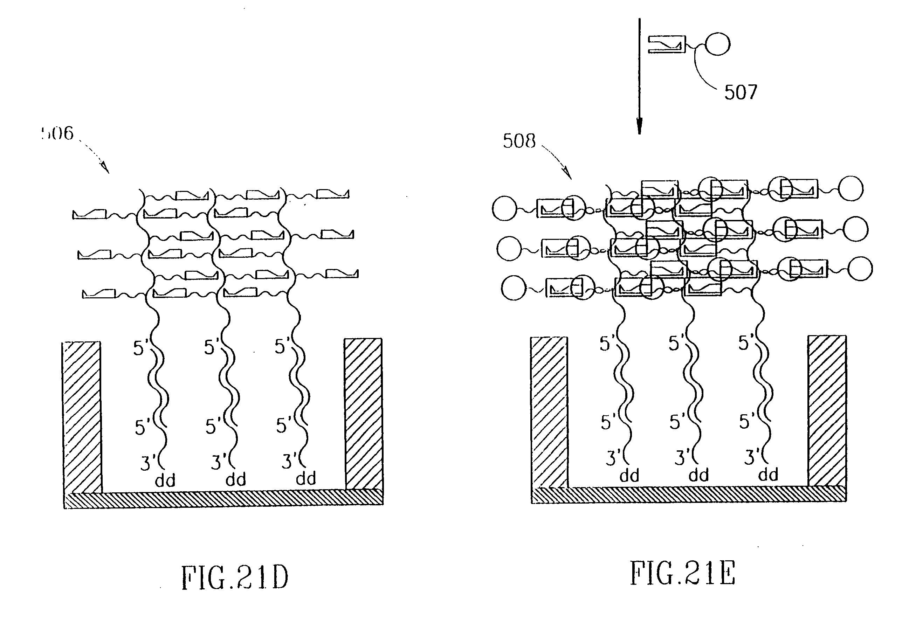

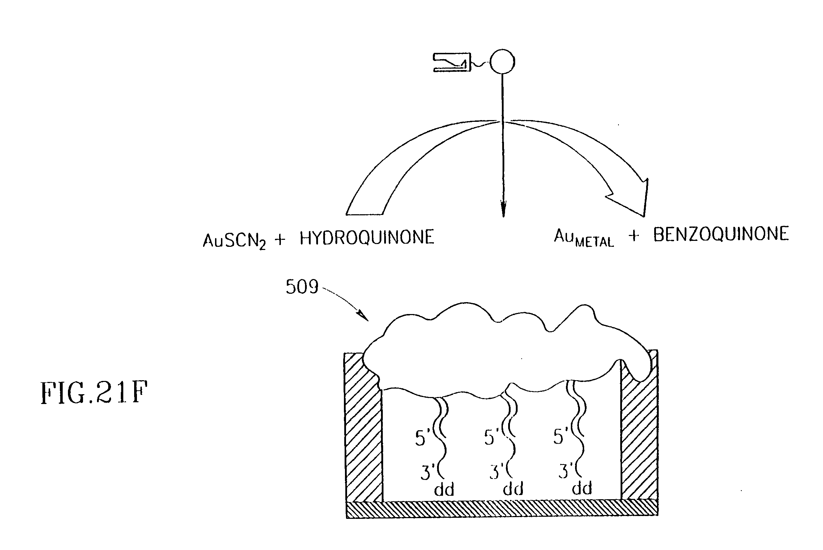

In Fig. 21(A) two conducting electrodes **502** are defined on an insulating substrate **501.** In Fig. 21(B) a monolayer of short, single-stranded oligonucleotides **503** is constructed in the gap between a pair of electrodes **502** of the assay device. The sequence of the oligonucleotides is complementary to the sequence of the target to be deleted. The oligonucleotides have a dideoxy base at their 3' terminus and are therefore incapable of being extended with nucleic bases by use of transferase.

Fig. 21(C) shows that upon contacting said assay device with the sample, the target oligonucleotide **504** binds to the recognition moiety **503** thus forming a recognition group (double-stranded DNA) **505.** Different post- hybridization treatments such as washing at different temperatures and different salt concentrations ensure high selectivity in duplex formation.

In step (D), the assay device bearing the DNA duplex is contacted with a solution containing transferase and biotinylated bases which induces the elongation of the DNA skeleton at the 3'-deoxy site **506.**

In a subsequent step (step E), the assay device is exposed to a solution containing gold colloids coupled to streptavidin units **507.** The resulting assay device bears DNA molecules with pendant gold colloids **508.**

In the step step (step F), said assay device is exposed to a solution containing hydroquinone and Au(SCN)₂. Gold is deposited only on metal surfaces that act as catalysis centers. The colloids grow and merge to form a conductive path **509** bridging the two gold electrodes. The detection of current at applied bias signals the presence of the target DNA sequence in solution.

In the absence of the target DNA sequence, no recognition group is formed between the electrode pair and no gold particles bind between the electrodes. The absence of metal nucleation centers prevents the formation of a conductive path between the electrode pair. The absence of electric current upon induced bias signals the absence of the target in the sample.

### Example 13: Detection of an Antibody by an Antigen or vice versa

The recognition moiety in this case is an antigen or an antibody selective to its antibody or antigen, respectively. The recognition moiety is attached to the electrode(s) by one of a variety of different methods, for example, by complexing it with another group that can bind to gold, by attaching a thiol group, etc; or it can be directly covalently linked to the electrode. In many cases van der Walls forces are sufficient to ensure binding of the recognition moiety to the electrode. The target may be attached to a modifier which eventually serves to bridge the gap between the electrodes. For example, an antigen can be attached to an end of a DNA fiber and bind to its antibody on the electrode with this modifier attached. The other side of the DNA fiber can selectively bind to another electrode in the assay set, or it can be non-selectively attached to it. Substrate passivation, electrode definition, metalization and detection follows the general principles outlined above, e.g. in Example 8.

### Example 14: PCR and other methods allowing the introduction of modified nucleotides

The introduction of modified bases into DNA or RNA fibers may help in constructing the detection system. Examples of modified nucleotides are: biotin derivatized nucleotides or nucleotides with prime amine groups connected to them. There are different standard molecular biological techniques allowing the introduction of modified nucleotides in specific location along an existing DNA or RNA fiber or constructing copies of a nucleic acid template with a complete sequence of modified nucleotides. For example, the polymerase chain reaction (PCR) technique can be used to amplify a nucleic acid template with modified nucleotides. In this case the modified bases serve as the nucleotides in the PCR solution (mixed with unmodified bases or not) and together with the provided primers (the latter can be synthesized with the same modified nucleotides if necessary) facilitating the amplification process. Alternatively, the method of random priming allows the replacement of nucleotides with modified nucleotides. In some cases ligation of the polymerization products along the template is required to ensure a continuous fiber. Another possibility is to use one of the stranded polymerases (e.g. a Klenow fragment) to fill gaps along double-stranded DNA fibers or to fill sticky-ends with modified bases. Alternatively, DNA terminal transferase can be used to attach modified bases to the 3' side of a nucleic acid fiber (single or double-stranded). In cases where the modified bases are localized on specific points on the nucleic acid fiber they can be used to attach the fiber specifically to other groups, e.g. a thiol group, a streptavidin or another nucleic acid fiber, etc.

Constructing a complete sequence of a nucleic acid fiber with modified nucleotides allows to achieve highly selective metalization of the bridges. By this method, the modified bases attach specifically groups or complexes that can serve as nucleation centers to catalyze metalization prior to detection. For example, nucleotides derivatized with amine groups can bind specifically tiny gold clusters or colloids that serve as well defined nucleation centers for gold or silver (and many other metals) deposition for yielding a conduvtive bridge. Another examples is to use bases derivatized with biotin and to attach colloids or gold clusters coated with streptavidin along the DNA fiber. These colloids or gold clusters again can serve as nucleation centers for the metalization process.

### Example 15: In situ PCR on electrodes or between electrodes

*In situ* polymerase chain reaction (PCR) is a relatively recent technique used usually to detect minute quantities of DNA or RNA in tissue sections or intact cells. It uses the high selectivity of hybridization techniques, allowing, for example, to correlate a specific sequence with a tissue section, with the amplification power of the PCR allowing the many fold increase in detection sensitivity (for a recent review of the technique see: In Situ Polymerase Chain Reaction and Related Technology, edited by J.Gu, Eaton Publishing, 1995). This technique can be employed for specific target amplification, on the substrate or electrodes of the assay device, for example, a DNA sequence present in a minute concentration.

In a first step, the target in front of the sample binds to the specific recognition moiety immobilized on a substrate member situated between an assay set of electrodes, e.g. by hybridization with a complementary DNA sequence fixed to the substrate by e.g. biotin-streptavidin, amine-thiol, etc. A solution containing appropriate primers, optimized concentration of bases and the appropriate buffers and one of the standard PCR polymerases (e.g. a Taq polymerase) is added and then a thermal cycle can be started. In that case the assay device is placed in a temperature control apparatus allowing to control and modify its temperature in fast and automatic way. The template is now specifically duplicated in each cycle to form new templates for the next cycle, hence exponential amplification is possible. The new templates are generated *in situ,* and may attach to the substrate to form a network with the original DNA target. This may be achieved by non specific binding of the long DNA fibers or by specific binding. For example, by photoactivating, at the end of each cycle, a moiety group attached to the newly formed templates leads to their binding to other complexes on the substrate or to other templates already attached to the substrate. This binding should not interfere with the possibility to bind the primers at the next cycle for further amplification. After sufficient cycles (e.g. 30) a network of DNA fibers, exact copies of the original target, attached to the substrate, fills the gap between an assay set of electrodes. Metalization process then follows, according to one of the techniques specified above, allowing eventually an electric detection of the formation of this network bridge.

One modification to the above is the possibility to carry the amplification procedures between two substrates (e.g. closely spaced glass slides or nylon membranes) that will force newly formed templates to stay *in situ.* For example, a filter membrane with the proper cutoff, allowing the passage of primers, bases, polymerase and buffers but not long templates can serve for that purpose. In this case all the necessary ingredients can be continuously fed through the membrane ensuring no escape of the templates. Such a semi-permeable membrane also enables efficient washing before metalization and detection.

### Example 16: Ligase chain reaction (LCR) on electrodes or between electrodes

The ligase chain reaction is a technique for amplifying a specific sequence by ligating at each cycle two subsections of a template. By using a thermocycler identical to the one used for the PCR technique, denaturization of the templates followed by annealing of subsections that exactly match the template to form a continuous nucleic acid fiber with a single or multiple nicks. These nicks are ligated by a special ligase that work at high temperatures (e.g. pfu ligase). This technique can be applied *in situ,* similar to Example 15 above. An example for a possible amplification is to ligate two short subsections, to a strand not long enoguh to bridge the gap between electrodes. Because of the amplification power of this technique (again in each cycle newly formed ligated fibers serve as templates for the next cycle), it allows to form a bridge across electrodes made specifically from copies of the target to be detected which is introduced originally in only a minute concentration.

### Example 17: Enzyme-substrate or protein-molecule as possible affinity groups

To allow electrical detection of small molecules such as an enzyme (or its substrate) or a protein in some cases they may be attached to a modifier. For example, biotin can be attached to a nucleic acid fiber to enable the detection of avidin or streptavidin. In some cases the modifier can be a synthetic polymer. Another example is the use of a conducting polymer as a modifier facilitating electrical detection in later stages.

### Example 18: Bacterium detection

The target to be detected in this case is a bacterium. The recognition group can be, for example, an antibody to a specific antigen on the bacterium. Alternatively, biotin-avidin or other specific binding between a molecule or a supramolecular structure on the bacterium membrane and a proper recognition moiety on the electrodes or on the substrate between electrodes is possible. The bacterium will form a bridge across electrodes. Metalization then facilitates electrical detection. An electric field may assist in directing the bacterium to the proper location on the substrate or electrodes. In some cases, it is possible to use ionic currents (alone or in combination with electronic currents) through the bacterium (e.g. using the bacterium natural ionic channels) for electrical detection. Electric or magnetic fields or light can be used as tweezers for bacteria, trapping them between electrodes prior to their metalization and electrical detection.

### Example 19: Procedure for preparation of an array (chip) for nucleic acid attachment

### Step 1: Passivation of Chip

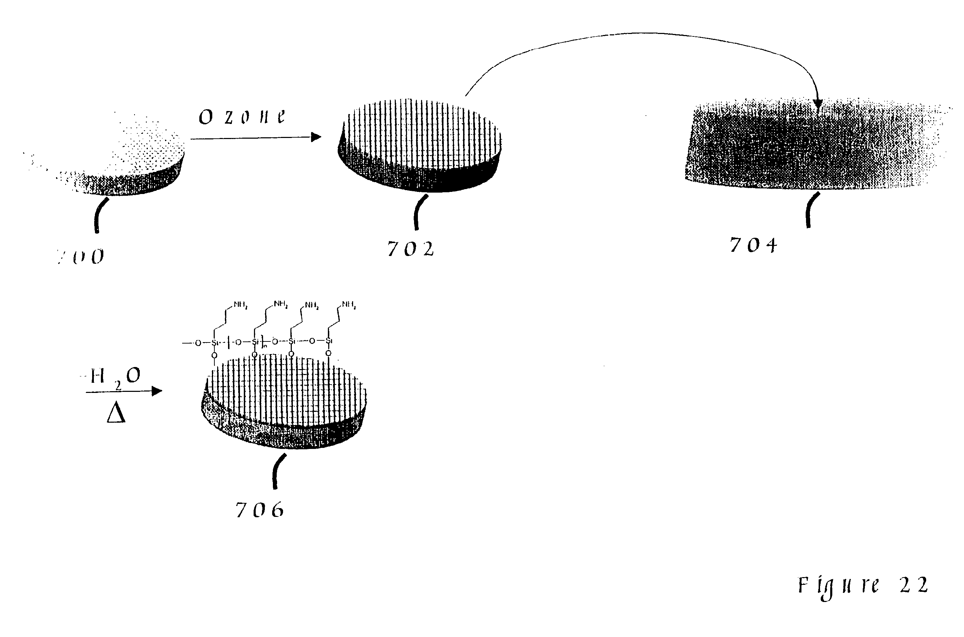

The method is schematically represented in Fig. 22.

Chip **700** was treated in an ozone chamber for one hour at room temperature to render it a chemically passivated, resulting in chip **702.** Chip **702** is then placed in an oven at 120°C for one hour and cooled to room temperature in a desiccator.

### Step 2: Derivatizing of chip with a layer of poly-amonopropyl siloxane

19 mL absolute ethanol, 1 mL water and 0.4 mL triethoxy aminopropyl silane were mixed and left to react for 5 minutes at room temperature and the resulting solution is designated **704.** The oven-dried chip **702** was immersed into the reaction solution **704** for 2.5 minutes, then washed successively with several portions of ethanol and water, spin dried and placed in an oven for one hour at 120°C for crosslinking of the surface attached polysiloxane. The chip coated with a layer of polyamonoproyp siloxane **706** serves for fixation of nucleic acid probes to selected locations by one of several methods such as electrostatic attachment, photoinduced crosslinking of nucleic acids to organic layer on surface, attachment of carboxy derivatives of nucleic acids, electrochemical attachment, etc. Alternatively, The organic layer on chip may be used as anchor to attach precursors for on-chip synthesis of oligonucleotides. Layers having different properties may be formed using different procedures known in the art.

### Example 20: Procedure for covalent attachment of nucleic acid probes to a chip

### Step 3: Attachment of Nucleic acid probes to chip 706 of Fig. 22

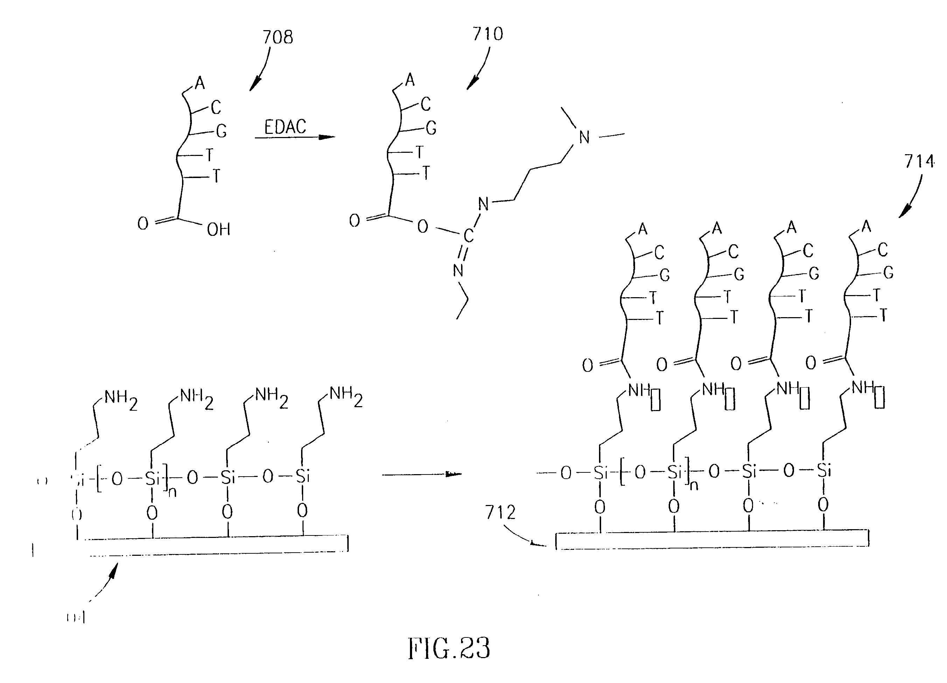

The method of attachment is schematically shown in Fig. 23.

Nucleic acids derivatized with carboxyalkyl groups **708** and EDAC used as coupling agent, were dissolved in phosphate buffer, pH = 8.0-8.5, to form activated ester **710.** The solution was contacted with chip **704** (prepared as described in Example 19) in a controlled manner so that the nucleic acids become bound to the chip at selected places. The solution is removed from derivatized chip **712,** bearing probe nucleic acids **714,** by successive washing with water.

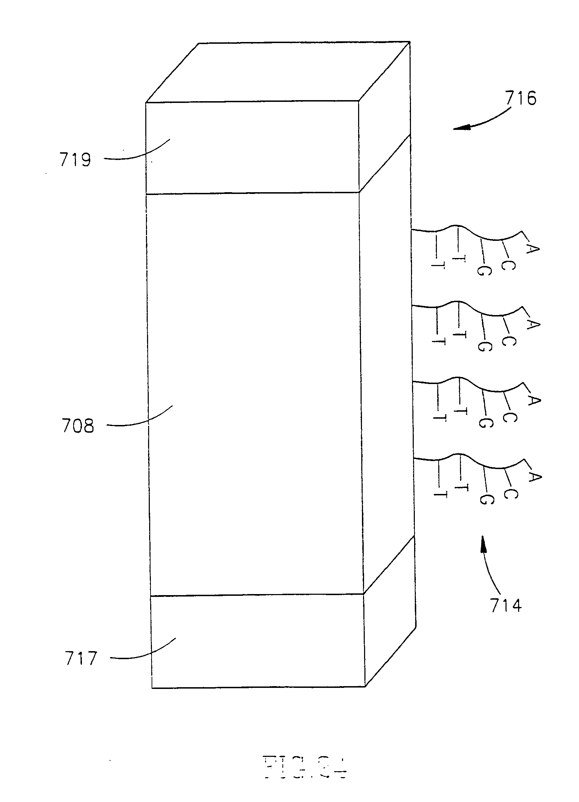

Fig. 24 presents essentially the same as Fig. 23. However, in the present figure, the electrodes are open ends of conductive layers, **717** and **719,** separated from each other by non-conductive layer, **718.** The electrodes are part of one detection site, **716** present on derivatized chip in which the open end of the non-conductive layer (gap) **718** is derivatized with recognition moiety nucleic acids **714.** In this case the electrodes are formed by layering conductive layers, separated by non conductive layers (serving as gap **718)** and then exposing the conductive layers by cutting holes, boring openings, etc.

### Example 21: A process of attaching biotin to nucleic acid molecules in a sample

### Step 4: Attachment of biotin to nucleic acids in a sample

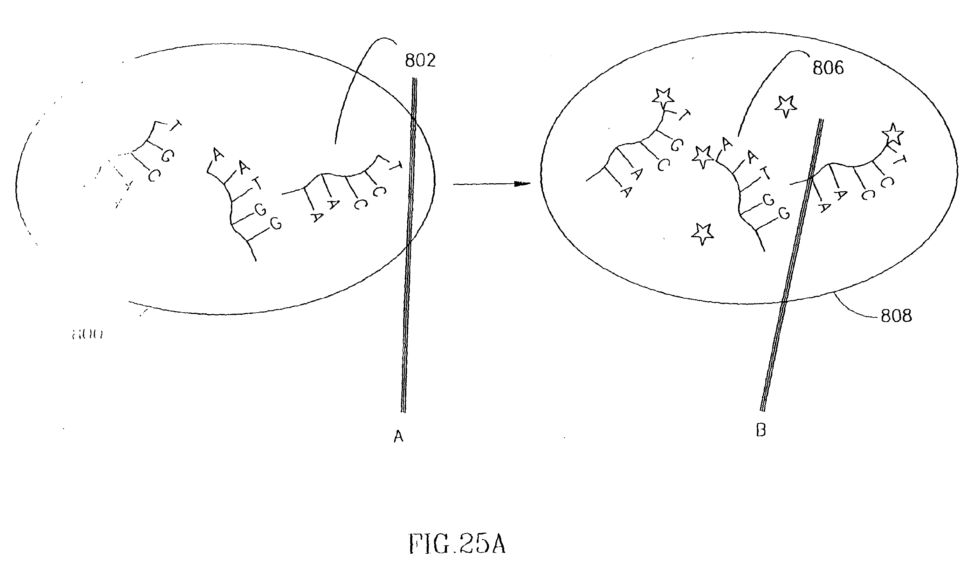

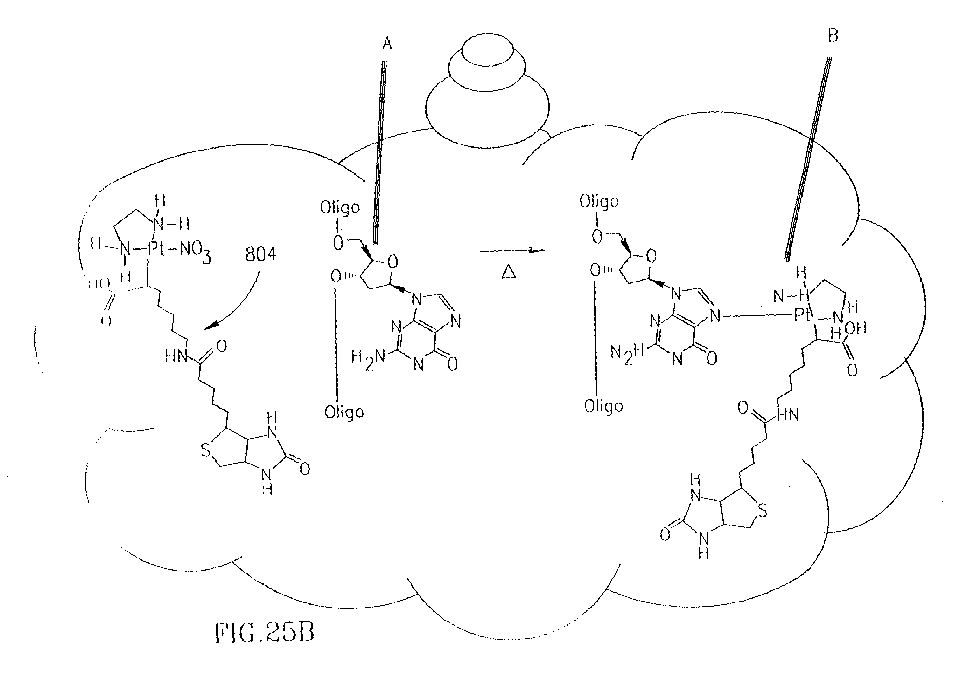

The process is shown schematically in Fig. 25. Attachment procedure used was according to the Biotin-Chem-Link, Cat. No. 1812149, Boehringer Mannheim. Nucleic acids were attached to biotin as a first step for attaching later nucleation-center forming entities (using avidin). Target nucleic acid sequences are reacted with an agent such as a biotinylated cis-platinum complex which binds to N7 of Guanosine and Adenosine bases in nucleic acid sequences. Using the following process, nucleic acids become attached to one or more of the cis-platinum complexes with only minor side reactions and cleavage. The binding of labeling groups to the nucleic acid still allows efficient and selective hybridization with its complementary sequences.

### Procedure for attachment of biotin:

Sample **800** which may contain one or more molecules of one or more target nucleic sequences **802** is mixed with cis-platinum biotin complexes **804.** The solution is heated to 85 C for 30 min. then cooled to room temperature, and mixed with a stop solution. Typical stop solutions contain one or more compounds that bind irreversibly to the agent thus rendering it inactive. Such solutions are solution containing tris-[hydroxymethyl]aminoethane (>40 mM), EDTA (>5mM), magnesium acetate (>100mM) etc.. The nucleic acid **806** bears one or more biotin fragments. The resulting biotin-bearing sample, **808,** which may contains one or more molecules of one or more biotin-containing target sequences **806,** can be purified using different purification procedures such as size exclusion chromatography.

### Example 22: Hybridization process between nucleic acid molecules in a biotin-containing sample and nucleic acid probes present on chip

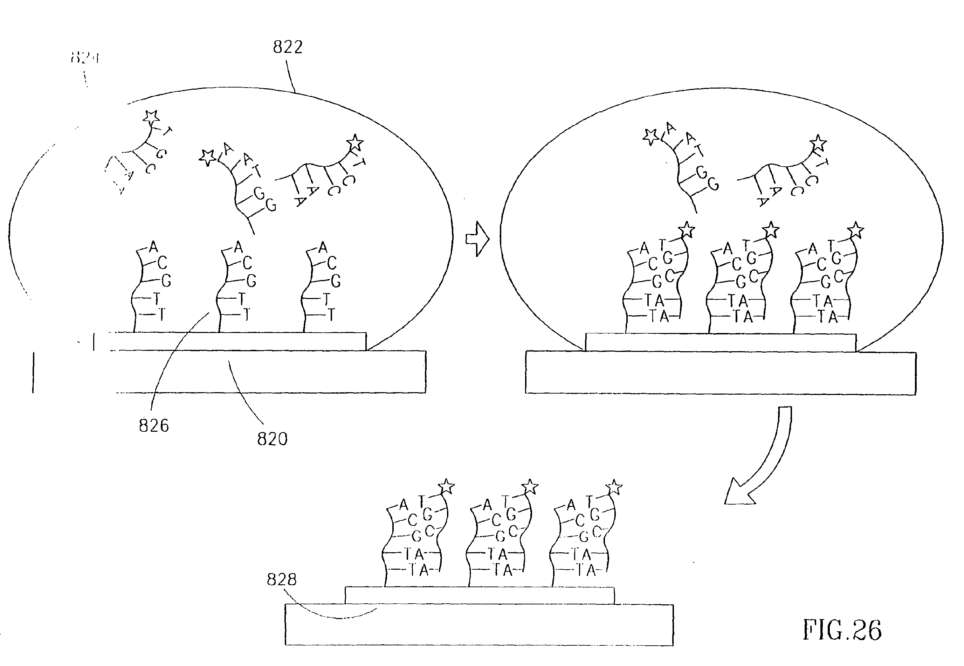

The hybridization procedure is shown schematically in Fig. 26. Different hybridization procedures may be used depending on specific requirments desired.

### Step 5: Hybridization and stringency wash.

**Hybridization solution:** 28 mL Formamide, 7 mL SSC (20X), 8 mL, Denharts solution (50X), 0.4 mL salmon sperm solution and a solution of 10% SDS were mixed and heated to 42 C for 30 min before use.

**Hybridization:** Each derivatized chip, **820** prepared as described abovewas immersed in hybridization solution and shaken for 30 min at 42°C. Then, the biotin-containing sample, **822** (prepared as described in Example 21) and treated according to step 4, was added to the solution. The chip was shaken in solution for 12 hours at 42°C in order to hybridize which serve as targets **824,** with their complementary sequences, **826,** (recognition moieties) present on surface of derivatized chip **820.**

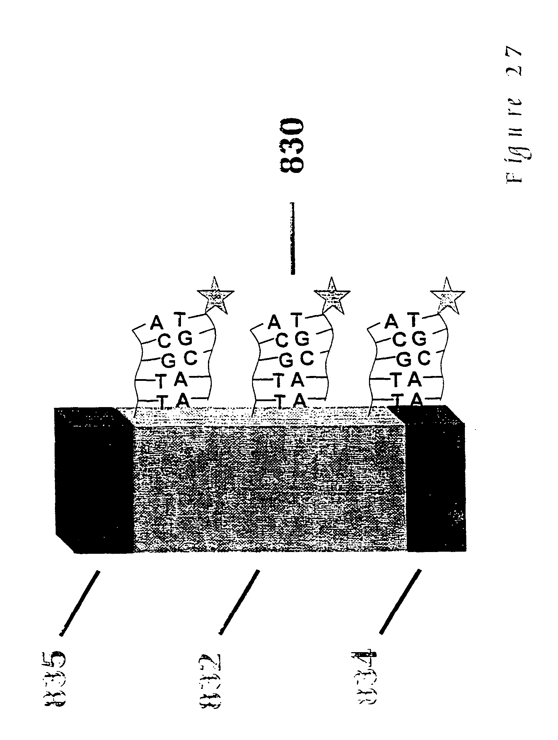

**Stringency wash:** Derivatized chip, **820,** was removed from hybridization solution and immersed in a 0.2 X SSC solution at 42°C while shaking. The stringency process was repeated three times, yielding hybridized chip **828,** wherein biotin-containing targets, if present in sample **822,** are bound to the recognition moieties. Fig. 27 shows formation of the complex **830** between the target and recognition moiety present on an insulating gap **832** formed by a non-conductive layer separating between two conductive layers **834** and 835 which open ends serve as electrodes.

### Example 23: Attachment of Steptavidin-gold conjugate (serving as nucleation centers) to biotin-containing nucleic acids hybridized to surface-bound recognition moieties

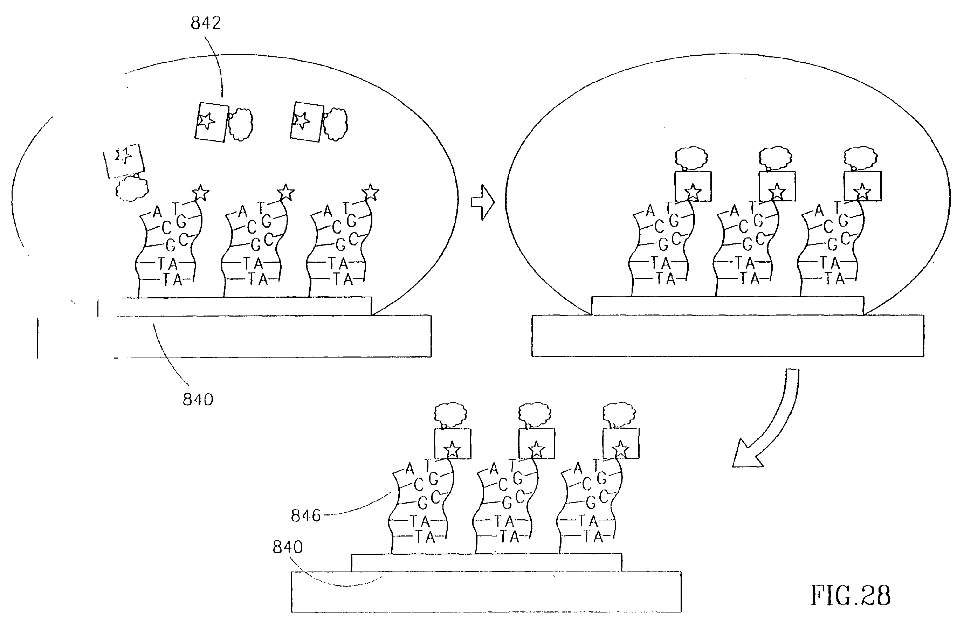

The attachment is shown schematically in Fig. 28.

### Step 6: Attachment of avidin-containing nucleation centers to biotin-containing nucleic acids that were hybridized to surface-bound recognition moieties.

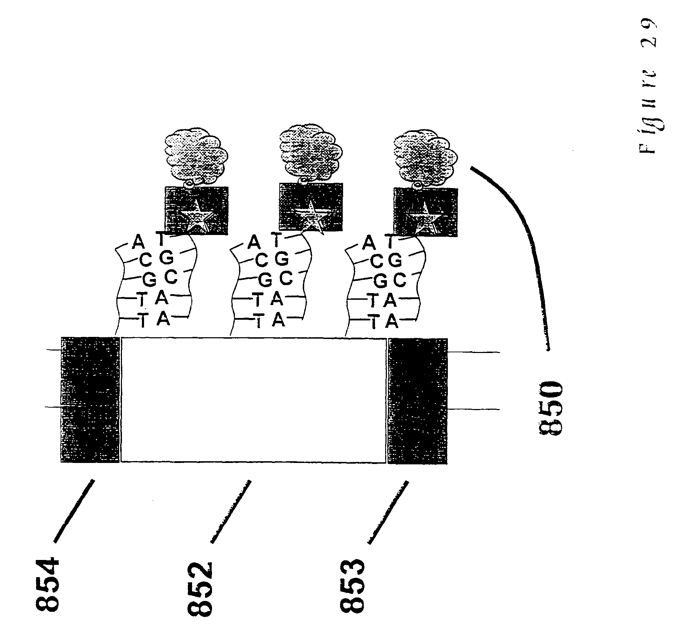

Chips after stringency wash, **840,** were shaken for 40 min in a solution of a 1:4 ratio of milk blocking solution in distilled water at room temperature. Then, a solution of Steptavidin-gold conjugate (Streptavidin-nanogold, Nanoprobes), **842,** was added. The chip was further shaken in solution at room temperature, for additional two hours after which it was washed three times with 0.1 X membrane wash solution at room temperature (shaken in each wash solution for five minutes). After drying, chip, **840,** bears a complex of the target and recognition moiety derivatized with Steptavidin-gold conjugate, **846** if biotin-containing target sequences to be detected were present in sample, **822,** in previous step 5. Fig. 29 shows essentially the same as Fig. 27, where nucleation centers are attached to biotin on the complex between target and recognition moiety so that complex between target and recognition moiety bears a nucleation center **850,** which is present on a gap **852** formed by a non-conductive layer separating between two conductive layers **853** and **854** which open ends serve as electrodes as described in Fig. 24.

### Example 24: Gold deposition on nucleation centers:

### A. Solutions

The following stock solutions were used:
**Solution a:** 240 mg of KAuCl₄ were dissolved in 10 mL distilled water and filtered through a 0.22 µ filter.
**Solution b:** 600 mg of KSCN were dissolved in 10 mL distilled water and filtered through a 0.22 µ filter.
**Solution c:** 550 mg of hydroquinone were dissolved in 10 mL distilled water and filtered through a 0.22 µ filter.
**Solution d:** 320 mg of thiodipropionic acid were dissolved in distilled water and the pH adjusted to 5.50. The solution was adjusted to 10 mL and filtered through a 0.22 µ filter.
**Solution e:** 1M phosphate buffer pH=5.50 was prepared from distilled water and filtered through a 0.22 µ filter.

For gold deposition, the following solutions were used:
**Solution l:** 600 µL of **solution a** were mixed with 600 µL of **solution b** at room temperature. The resulting orange precipitate was collected and redissolved in 5000 ml of **solution e,** forming **solution g.** 1200 µL of **solution c** were added to **solution g** as soon as it turns fully transparent, forming **solution h. Solution h** was filtered through a 0.22 µ filter and used immediately.
**Solution II:** 3000 µL of **solution a** were mixed with 3000 µL of **solution d** at room temperature, forming **solution i. Solution i** was filtered through a 0.22 µ filter and used immediately.

### B. Procedure

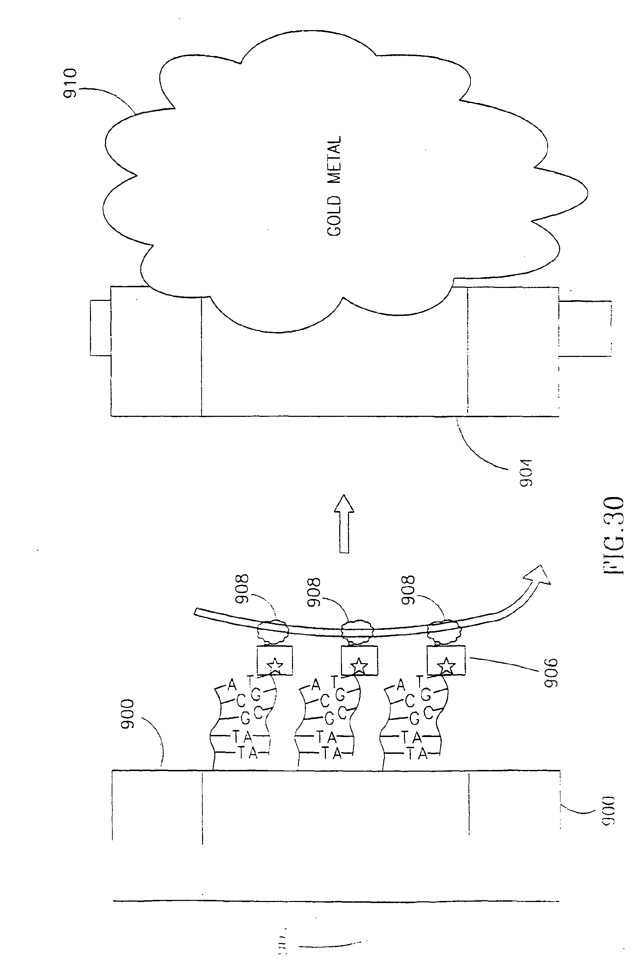

Fig. 30 presents a gold deposition process on one detection site comprising a set of two electrodes **900 and 901** (by open ends of exposed conductive layers separated by a gap **904** of non-conductive layer). The gap **904** holds complexes of labeled targets and recognition moieties derivatized with Steptavidin-gold conjugate, **906** each containing a nucleation center **908.** Freshly prepared solutions I or II were contacted with the treated substance so that each nucleation center **908** catalyzes gold deposition from the metastable gold solution, forming large single and/or polycrystallite deposit **910** which serves as a conductive bridge between electrodes **900** and **901.** As can be seen, although the complexes between the target and the recognition moieties themselves do not form a physical bridge between the electrodes, the large crystallite do.

### Example 25: AFM pictures of chips

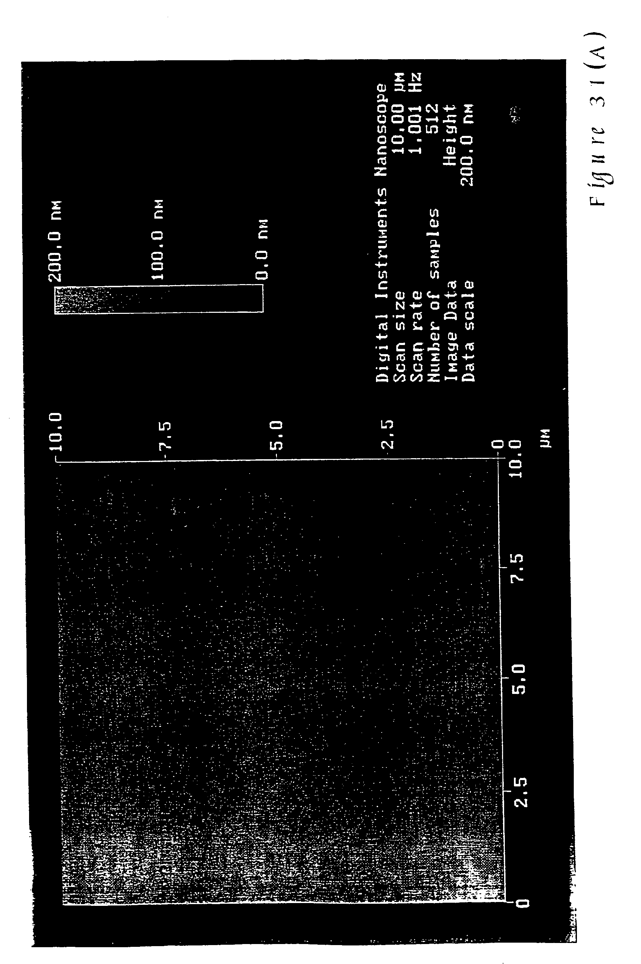

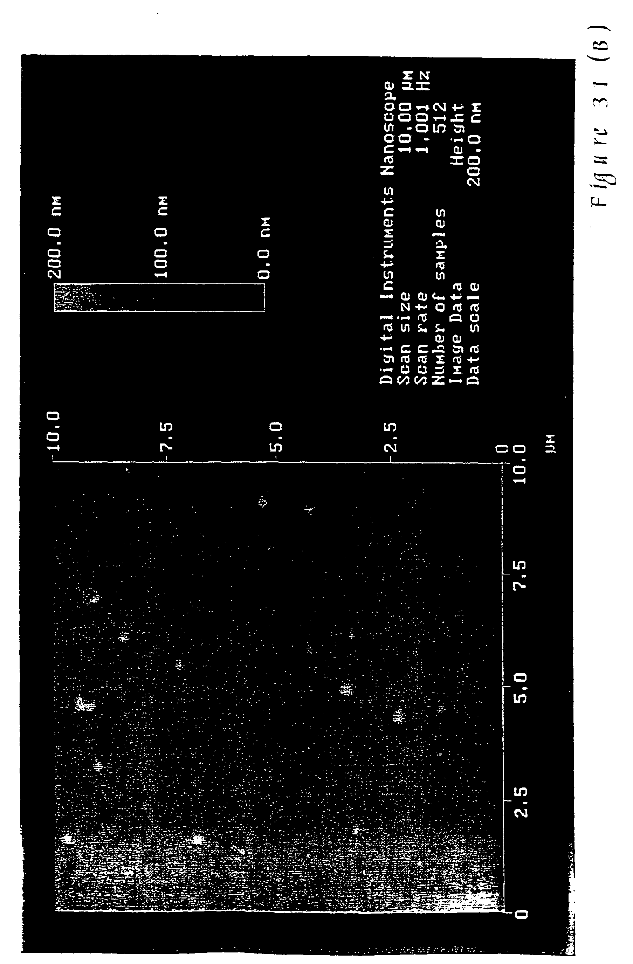

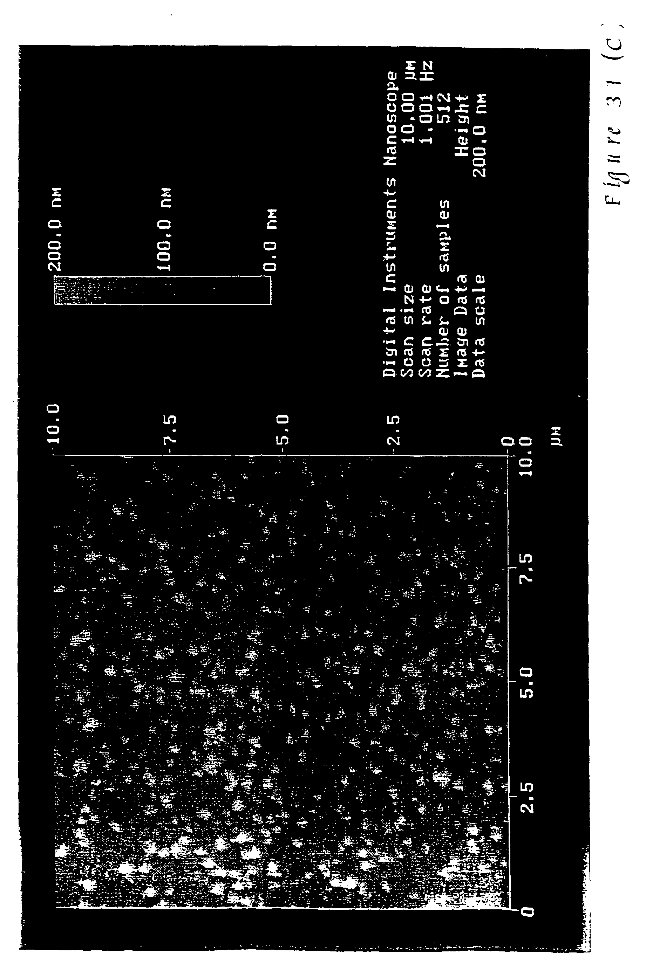

Figs. 31(A), 31(B), 31(C) are representative AFM pictures of three typical areas on one chip that underwent the entire process described above: 31(A) depicts a surface lacking any DNA recognition moieties. 31(B) depicts a surface bearing recognition moieties having a sequence that complement only partially the sequence of the target in the sample and 31(C) depicts a surface bearing recognition moieties having sequences that fully complement the sequence of the target in the sample. As can be seem, virtually no target gold particles were formed in the absence of appropriate recognition moieties, few gold particles were formed where the recognition moiety was only partially specific to the target, while an abundance of particles are evident with a recognition moiety which is fully complementary to the target.

### Example 26: Electrical Detection

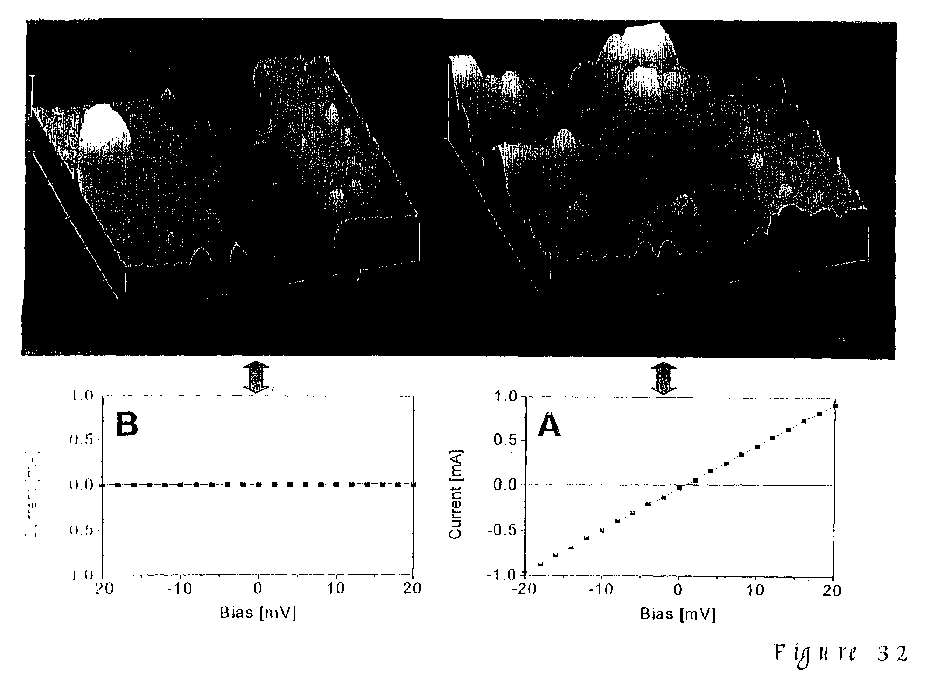

Fig. 32 depicts AFM pictures of two assay sets comprising two electrodes on a chip treated as described above. Upper left picture depicts the surface of one assay set comprising two electrodes lacking any DNA recognition moieties. Lower left picture (B) depicts the current-voltage curve of the above detection site showing no electrical conductance. Upper right picture depicts the surface of one assay set having as a recognition moiety of a sequence that fully complement the sequence of the target in the sample. Lower right picture (A) depicts the current-voltage curve of the above detection site showing that the gold deposition on hybridized target recognition moiety derivatized with Steptavidin-gold conjugate, bridges the electrically insulating gap, resulting in an electrically conducting detection site. Thus, the electrical conductance of an assay set, is a measure of the presence of hybridized complexes of targets and recognition moieties.

### Example 27: Multiplexing

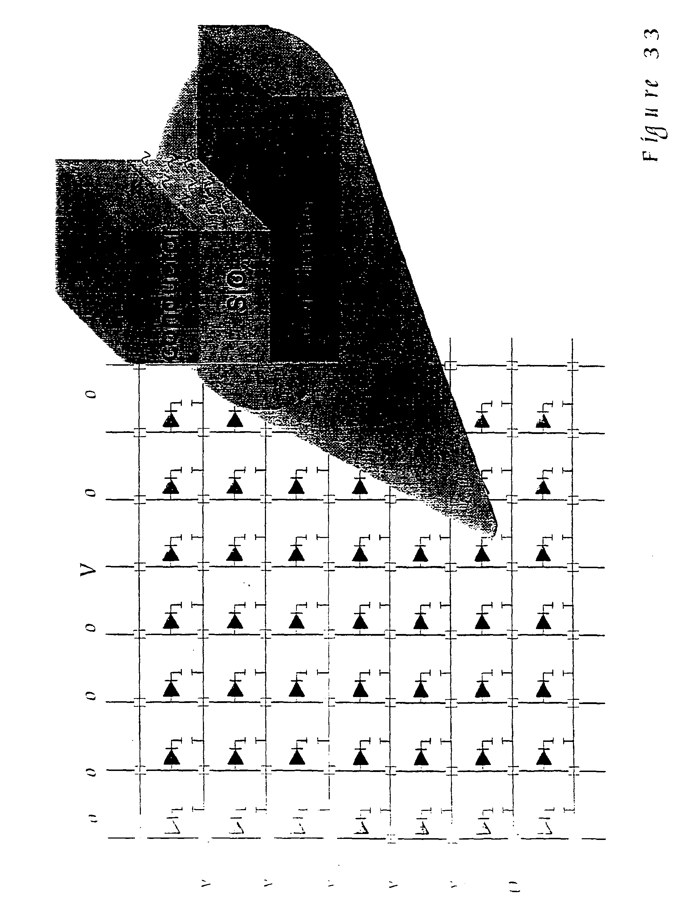

The application of the electronic device of the invention in a multisite array may require a more sophisticated reading scheme than direct connection to each electrode pair. For such arrays, a multiplexing scheme can be applied. In such scheme, the number of input-output lines is only twice the square root of the number of sites. Figure 33 presents a schematic representation of a multiplexed array detection device. Each detection site is in fact two electrodes separated by a gap wherein the gap serves as a gap between a vertical line and a horizontal line, in series with a diode or non-linear component. A biasing scheme is now applied to read the conductance of the different sites. The nonlinear elements are used to prevent *"cross-talk"* between different sites. Detection of the conductance of a specific site is achieved by setting all vertical lines in the set to zero bias except the one leading to the specific detection site to be read which is set to a positive voltage. All horizontal lines are set to positive voltage except the one connected to the same site which was set to zero. In a case that one specific assay set comprising of two electrodes is made conductive due to hybridization of a labeled target to it and deposition process as described above, the electrical circuit is closed. Thus, the current can flow between the *"plus V"* side of the voltage source (not shown in the Fig.) through the vertical line, through the diode or non-linear component, through the deposited bridge in the assay set tested or to the electrode and the horizontal line to the *"minus V*" side of the source. Currents can be monitored by an amperemeter in series. In this scheme, no other site, even if conductive, can contribute to conductance between any two lines in the system as all the relevant diodes are negatively biased. Scanning the vertical positive voltage across all vertical lines and setting a zero bias for the different horizontal lines, the conductivity of all detection sites can be monitored. Moreover, fast scanning techniques can be applied allowing the monitoring of 1,000,000, detection sites in seconds or less with only 1000 vertical and 1000 horizontal lines.

### Example 28: Quantitative measurement of amount of nucleic acid sequences in a sample

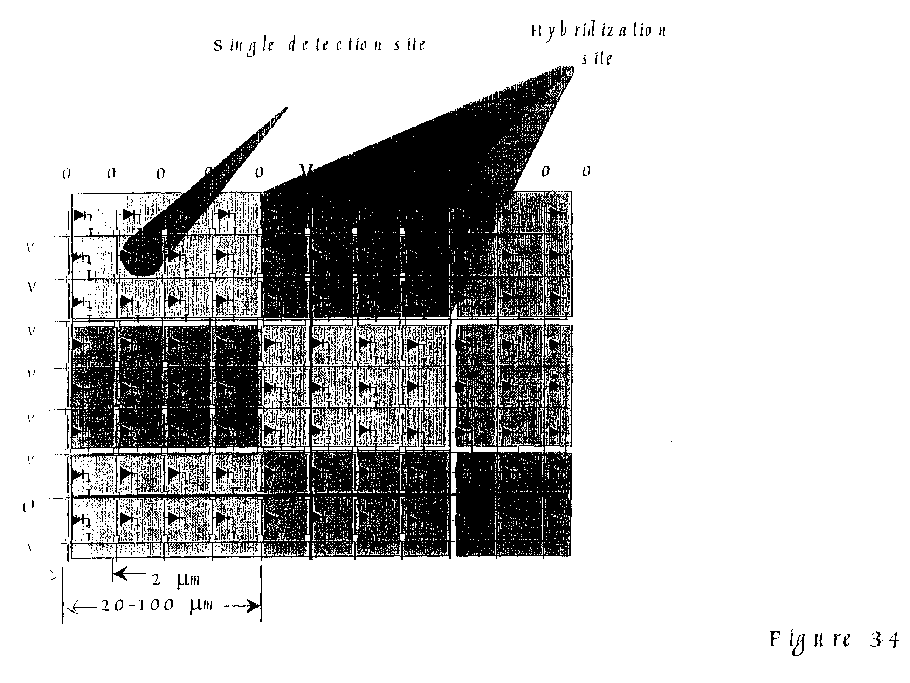

Electrical detection in a DNA array is performed at detection sites having minimal size set by microelectronics (areas as small as fraction of square micron). A hybridization site containing one type of DNA (or RNA) reagents may span a larger area and may therefore contain many detection sites. One should then distinguish between individual detection sites (shown schematically in Fig. 34 a single electrode component of spaced apart electrodes and a diode or non-linear component and hybridization sites (shown schematically in Fig. 34 as different areas containing a plurality of individual detection sites having all the same probe sequence at the gap bridging the electrode pair of the individual detection site). The fact that a hybridization site contains many, distinguishable, individual detection sites is of great advantage and can be used to carry out a quantitative measurement of the sample's target quantity, increasing the signal-to-noise ratio, and considerably decreasing the amount of false positive results.

Fig. 34 depicts a schematic multiplex configuration divided into many individual detection sites, inside each hybridization site. For example, 10,000 hybridization site array (namely 10,000 different oligonucleotides attached or synthesized at sites or 10,000 cDNAs at different sites) can be composed of 100 individual detection sites per hybridization site to give a 1,000,000 site multiplex array.

Consider now the figure, which shows **n** detection-sites per one hybridization site. The detection site is made small enough, such that for a given target sample, the probability to have a DNA (or RNA) molecule hybridized to it is less than unity. After gold deposition process, in each hybridization site we will find **m<n** conductive detection sites due to hybridization and formation of a metal bridge. A quantitative measurement of the amount of target molecules can be performed by counting the fraction of positive conducting sites, **m/n** within each hybridization site. One can also lump together **p** different detection sites to create a meta-site.Setting a detection threshold such that only for **p>p_{critical}** a meta-signal is considered positive detection, one can suppress false positives exponentially by choosing a proper **p.** Thus, the signal-to-noise ratio can also be significantly enhanced. This method allows the increase of dynamic range of the measurement as the detection sites at different hybridization sites can be lumped to different meta-site sizes, allowing different sensitivities and thresholds. The electronic multiplexing measurement allows easy_control of such manipulation by, for example, cumputer control and proper software.

### Example 29: Multiplex array having different layers

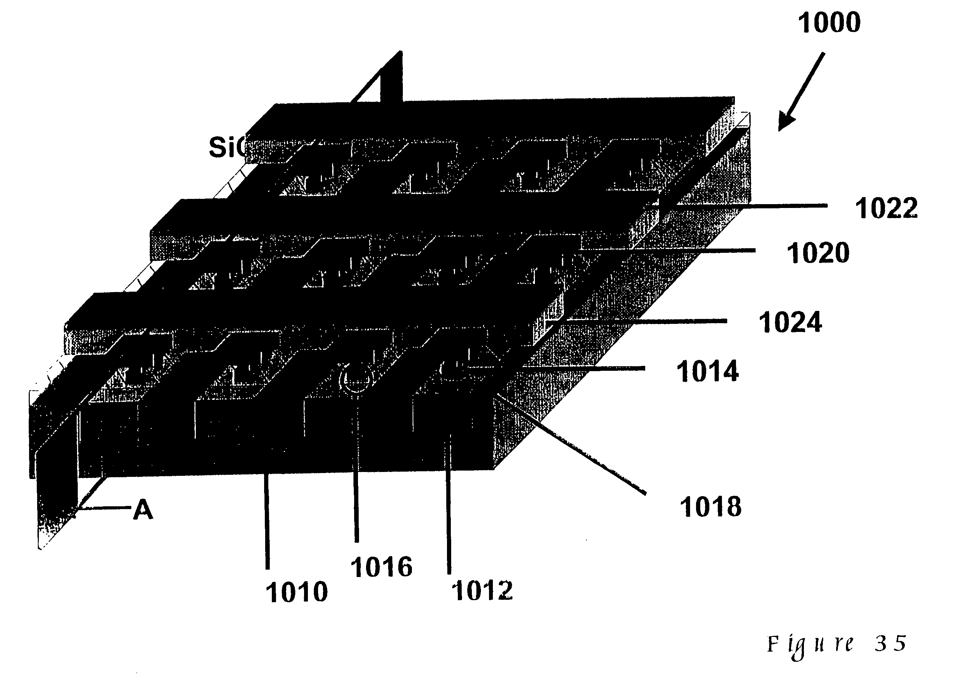

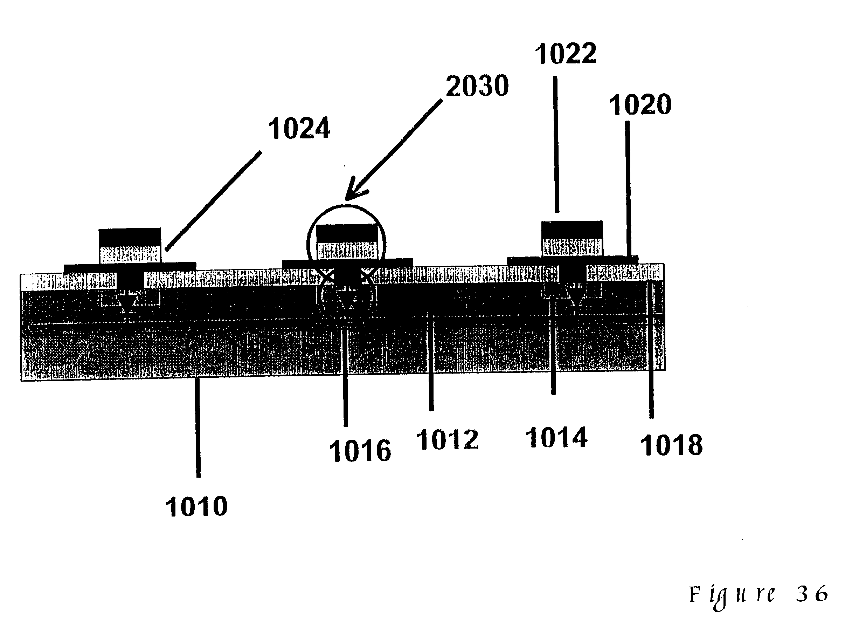

Fig. 35 depicts a specific microelectronic embodiment of a multiplexed DNA array of Fig. 33 or 34, **1000**, comprising different layers. The base, **1010,** is composed of a doped p-type silicon. Using a photoresist mask defined by photolithography, n-type parallel channels, **1012,** are defined using ion implantation techniques. The photoresist is then removed and the implanted area is thermally activated. A thin silicon dioxide layer is then grown on the surface. A second photoresist mask is defined with holes for p-type implantation where the diode's anodes are to be created. The wafer is then p-type implanted to form p-type areas, **1014,** the photoresist is removed, and the implantation is thermally activated. A p-type area, relative to the n-type strips form the non-linear element, **1016,** needed for a multiplexed reading scheme. The silicon dioxide is then etched off and a new layer is grown, **1018.** Holes are opened in the silicon dioxide layer and bottom conductive electrodes, **1020,** are deposited by evaporation and lift-off. A low temperature oxide layer is then grown, followed by a second conductive layer. The top conductor and the low temperature oxide layers are then etched down to the silicon dioxide layer, to form the other electrode, **1022,** and the insulating gap **1024.** Fig. 36 presents the cross section of **1000** along the plane A, i.e. an array of two electrodes separated by gap **2030,** on chip **1000** is defined by a pair of electrodes **1020** and **1022** separated by a gap **1024.** Gap, **1024** will next be used as a hybridization site as explained above.

### Example 30: Microfluidics for reduced sample volume

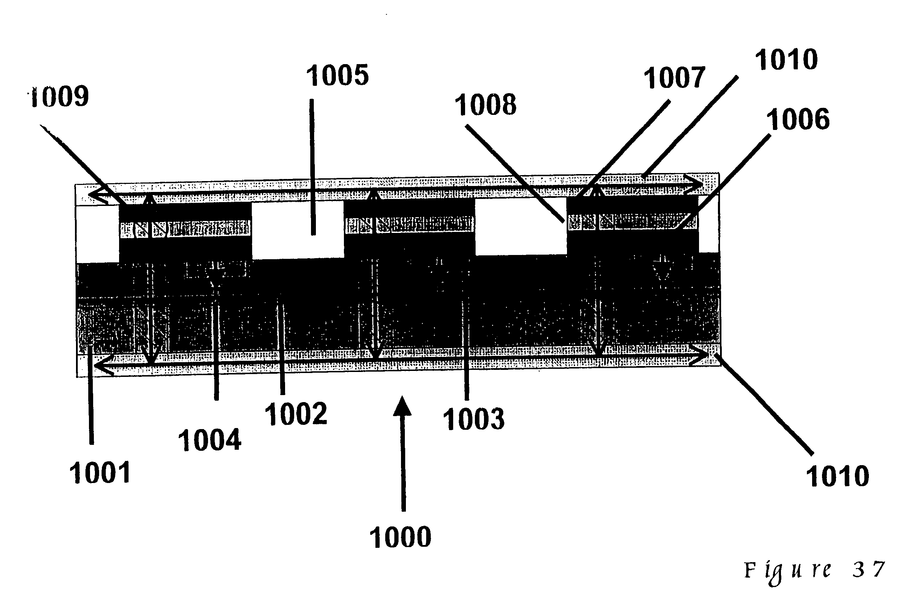

In some applications the ability of working with small sample volumes is of great advantage. For such purposes, embodiments making use of microfluidics techniques may be used. Fig. 37 is a cross-section through a chip which is similar (although not identical) to chip **1000** shown in Fig. 35 or 36. Identical elements to those shown in Fig. 25 have the same number. The base **1001,** is composed of a doped-p-type silicon. Using a photoresist mask defined by photolithography, n-type parallel channels **1002** are defined using ion implantation techniques. The photoresist is then removed and the implanted area is thermally activated. A thin silicon dioxide layer is then grown on the surface. A second photoresist mask is defined with holes for p-type implantation where the diode's anodes are to be created. The wafer is then p-type implanted to form p-type areas **1003,** the photoresist is removed, and the implantation is thermally activated. A p-type area, relative to the n-type strips form the non-linear element **1004,** needed for a multiplexed reading scheme. The silicon dioxide is then etched off and a new layer is grown **1005.** Holes are opened in the silicon dioxide layer and bottom conductive electrodes **1006,** low temperature oxide **1008,** and upper aluminum electrodes **1007,** are deposited by evaporation. Lift-off terminates this step. A third photoresist mask is defined with holes where the holes in the device are to be created. The detection site **1009** on chip **1000** is defined by a pair of electrodes **1006** and **1007** separated by a gap **1008.** Gap **1008** will next be used as a hyridization site. Chip **1000** is placed between two solution ducts that serve as a reservoir **1010** for the sample solution. Solutions are driven back and forth between the two parts of the reservoir through the holes in the chip, thus ensuring efficient contact of sample with hybridization sites **1009** while maintaining low sample volume.

## Claims

1. A system for assaying one or more targets in a sample, comprising:
(a) an assay device having one or more assay sets at least one for each target to be assayed; each of the assay sets comprising at least two electrodes and a recognition moiety immobilized either to one or more of the at least two electrodes and/or onto a substrate between the at least two electrodes; the recognition moiety being capable of specific binding to one of the targets;
(b) an electric or electronic module for determining electric conductance between the at least two electrodes of each assay set; and
(c) reagents for growing a conducting substance from nucleation centers-forming entities deposited onto or bound to a complex formed between said recognition moiety and said target, which substance forms a conductive bridge between at least two of the electrodes of a set.

2. A system according to Claim 1, wherein said reagents comprise:
(c1) a solution comprising nucleation-centers forming entities for binding to said target if present in the sample; and
(c2) a combination of metal ions and a reducing agent to allow growth of said metal substance on said entities.

3. A system according to Claim 1, wherein said reagents comprise:
(c1) one or more reagents to allow deposition and/or formation of said nucleation center-forming entities on a complex formed between said recognition moiety and said target; and
(c2) a combination of metal ions and a reducing agent to allow growth of said metal substance from said entities.

4. A system according to Claim 2 or 3, wherein said nucleation-center forming entities are colloid particles.

5. A system according, to Claim 2 or 3, wherein said nucleation-center forming entities are metal complexes and/or clusters.

6. A system according to Claim 4, wherein said colloid particles are colloid gold particles.

7. A system according to Claim 5, wherein said metal complexes and/or clusters are gold complexes and/or clusters.

8. A system according to Claim 4, wherein said colloid particles are colloid platinum particles.

9. A system according to Claim 5, wherein said metal complexes and/or clusters are platinum complexes and/or clusters.

10. A system for assaying one or more targets in a sample, comprising:
(a) an assay device having one or more assay sets at least one for each target to be assayed; each of the assay sets comprising at least two electrodes and a recognition moiety immobilized either to one or more of the at least two electrodes and/or on a substrate between the at least two electrodes; the recognition moiety being capable of specific binding to one of the targets;
(b) an electric or electronic module for determining electric conductance between the at least two electrodes of each assay set; and
(c) reagents comprising monomers of a conducting polymer which can bind to a complex formed between said recognition moiety and said target, such that upon polymerization of the monomers a conducting bridge between the at least two electrodes of a set is formed.

11. A system according to Claim 10, wherein said monomers are monomers of polyanyline.

12. A system according to any one of the preceding claims, wherein said one or more targets are one or more nucleic acid sequences.

13. A system according to Claim 12, wherein said recognition moiety is an oligonucleotide having a sequence complementary to at least a portion of sequence of one of said one or more targets.

14. A system according to any one of the preceding claims, wherein a recognition moiety is immobilized on at least one electrode of each assay set.

15. A system according to Claim 14, wherein at least two electrodes of the assay set have each a recognition moieties immobilized thereon, these recognition moieties, being the same or different, bind specifically to the same target.

16. A system according to Claim 14 or 15, wherein the recognition moiety is immobilized onto the electrode by means of a linker conjugated or complexed with the recognition moiety and attached by a covalent or non covalent bond, to the electrode.

17. A system according to any one of the preceding claims, wherein the recognition moiety is immobilized on a carrier substrate which is other than the electrode.

18. A system according to any one of the preceding claims, comprising a plurality of assay sets of electrodes.

19. A system according to Claim 18, wherein all assay sets of electrodes are for assaying the same target.

20. A system according to Claim 18, wherein different assay sets of electrodes or different groups of assay sets are for assaying different targets.

21. A system according to Claim 20, for simultaneous determination at different targets in a sample.

22. A system according to any one of Claims 1-11, when the target is a protein or polypeptide and the recognition moiety is a protein-binding molecule which specifically binds to the target protein.

23. A system according to Claim 22, wherein said recognition moiety is an antibody or antibody fraction comprising at least the antigen-binding domain of the antibody.

24. A method for assaying one or more targets in a sample comprising:
(a) providing an assay device having one or more assay sets at least one for each target to be assayed; each of the, assay sets comprising at least two electrodes and a recognition moiety immobilized either to one or more of the at least two electrodes and/or on a substrate between the at least two electrodes; the recognition moiety being capable of specific binding to one of the targets;
(b) contacting said assay device with said sample under conditions permitting binding of targets to specific recognition moieties;
(c) contacting said device with a first reagent solutions to form nucleation-center forming entities on complexes formed between a target and a recognition moiety;
(d) connecting said device with a second reagent solution to grow a conducting metal substance from said nucleation center for a time sufficient to yield a conductive bridge between said at least two electrodes;
(e) connecting said at least two electrodes to an electric or electronic module to measure conductance between said at least two electrodes; and
(f) determining conductance between said at least two electrodes, conductance above a threshold conductance indicating the presence of a respective target in the sample.

25. A method for assaying one or more targets in a sample, comprising:
(a) reacting the sample targets with a first reagent solution to bind nucleation-center forming entities to said targets;
(b) providing an assay device having one or more assay sets at least one for each target to be assayed; each of the assay sets comprising at least two electrodes and a recognition moiety immobilized either to one or more of the at least two electrodes and/or on a substrate between the at least two electrodes; the recognition moiety being capable of specific binding to one of the targets;
(c) contacting said assay device with said sample under conditions permitting binding of targets to specific recognition moieties;
(d) contacting said device with a second reagent solution to grow a conducting metal substance from said nucleation center for a time sufficient to yield a conductive bridge between said at least two electrodes;
(e) connecting said at least two electrodes to an electric or electronic module to measure conductance between said at least two electrodes; and
(f) determining conductance between said at least two electrodes, conductance above a threshold conductance indicating the presence of a respective target in the sample.

26. A method for assaying one or more targets in a sample, comprising:
(a) providing an assay device having one or more assay sets at least one for each target to be assayed; each of the assay sets comprising at least two electrodes and a recognition moiety immobilized either to one or more of the at least two electrodes and/or on a substrate between the at least two electrodes; the recognition moiety being capable of specific binding to one of the targets;
(b) contacting said assay device with said sample under conditions permitting binding of targets to specific recognition moieties;
(c) contacting said device with a first reagent solution comprising monomers of a conductive polymer such that said monomers can bind to complexes formed between the targets and recognition moieties;
(d) treating said device such that said monomers will polymerize to form a conducting polymer, and thereby forming a conducting bridge between at least two electrodes of at least one assay set; and
(e) determining a conductance between said at least two electrodes, conductance above a threshold conductance indicating the presence of a respective target in the sample,

27. A method according to Claim 26, comprising the following step (a₀) before step (a):
(a₀) reacting the sample with a second reagent solution containing entities which can form nucleation centers for growing therefrom a conductive polymer from said monomers, such that said entities bind to said targets if present in the sample.

28. A method according to Claim 26, comprising the following step (at) after step (a):
(a₁) contacting said assay device with a second reagent solution containing entities which can form nucleation centers for growing therefrom a conductive polymer from said monomers, such that said entities bind to said targets of bound to said recognition moieties.

29. A method according to any one of Claims 24 to 28, wherein said targets are nucleic acid sequences and the recognition moieties are oligonucleotides, each of which has a sequence which is complementary to one of the sequences of said targets.

30. A method according to any one of Claims 24 to 29; wherein the level of determining conductance serves as a measure of concentration of the target in the sample.

31. A kit for use in assaying one or more targets in a sample, comprising:
(a) an assay device having one or more assay sets at least one for each target to be assayed; each of the assay sets comprising at least two electrodes and a recognition moiety immobilized either to one or more of the at least two electrodes and/or onto a substrate between the at least two electrodes; the recognition moiety being capable of specific binding to one of the targets; and (b) reagents for growing a conducting substance from nucleation centers-forming entities deposited onto or bound to a complex formed between said recognition moiety and said target, which substance forms a conductive bridge between at least two of the electrodes of a set.

32. A kit according to Claim 31, where said reagents comprise:
(b₁) a solution comprising nucleation-centers forming entities for binding to said target if present in the sample; and
(b₂) a combination of metal ions and a reducing agent to allow growth of said metal substance on said entities.

33. A kit according to Claim 31, where said reagents comprise:
(b₁) one or more reagents to allow deposition and/or formation of said nucleation-center forming entities on a complex formed between said recognition moiety and said target; and
(b₂) a combination of metal ions and a reducing agent to allow growth of said metal substance from said entities.

34. A kit for use in assaying one or more targets in the sample comprising:
(a) an assay device having one or more assay sets at least one for each target to be assayed; each of the assay sets comprising at least two electrodes and a recognition moiety immobilized either to one or more of the at least two electrodes and/or onto a substrate between the at least two electrodes; the recognition moiety being capable of specific binding to one of the targets; and
(b) reagents comprising monomers of a conducting polymer which can bind to the target or to a complex formed between said recognition moiety and said target, such that upon polymerization of the monomers a conducting bridge between the at least two electrodes of a set is formed.

35. A system according to any one of claims 18 to 23, wherein the device is an electronic device comprising an integrated circuit comprising the first group of N₁ conductors and a second group of N₂ conductors, defining between them N₁xN₂ junctions, each such junction being formed with an electronic module comprising two electrodes, each one linked to or defined as an integral portion of one of the conductors, and comprises a diode or non-linear component permitting current flow through the electronic module only in the direction from the first group of conductors to the second group of conductors, whereby a current flowing between one conductor of the first group to one conductor of the second group of conductors defines a single junction point between them; each pair of electrodes forming part of an assay set, each assay set having a recognition moiety bound either to at least one of the electrodes or to a non-conducting substance disposed between the electrodes,
or comprising a microelectronic device having a plurality of layers, with a first group of conductors being defined as stripes in one of more first layers and a second group of conductors being defined as stripes in one or more second layers of the device with each of said second layers being separated from a first layer by a non-conducting substance, electrodes of the device being formed as open ends of the conductors by openings or cut-outs in a vertical direction through the layers;
each pair of electrodes forming part of an assay set, each assay set, having a recognition moiety bound either to at least one of the electrodes or to a non-conducting substance present between the electrodes.

36. A system according to claim 35, wherein the distance of center of one assay set to a center of an adjacent assay set is 100 µM or less.

37. A method according to any one of claims 24 to 30, wherein said device is an electronic device comprising an integrated circuit comprising the first group of N₁ conductors and a second group of N₂ conductors, defining between them N₁xN₂ junctions, each such junction being formed with an electronic module comprising two electrodes, each one linked to or defined as an integral portion of one of the conductors, and comprises a diode or non-linear component permitting current flow through the electronic module only in the direction from the first group of conductors to the second group of conductors, whereby a current flowing between one conductor of the first group to one conductor of the second group of conductors defines a single junction point between them; each pair of electrodes forming part of an assay set, each assay set having a recognition moiety bound either to at least one of the electrodes or to a non-conducting substance disposed between the electrodes,
or comprising a microelectronic device having a plurality of layers, with a first group of conductors being defined as stripes in one of more first layers and a second group of conductors being defined as stripes in one or more second layers of the device with each of said second layers being separated from a first layer by a non-conducting substance, electrodes of the device being formed as open ends of the conductors by openings or cut-outs in a vertical direction through the layers;
each pair of electrodes forming part of an assay set, each assay set, having a recognition moiety bound either to at least one of the electrodes or to a non-conducting substance present between the electrodes.

38. A method according to claim 37, wherein said device has a plurality of assay sets for each target to be assayed, the method comprising determining the proportion of assay sets displaying a conductance above thresholds, out of all assay sets for one target and based on such determination determining concentration of the target in the sample.

39. A method according to any one of claims 24 to 26 wherein the level of conductance between said at least two electrodes is a measure of the concentration of the target in the sample.

## Patentansprüche

1. System zum Untersuchen einer oder mehrerer Zielsubstanzen in einer Probe, umfassend:
(a) eine Untersuchungsvorrichtung mit einem oder mehreren Assay-Sets, mindestens eines für jede zu untersuchende Zielsubstanz, wobei jedes der Assay-Sets mindestens zwei Elektroden und eine Erkennungsstruktur umfasst, die entweder auf einer oder mehreren der mindestens zwei Elektroden und/oder auf einem Substrat zwischen den mindestens zwei Elektroden immobilisiert ist, wobei die Erkennungsstruktur geeignet ist, spezifisch eine der Zielsubstanzen zu binden,
(b) ein elektrisches oder elektronisches Modul zum Bestimmen einer elektrischen Leitfähigkeit zwischen den mindestens zwei Elektroden in jedem Assay-Set und
(c) Reagenzien zum Entwickeln einer leitfähigen Substanz aus Nukleationszentren bildenden Funktionseinheiten, die an einen zwischen der Erkennungsstruktur und der Zielsubstanz gebildeten Komplex angelagert oder gebunden sind, wobei die Substanz eine leitfähige Brücke zwischen mindestens zwei Elektroden eines Sets bildet.

2. System nach Anspruch 1, wobei die Reagenzien umfassen:
(c1) eine Lösung, die Nukleationszentren bildende Funktionseinheiten umfasst, um die Zielsubstanz zu binden, wenn sie in der Probe vorhanden ist, und
(c2) eine Kombination von Metallionen und einem Reduktionsmittel, um ein Entwickeln der Metallsubstanz auf den Funktionseinheiten zu ermöglichen.

3. System nach Anspruch 1, wobei die Reagenzien umfassen:
(c1) ein oder mehrere Reagenzien, um eine Anlagerung und/oder Bildung der Nukleationszentren bildenden Funktionseinheiten auf einem zwischen der Erkennungsstruktur und der Zielsubstanz gebildeten Komplex zu ermöglichen, und
(c2) eine Kombination von Metallionen und einem Reduktionsmittel, um ein Entwickeln der Metallsubstanz aus den Funktionseinheiten zu ermöglichen.

4. System nach Anspruch 2 oder 3, wobei die Nukleationszentren bildenden Funktionseinheiten Kolloidpartikel sind.

5. System nach Anspruch 2 oder 3, wobei die Nukleationszentren bildenden Funktionseinheiten Metallkomplexe und/oder -cluster sind.

6. System nach Anspruch 4, wobei die Kolloidpartikel kolloide Goldpartikel sind.

7. System nach Anspruch 5, wobei die Metallkomplexe und/oder - cluster Goldkomplexe und/oder -cluster sind.

8. System nach Anspruch 4, wobei die Kolloidpartikel kolloide Platinpartikel sind.

9. System nach Anspruch 5, wobei die Metallkomplexe und/oder - cluster Platinkomplexe und/oder -cluster sind.

10. System zum Untersuchen einer oder mehrerer Zielsubstanzen in einer Probe, umfassend:
(a) eine Untersuchungsvorrichtung mit einem oder mehreren Assay-Sets, mindestens eines für jede zu untersuchende Zielsubstanz, wobei jedes der Assay-Sets mindestens zwei Elektroden und eine Erkennungsstruktur umfasst, die entweder auf einer oder mehreren der mindestens zwei Elektroden und/oder auf einem Substrat zwischen den mindestens zwei Elektroden immobilisiert ist, wobei die Erkennungsstruktur geeignet ist, spezifisch eine der Zielsubstanzen zu binden,
(b) ein elektrisches oder elektronisches Modul zum Bestimmen einer elektrischen Leitfähigkeit zwischen den mindestens zwei Elektroden in jedem Assay-Set und
(c) Reagenzien umfassend Monomere eines leitfähigen Polymers, die an einen zwischen der Erkennungsstruktur und der Zielsubstanz gebildeten Komplex derart binden können, dass bei Polymerisation der Monomere eine leitfähige Brücke zwischen den mindestens zwei Elektroden eines Sets gebildet wird.

11. System nach Anspruch 10, wobei die Monomere Polyanilinmonomere sind.

12. System nach einem der vorhergehenden Ansprüche, wobei die eine oder die mehreren Zielsubstanzen eine oder mehrere Nukleinsäuresequenzen sind.

13. System nach Anspruch 12, wobei die Erkennungsstruktur ein Oligonukleotid ist, das eine Sequenz aufweist, die zu mindestens einem Sequenzabschnitt einer der einen oder mehreren Zielsubstanzen komplementär ist.

14. System nach einem der vorhergehenden Ansprüche, wobei eine Erkennungsstruktur auf mindestens einer Elektrode jedes Assay-Sets immobilisiert ist.

15. System nach Anspruch 14, wobei mindestens zwei Elektroden des Assay-Sets jeweils eine darauf immobilisierte Erkennungsstruktur aufweisen, wobei diese Erkennungsstrukturen, die gleich oder unterschiedlich sind, spezifisch die gleiche Zielsubstanz binden.

16. System nach Anspruch 14 oder 15, wobei die Erkennungsstruktur auf der Elektrode mittels eines Linkers immobilisiert ist, der mit der Erkennungsstruktur konjugiert oder komplexiert ist und durch eine kovalente oder nicht-kovalente Bindung mit der Elektrode verknüpft ist.

17. System nach einem der vorhergehenden Ansprüche, wobei die Erkennungsstruktur auf einem Trägersubstrat immobilisiert ist, das nicht die Elektrode ist.

18. System nach einem der vorhergehenden Ansprüche, umfassend eine Mehrzahl von Assay-Sets mit Elektroden.

19. System nach Anspruch 18, wobei alle Assay-Sets mit Elektroden zum Untersuchen der gleichen Zielsubstanz dienen.

20. System nach Anspruch 18, wobei unterschiedliche Assay-Sets mit Elektroden oder untererschiedliche Gruppen von Assay-Sets zum Untersuchen unterschiedlicher Zielsubstanzen dienen.

21. System nach Anspruch 20, zur simultanen Bestimmung von unterschiedlichen Zielsubstanzen in einer Probe.

22. System nach einem der Ansprüche 1 bis 11, wobei die Zielsubstanz ein Protein oder Polypeptid ist und die Erkennungsstruktur ein proteinbindendes Molekül ist, das spezifisch das Zielprotein bindet.

23. System nach Anspruch 22, wobei die Erkennungsstruktur ein Antikörper oder eine Antikörperfraktion ist, die mindestens die antigenbindende Domäne des Antikörpers umfasst.

24. Verfahren zum Untersuchen einer oder mehrerer Zielsubstanzen in einer Probe, umfassend:
(a) Bereitstellen einer Untersuchungsvorrichtung mit einem oder mehreren Assay-Sets, mindestens eines für jede zu untersuchende Zielsubstanz, wobei jedes der Assay-Sets mindestens zwei Elektroden und eine Erkennungsstruktur umfasst, die entweder auf einer oder mehreren der mindestens zwei Elektroden und/oder auf einem Substrat zwischen den mindestens zwei Elektroden immobilisiert ist, wobei die Erkennungsstruktur geeignet ist, spezifisch eine der Zielsubstanzen zu binden,
(b) in Kontakt bringen der Untersuchungsvorrichtung mit der Probe unter Bedingungen, die ein Binden von Zielsubstanzen an spezifische Erkennungsstrukturen ermöglichen,
(c) in Kontakt bringen der Vorrichtung mit einer ersten Reagenslösung, um Nukleationszentren bildende Funktionseinheiten auf Komplexen zu bilden, die zwischen einer Zielsubstanz und einer Erkennungsstruktur gebildet sind,
(d) Verbinden der Vorrichtung mit einer zweiten Reagenslösung, um aus dem Nukleationszentrum eine leitfähige Metallsubstanz über eine Zeit zu entwickeln, die ausreichend ist, eine leitfähige Brücke zwischen den mindestens zwei Elektroden zu erzielen,
(e) Verbinden der mindestens zwei Elektroden mit einem elektrischen oder elektronischen Modul, um eine Leitfähigkeit zwischen den mindestens zwei Elektroden zu messen, und
(f) Bestimmen einer Leitfähigkeit zwischen den mindestens zwei Elektroden, wobei eine Leitfähigkeit über einer Schwellenleitfähigkeit das Vorliegen einer zugeordneten Zielsubstanz in der Probe anzeigt.

25. Verfahren zum Untersuchen einer oder mehrerer Zielsubstanzen in einer Probe, umfassend:
(a) Umsetzen der Zielsubstanzen mit einer ersten Reagenslösung, um Nukleationszentren bildende Funktionseinheiten an die Zielsubstanzen zu binden,
(b) Bereitstellen einer Untersuchungsvorrichtung mit einem oder mehreren Assay-Sets, mindestens eines für jede zu untersuchende Zielsubstanz, wobei jedes der Assay-Sets mindestens zwei Elektroden und eine Erkennungsstruktur umfasst, die entweder auf einer oder mehreren der mindestens zwei Elektroden und/oder auf einem Substrat zwischen den mindestens zwei Elektroden immobilisiert ist, wobei die Erkennungsstruktur geeignet ist, spezifisch eine der Zielsubstanzen zu binden,
(c) in Kontakt bringen der Untersuchungsvorrichtung mit der Probe unter Bedingungen, die ein Binden der Zielsubstanzen an spezifische Erkennungsstrukturen ermöglichen,
(d) in Kontakt bringen der Vorrichtung mit einer zweiten Reagenslösung, um aus dem Nukleationszentrum eine leitfähige Metallsubstanz über eine Zeit zu entwickeln, die ausreichend ist, eine leitfähige Brücke zwischen den mindestens zwei Elektroden zu erzielen,
(e) Verbinden der mindestens zwei Elektroden mit einem elektrischen oder elektronischen Modul, um eine Leitfähigkeit zwischen den mindestens zwei Elektroden zu messen, und
(f) Bestimmen einer Leitfähigkeit zwischen den mindestens zwei Elektroden, wobei eine Leitfähigkeit über einer Schwellenleitfähigkeit das Vorliegen einer zugeordneten Zielsubstanz in der Probe anzeigt.

26. Verfahren zum Untersuchen einer oder mehrerer Zielsubstanzen in einer Probe, umfassend:
(a) Bereitstellen einer Untersuchungsvorrichtung mit einem oder mehreren Assay-Sets, mindestens eines für jede zu untersuchende Zielsubstanz, wobei jedes der Assay-Sets mindestens zwei Elektroden und eine Erkennungsstruktur umfasst, die entweder auf einer oder mehreren der mindestens zwei Elektroden und/oder auf einem Substrat zwischen den mindestens zwei Elektroden immobilisiert ist, wobei die Erkennungsstruktur geeignet ist, spezifisch eine der Zielsubstanzen zu binden,
(b) in Kontakt bringen der Untersuchungsvorrichtung mit der Probe unter Bedingungen, die ein Binden von Zielsubstanzen an spezifische Erkennungsstrukturen ermöglichen,
(c) in Kontakt bringen der Vorrichtung mit einer ersten Reagenslösung, die Monomere eines leitfähigen Polymers umfasst, derart, dass die Monomere an Komplexe binden können, die zwischen den Zielsubstanzen und Erkennungsstrukturen gebildet sind,
(d) Behandeln der Vorrichtung derart, dass die Monomere unter Bildung eines leitfähigen Polymers polymerisieren, und **dadurch** eine leitfähige Brücke zwischen mindestens zwei Elektroden von mindestens einem Assay-Set bilden, und
(e) Bestimmen einer Leitfähigkeit zwischen den mindestens zwei Elektroden, wobei eine Leitfähigkeit über einer Schwellenleitfähigkeit das Vorliegen einer zugeordneten Zielsubstanz in der Probe anzeigt.

27. Verfahren nach Anspruch 26, umfassend den folgenden Schritt (a₀) vor Schritt (a):
(a₀) Umsetzen der Probe mit einer zweiten Reagenslösung, die Funktionseinheiten enthält, die Nukleationszentren bilden können, um daraus ein leitfähiges Polymer aus den Monomeren derart zu entwickeln, dass die Funktionseinheiten die Zielsubstanzen binden, wenn sie in der Probe vorhanden sind.

28. Verfahren nach Anspruch 26, umfassend den folgenden Schritt (a₁) nach Schritt (a):
(a₁) in Kontakt bringen der Untersuchungsvorrichtung mit einer zweiten Reagenslösung, die Funktionseinheiten enthält, die Nukleationszentren bilden können, um daraus ein leitfähiges Polymer aus den Monomeren derart zu entwickeln, dass die Funktionseinheiten die Zielsubstanzen binden, wenn sie an die Erkennungsstrukturen gebunden sind.

29. Verfahren nach einem der Ansprüche 24 bis 28, wobei die Zielsubstanzen Nukleinsäuresequenzen sind und die Erkennungsstrukturen Oligonukleotide sind, deren jedes eine Sequenz aufweist, die zu einer der Sequenzen der Zielsubstanzen komplementär ist.

30. Verfahren nach einem der Ansprüche 24 bis 29, wobei die Bestimmungshöhe der Leitfähigkeit als Konzentrationsmaß der Zielsubstanz in der Probe dient.

31. Kit zur Verwendung beim Untersuchen einer oder mehrerer Zielsubstanzen in einer Probe, umfassend:
(a) eine Untersuchungsvorrichtung mit einem oder mehreren Assay-Sets, mindestens eines für jede zu untersuchende Zielsubstanz, wobei jedes der Assay-Sets mindestens zwei Elektroden und eine Erkennungsstruktur umfasst, die entweder auf einer oder mehreren der mindestens zwei Elektroden und/oder auf einem Substrat zwischen den mindestens zwei Elektroden immobilisiert ist, wobei die Erkennungsstruktur geeignet ist, spezifisch eine der Zielsubstanzen zu binden, und
(b) Reagenzien zum Entwickeln einer leitfähigen Substanz aus Nukleationszentren bildenden Funktionseinheiten, die an einen zwischen der Erkennungsstruktur und der Zielsubstanz gebildeten Komplex angelagert oder gebunden sind, wobei die Substanz eine leitfähige Brücke zwischen mindestens zwei Elektroden eines Sets bildet.

32. Kit nach Anspruch 31, wobei die Reagenzien umfassen:
(b₁) eine Lösung, die Nukleationszentren bildende Funktionseinheiten umfasst, um die Zielsubstanz zu binden, wenn sie in der Probe vorhanden ist, und
(b₂) eine Kombination von Metallionen und einem Reduktionsmittel, um ein Entwickeln der Metallsubstanz auf den Funktionseinheiten zu ermöglichen.

33. Kit nach Anspruch 31, wobei die Reagenzien umfassen:
(b₁) ein oder mehrere Reagenzien, um eine Anlagerung und/oder Bildung der Nukleationszentren bildenden Funktionseinheiten an einen zwischen der Erkennungsstruktur und der Zielsubstanz gebildeten Komplex zu ermöglichen, und
(b₂) eine Kombination von Metallionen und einem Reduktionsmittel, um ein Entwickeln der Metallsubstanz aus den Funktionseinheiten zu ermöglichen.

34. Kit zur Verwendung beim Untersuchen einer oder mehrerer Zielsubstanzen in einer Probe, umfassend:
(a) eine Untersuchungsvorrichtung mit einem oder mehreren Assay-Sets, mindestens eines für jede zu untersuchende Zielsubstanz, wobei jedes der Assay-Sets mindestens zwei Elektroden und eine Erkennungsstruktur umfasst, die entweder auf einer oder mehreren der mindestens zwei Elektroden und/oder auf einem Substrat zwischen den mindestens zwei Elektroden immobilisiert ist, wobei die Erkennungsstruktur geeignet ist, spezifisch eine der Zielsubstanzen zu binden, und
(b) Reagenzien umfassend Monomere eines leitfähigen Polymers, die an eine Zielsubstanz oder an einen zwischen der Erkennungsstruktur und der Zielsubstanz gebildeten Komplex derart binden können, dass bei Polymerisation der Monomere eine leitfähige Brücke zwischen den mindestens zwei Elektroden eines Sets gebildet wird.

35. System nach einem der Ansprüche 18 bis 23, wobei die Vorrichtung ein elektronisches Bauteil ist, das eine integrierte Schaltung umfasst, die eine erste Gruppe von N₁ Leitern und eine zweite Gruppe von N₂ Leitern umfasst, die zwischen sich N₁xN₂ Übergänge definieren, wobei jeder dieser Übergänge mit einem elektronischen Modul gebildet ist, das zwei Elektroden umfasst, wobei jede mit einem integralen Abschnitt eines der Leiter verknüpft oder als solcher definiert ist, und eine Diode oder eine nicht-lineare Komponente umfasst, die einen Stromfluss durch das elektronische Modul nur in die Richtung von der ersten Leitergruppe zur zweiten Leitergruppe ermöglicht, wodurch ein Strom, der zwischen einem Leiter der ersten Leitergruppe zu einem Leiter der zweiten Leitergruppe fließt, einen einzelnen Übergangspunkt zwischen ihnen definiert, wobei jedes Paar Elektroden Teil eines Assay-Sets bildet, wobei jedes Assay-Set eine Erkennungsstruktur umfasst, die entweder an mindestens eine der Elektroden oder an eine zwischen den Elektroden angeordnete nicht-leitende Substanz gebunden ist,
oder ein mikroelektronisches Bauteil mit einer Mehrzahl von Schichten umfasst, wobei eine erste Leitergruppe als Streifen in einer von mehreren ersten Schichten definiert ist und eine zweite Leitergruppe als Streifen in einer oder mehreren zweiten Schichten des Bauteils definiert ist, wobei jede der zweiten Schichten von einer ersten Schicht durch eine nicht-leitende Substanz getrennt ist, wobei Elektroden des Bauteils als offene Enden der Leiter in Form von Öffnungen oder Ausschnitten in vertikaler Richtung durch die Schichten gebildet sind,
wobei jedes Paar Elektroden Teil eines Assay-Sets bildet, wobei jedes Assay-Set eine Erkennungsstruktur umfasst, die entweder an mindestens eine der Elektroden oder an eine zwischen den Elektroden angeordnete nicht-leitende Substanz gebunden ist.

36. System nach Anspruch 35, wobei der Abstand des Mittelpunkts eines Assay-Sets zu einem Mittelpunkt eines benachbarten Assay-Sets 100 µm oder weniger beträgt.

37. Verfahren nach einem der Ansprüche 24 bis 30, wobei die Vorrichtung ein elektronisches Bauteil ist, das eine integrierte Schaltung umfasst, die eine erste Gruppe von N₁ Leitern und eine zweite Gruppe von N₂ Leitern umfasst, die zwischen sich N₁xN₂ Übergänge definieren, wobei jeder dieser Übergänge mit einem elektronischen Modul gebildet ist, das zwei Elektroden umfasst, wobei jede mit einem integralen Abschnitt eines der Leiter verknüpft oder als solcher definiert ist, und eine Diode oder eine nicht-lineare Komponente umfasst, die einen Stromfluss durch das elektronische Modul nur in die Richtung von der ersten Leitergruppe zur zweiten Leitergruppe ermöglicht, wodurch ein Strom, der zwischen einem Leiter der ersten Leitergruppe zu einem Leiter der zweiten Leitergruppe fließt, einen einzelnen Übergangspunkt zwischen ihnen definiert, wobei jedes Paar Elektroden Teil eines Assay-Sets bildet, wobei jedes Assay-Set eine Erkennungsstruktur umfasst, die entweder an mindestens eine der Elektroden oder an eine zwischen den Elektroden angeordnete nicht-leitende Substanz gebunden ist,
oder ein mikroelektronisches Bauteil mit einer Mehrzahl von Schichten umfasst, wobei eine erste Leitergruppe als Streifen in einer von mehreren ersten Schichten definiert ist und eine zweite Leitergruppe als Streifen in einer oder mehreren zweiten Schichten des Bauteils definiert ist, wobei jede der zweiten Schichten von einer ersten Schicht durch eine nicht-leitende Substanz getrennt ist, wobei Elektroden des Bauteils als offene Enden der Leiter in Form von Öffnungen oder Ausschnitten in vertikaler Richtung durch die Schichten gebildet sind,
wobei jedes Paar Elektroden Teil eines Assay-Sets bildet, wobei jedes Assay-Set eine Erkennungsstruktur umfasst, die entweder an mindestens eine der Elektroden oder an eine zwischen den Elektroden angeordnete nicht-leitende Substanz gebunden ist.

38. Verfahren nach Anspruch 37, wobei die Vorrichtung eine Mehrzahl von Assay-Sets für jede zu untersuchende Zielsubstanz aufweist, wobei das Verfahren Bestimmen des Anteils an Assay-Stets aus allen Assay-Sets für eine Zielsubstanz, die eine Leitfähigkeit über Schwellenwerten anzeigen, und basierend auf einer solchen Bestimmung Bestimmen einer Konzentration der Zielsubstanz in der Probe umfasst.

39. Verfahren nach einem der Ansprüche 24 bis 26, wobei die Höhe der Leitfähigkeit zwischen den mindestens zwei Elektroden als Maß der Konzentration der Zielsubstanz in der Probe dient.

## Revendications

1. Système de dosage d'une ou plusieurs cibles dans un échantillon, comprenant :
(a) un dispositif de dosage ayant un ou plusieurs ensembles de dosage, au moins un pour chaque cible devant être dosée ; chacun des ensembles de dosage comprenant au moins deux électrodes et un groupe caractéristique de reconnaissance immobilisé sur une ou plus des au moins deux électrodes et/ou sur un substrat entre les au moins deux électrodes ; le groupe caractéristique de reconnaissance étant apte à une liaison spécifique à l'une des cibles ;
(b) un module électrique ou électronique pour déterminer une conductance électrique entre les au moins deux électrodes de chaque ensemble de dosage ; et
(c) des réactifs pour la croissance d'une substance conductrice à partir d'entités de formation de centres de nucléation déposées sur ou liées à un complexe formé entre ledit groupe caractéristique de reconnaissance et ladite cible, laquelle substance forme un pont conducteur entre au moins deux des électrodes d'un ensemble.

2. Système selon la revendication 1, dans lequel lesdits réactifs comprennent :
(c1) une solution comprenant des entités de formation de centres de nucléation pour une liaison à ladite cible si celle-ci est présente dans l'échantillon ; et
(c2) une combinaison d'ions métal et d'un agent réducteur pour permettre la croissance de ladite substance métallique sur lesdites entités.

3. Système selon la revendication 1, dans lequel lesdits réactifs comprennent :
(c1) un ou plusieurs réactifs pour permettre le dépôt et/ou la formation desdites entités de formation de centres de nucléation sur un complexe formé entre ledit groupe caractéristique de reconnaissance et ladite cible ; et
(c2) une combinaison d'ions métal et d'un agent réducteur pour permettre la croissance de ladite substance métallique à partir desdites entités.

4. Système selon la revendication 2 ou 3, dans lequel lesdites entités de formation de centres de nucléation sont des particules colloïdales.

5. Système selon la revendication 2 ou 3, dans lequel lesdites entités de formation de centres de nucléation sont des complexes et/ou des agrégats métalliques.

6. Système selon la revendication 4, dans lequel lesdites particules colloïdales sont des particules d'or colloïdal.

7. Système selon la revendication 5, dans lequel lesdits complexes et/ou agrégats métalliques sont des complexes et/ou des agrégats d'or.

8. Système selon la revendication 4, dans lequel lesdites particules colloïdales sont des particules de platine colloïdal.

9. Système selon la revendication 5, dans lequel lesdits complexes et/ou agrégats métalliques sont des complexes et/ou des agrégats de platine.

10. Système de dosage d'une ou plusieurs cibles dans un échantillon, comprenant :
(a) un dispositif de dosage ayant un ou plusieurs ensembles de dosage, au moins un pour chaque cible devant être dosée ; chacun des ensembles de dosage comprenant au moins deux électrodes et un groupe caractéristique de reconnaissance immobilisé sur une ou plus des au moins deux électrodes et/ou sur un substrat entre les au moins deux électrodes ; le groupe caractéristique de reconnaissance étant apte à une liaison spécifique à l'une des cibles ;
(b) un module électrique ou électronique pour déterminer une conductance électrique entre les au moins deux électrodes de chaque ensemble de dosage ; et
(c) des réactifs comprenant des monomères d'un polymère conducteur qui peuvent se lier à un complexe formé entre ledit groupe caractéristique de reconnaissance et ladite cible, de telle sorte qu'à la polymérisation des monomères, un pont conducteur entre les au moins deux électrodes d'un ensemble est formé.

11. Système selon la revendication 10, dans lequel lesdits monomères sont des monomères de polyaniline.

12. Système selon l'une quelconque des revendications précédentes, dans lequel lesdites une ou plusieurs cibles sont une ou plusieurs séquences d'acides nucléiques.

13. Système selon la revendication 12, dans lequel ledit groupe caractéristique de reconnaissance est un oligonucléotide ayant une séquence complémentaire d'au moins une partie de séquence de l'une desdites une ou plusieurs cibles.

14. Système selon l'une quelconque des revendications précédentes, dans lequel un groupe caractéristique de reconnaissance est immobilisé sur au moins une électrode de chaque ensemble de dosage.

15. Système selon la revendication 14, dans lequel au moins deux électrodes de l'ensemble de dosage ont chacune des groupes caractéristiques de reconnaissance immobilisés sur celles-ci, ces groupes caractéristiques de reconnaissance, étant identiques ou différents, se lient spécifiquement à la même cible.

16. Système selon la revendication 14 ou 15, dans lequel le groupe caractéristique de reconnaissance est immobilisé sur l'électrode au moyen d'un lieur conjugué ou complexé avec le groupe caractéristique de reconnaissance et lié par une liaison covalente ou non covalente à l'électrode.

17. Système selon l'une quelconque des revendications précédentes, dans lequel le groupe caractéristique de
reconnaissance est immobilisé sur un substrat support qui est autre que l'électrode.

18. Système selon l'une quelconque des revendications précédentes, comprenant une pluralité d'ensembles d'électrodes de dosage.

19. Système selon la revendication 18, dans lequel tous les ensembles d'électrodes de dosage servent à doser la même cible.

20. Système selon la revendication 18, dans lequel différents ensembles d'électrodes de dosage ou différents groupes d'ensembles de dosage servent à doser différentes cibles.

21. Système selon la revendication 20, pour une détermination simultanée de différentes cibles dans un échantillon.

22. Système selon l'une quelconque des revendications 1 à 11, dans lequel la cible est une protéine ou un polypeptide et le groupe caractéristique de reconnaissance est une molécule de liaison à une protéine qui se lie spécifiquement à la protéine cible.

23. Système selon la revendication 22, dans lequel ledit groupe caractéristique de reconnaissance est un anticorps ou une fraction d'anticorps comprenant au moins le domaine de liaison à l'antigène de l'anticorps.

24. Procédé de dosage d'une ou plusieurs cibles dans un échantillon comprenant :
(a) la fourniture d'un dispositif de dosage ayant un ou plusieurs ensembles de dosage, au moins un pour chaque cible devant être dosée ; chacun des ensembles de dosage comprenant au moins deux électrodes et un groupe caractéristique de reconnaissance immobilisé sur une ou plus des au moins deux électrodes et/ou sur un substrat entre les au moins deux électrodes ; le groupe caractéristique de reconnaissance étant apte à une liaison spécifique à l'une des cibles ;
(b) la mise en contact dudit dispositif de dosage avec ledit échantillon sous des conditions permettant la liaison des cibles à des groupes caractéristiques de reconnaissance spécifiques ;
(c) la mise en contact dudit dispositif avec une première solution de réactif pour former des entités de formation de centres de nucléation sur des complexes formés entre une cible et un groupe caractéristique de reconnaissance ;
(d) la connexion dudit dispositif avec une deuxième solution de réactif pour faire croître une substance métallique conductrice à partir dudit centre de nucléation pendant une durée suffisante pour créer un pont conducteur entre lesdites au moins deux électrodes ;
(e) la connexion desdites au moins deux électrodes à un module électrique ou électronique pour mesurer la conductance entre lesdites au moins deux électrodes ; et
(f) la détermination de la conductance entre lesdites au moins deux électrodes, une conductance supérieure à une conductance seuil indiquant la présence d'une cible respective dans l'échantillon.

25. Procédé de dosage d'une ou plusieurs cibles dans un échantillon comprenant :
(a) la réaction des cibles échantillon avec une première solution de réactif pour lier des entités de formation de centres de nucléation auxdites cibles ;
(b) la fourniture d'un dispositif de dosage ayant un ou plusieurs ensembles de dosage, au moins un pour chaque cible devant être dosée ; chacun des ensembles de dosage comprenant au moins deux électrodes et un groupe caractéristique de reconnaissance immobilisé sur une ou plus des au moins deux électrodes et/ou sur un substrat entre les au moins deux électrodes ; le groupe caractéristique de reconnaissance étant apte à une liaison spécifique à l'une des cibles ;
(c) la mise en contact dudit dispositif de dosage avec ledit échantillon sous des conditions permettant la liaison des cibles à des groupes caractéristiques de reconnaissance spécifiques ;
(d) la mise en contact dudit dispositif avec une deuxième solution de réactif pour faire croître une substance métallique conductrice à partir dudit centre de nucléation pendant une durée suffisante pour créer un pont conducteur entre lesdites au moins deux électrodes ;
(e) la connexion desdites au moins deux électrodes à un module électrique ou électronique pour mesurer la conductance entre lesdites au moins deux électrodes ; et
(f) la détermination de la conductance entre lesdites au moins deux électrodes, une conductance supérieure à une conductance seuil indiquant la présence d'une cible respective dans l'échantillon.

26. Procédé de dosage d'une ou plusieurs cibles dans un échantillon comprenant :
(a) la fourniture d'un dispositif de dosage ayant un ou plusieurs ensembles de dosage, au moins un pour chaque cible devant être dosée ; chacun des ensembles de dosage comprenant au moins deux électrodes et un groupe caractéristique de reconnaissance immobilisé sur une ou plus des au moins deux électrodes et/ou sur un substrat entre les au moins deux électrodes ; le groupe caractéristique de reconnaissance étant apte à une liaison spécifique à l'une des cibles ;
(b) la mise en contact dudit dispositif de dosage avec ledit échantillon sous des conditions permettant la liaison des cibles à des groupes caractéristiques de reconnaissance spécifiques ;
(c) la mise en contact dudit dispositif avec une première solution de réactif comprenant des monomères d'un polymère conducteur de telle sorte que lesdits monomères peuvent se lier aux complexes formés entre les cibles et les groupes caractéristiques de reconnaissance ;
(d) le traitement dudit dispositif de telle sorte que lesdits monomères se polymérisent pour former un polymère conducteur, et formant ainsi un pont conducteur entre au moins deux électrodes d'au moins un ensemble de dosage ; et
(e) la détermination d'une conductance entre lesdites au moins deux électrodes, une conductance supérieure à une conductance seuil indiquant la présence d'une cible respective dans l'échantillon.

27. Procédé selon la revendication 26, comprenant l'étape (a₀) suivante avant l'étape (a) :
(a₀) réaction de l'échantillon avec une deuxième solution de réactif contenant des entités qui peuvent former des centres de nucléation pour la croissance à partir de ceux-ci d'un polymère conducteur à partir desdits monomères, de telle sorte que lesdites entités se lient auxdites cibles si celles-ci sont présentes dans l'échantillon.

28. Procédé selon la revendication 26, comprenant l'étape (a₁) suivante après l'étape (a) :
(a₁) mise en contact dudit dispositif de dosage avec une deuxième solution de réactif contenant des entités qui peuvent former des centres de nucléation pour la croissance à partir de ceux-ci d'un polymère conducteur à partir desdits monomères, de telle sorte que lesdites entités se lient auxdites cibles si celles-ci sont liées auxdits groupes caractéristiques de reconnaissance.

29. Procédé selon l'une quelconque des revendications 24 à 28, dans lequel lesdites cibles sont des séquences d'acides nucléiques et les groupes caractéristiques de reconnaissance sont des oligonucléotides, chacun desquels a une séquence qui est complémentaire de l'une des séquences desdites cibles.

30. Procédé selon l'une quelconque des revendications 24 à 29, dans lequel le niveau de détermination de la conductance sert de mesure de concentration de la cible dans l'échantillon.

31. Trousse destinée à une utilisation dans le dosage d'une ou plusieurs cibles dans un échantillon, comprenant :
(a) un dispositif de dosage ayant un ou plusieurs ensembles de dosage, au moins un pour chaque cible devant être dosée ; chacun des ensembles de dosage comprenant au moins deux électrodes et un groupe caractéristique de reconnaissance immobilisé sur une ou plus des au moins deux électrodes et/ou sur un substrat entre les au moins deux électrodes ; le groupe caractéristique de reconnaissance étant apte à une liaison spécifique à l'une des cibles ; et
(b) des réactifs pour la croissance d'une substance conductrice à partir d'entités de formation de centres de nucléation déposées sur ou liées à un complexe formé entre ledit groupe caractéristique de reconnaissance et ladite cible, laquelle substance forme un pont conducteur entre au moins deux des électrodes d'un ensemble.

32. Trousse selon la revendication 31, dans laquelle lesdits réactifs comprennent :
(b1) une solution comprenant des entités de formation de centres de nucléation pour une liaison à ladite cible si celle-ci est présente dans l'échantillon ; et
(b2) une combinaison d'ions métal et d'un agent réducteur pour permettre la croissance de ladite substance métallique sur lesdites entités.

33. Trousse selon la revendication 31, dans laquelle lesdits réactifs comprennent :
(b1) un ou plusieurs réactifs pour permettre le dépôt et/ou la formation desdites entités de formation de centres de nucléation sur un complexe formé entre ledit groupe caractéristique de reconnaissance et ladite cible ; et
(b2) une combinaison d'ions métal et d'un agent réducteur pour permettre la croissance de ladite substance métallique à partir desdites entités.

34. Trousse destinée à une utilisation dans le dosage d'une ou plusieurs cibles dans un échantillon, comprenant :
(a) un dispositif de dosage ayant un ou plusieurs ensembles de dosage, au moins un pour chaque cible devant être dosée ; chacun des ensembles de dosage comprenant au moins deux électrodes et un groupe caractéristique de reconnaissance immobilisé sur une ou plus des au moins deux électrodes et/ou sur un substrat entre les au moins deux électrodes ; le groupe caractéristique de reconnaissance étant apte à une liaison spécifique à l'une des cibles ;
(b) des réactifs comprenant des monomères d'un polymère conducteur qui peuvent se lier à la cible ou à un complexe formé entre ledit groupe caractéristique de reconnaissance et ladite cible, de sorte qu'à la polymérisation des monomères, un pont conducteur entre les au moins deux électrodes d'un ensemble est formé.

35. Système selon l'une quelconque des revendications 18 à 23, dans lequel le dispositif est un dispositif électronique comprenant un circuit intégré comprenant le premier groupe de N₁ conducteurs et un deuxième groupe de N₂ conducteurs, définissant entre eux N₁ x N₂ jonctions, chaque telle jonction étant formée avec un module électronique comprenant deux électrodes, chacune liée à ou définie comme une partie intégrante de l'un des conducteurs, et comprend une diode ou un composant non linéaire permettant qu'un courant ne passe à travers le module électronique que dans la direction du premier groupe de conducteurs vers le deuxième groupe de conducteurs, d'où il résulte qu'un courant passant entre un conducteur du premier groupe et un conducteur du deuxième groupe de conducteurs définit un point de jonction unique entre eux ; chaque paire d'électrodes faisant partie d'un ensemble de dosage, chaque ensemble de dosage ayant un groupe caractéristique de reconnaissance lié soit à au moins une des électrodes, soit à une substance non conductrice disposée entre les électrodes,
ou comprenant un dispositif microélectronique ayant une pluralité de couches, avec un premier groupe de conducteurs étant défini comme des bandes dans une ou plusieurs premières couches et un deuxième groupe de conducteurs défini comme des bandes dans une ou plusieurs deuxièmes couches du dispositif, chacune desdites deuxièmes couches étant séparée d'une première couche par une substance non conductrice, des électrodes du dispositif étant formées comme des extrémités ouvertes des conducteurs par des ouvertures ou des découpes dans un sens vertical à travers les couches ;
chaque paire d'électrodes faisant partie d'un ensemble de dosage, chaque ensemble de dosage ayant un groupe caractéristique de reconnaissance lié soit à au moins une des électrodes, soit à une substance non conductrice présente entre les électrodes.

36. Système selon la revendication 35, dans lequel la distance du centre d'un ensemble de dosage au centre d'un ensemble de dosage adjacent est 100 µm ou moins.

37. Procédé selon l'une quelconque des revendications 24 à 30, dans lequel ledit dispositif est un dispositif électronique comprenant un circuit intégré comprenant le premier groupe de N₁ conducteurs et un deuxième groupe de N₂ conducteurs, définissant entre eux N₁ x N₂ jonctions, chaque telle jonction étant formée avec un module électronique comprenant deux électrodes, chacune liée à ou définie comme une partie intégrante de l'un des conducteurs, et comprend une diode ou un composant non linéaire permettant qu'un courant ne passe à travers le module électronique que dans la direction du premier groupe de conducteurs vers le deuxième groupe de conducteurs, d'où il résulte qu'un courant passant entre un conducteur du premier groupe et un conducteur du deuxième groupe de conducteurs définit un point de jonction unique entre eux ; chaque paire d'électrodes faisant partie d'un ensemble de dosage, chaque ensemble de dosage ayant un groupe caractéristique de reconnaissance lié soit à au moins une des électrodes, soit à une substance non conductrice disposée entre les électrodes,
ou comprenant un dispositif microélectronique ayant une pluralité de couches, avec un premier groupe de conducteurs étant défini comme des bandes dans une ou plusieurs premières couches et un deuxième groupe de conducteurs défini comme des bandes dans une ou plusieurs deuxièmes couches du dispositif, chacune desdites deuxièmes couches étant séparée d'une première couche par une substance non conductrice, des électrodes du dispositif étant formées comme des extrémités ouvertes des conducteurs par des ouvertures ou des découpes dans un sens vertical à travers les couches ;
chaque paire d'électrodes faisant partie d'un ensemble de dosage, chaque ensemble de dosage ayant un groupe caractéristique de reconnaissance lié soit à au moins une des électrodes, soit à une substance non conductrice présente entre les électrodes.

38. Procédé selon la revendication 37, dans lequel ledit dispositif a une pluralité d'ensembles de dosage pour chaque cible devant être dosée, le procédé comprenant la détermination de la proportion d'ensembles de dosage affichant une conductance supérieure à des seuils, parmi la totalité des ensembles de dosage pour une cible et, sur la base d'une telle détermination, la détermination de la concentration de la cible dans l'échantillon.

39. Procédé selon l'une quelconque des revendications 24 à 26, dans lequel le niveau de conductance entre lesdites au moins deux électrodes sert de mesure de la concentration de la cible dans l'échantillon.
